# EUROPEAN PATENT APPLICATION

(11) **EP 1 987 837 A1**
(43) Date of publication of application: **05.11.2008**
(21) Application number: 07008880.2
(22) Date of filing: 02.05.2007
(51) Int. Cl.: A61K 38/17, G01N 33/50, A61K 31/66, A61P 3/10

(54) **Pharmaceutical composition for increasing insulin secretion from pancreatic ß-cells**

(71) Applicant: Technische Universität Dresden Medizinische Fakultät Carl Gustav Carus, 01307 Dresden (DE)
(72) Inventor: Solimena, Michele, 01326 Dresden (DE); Trajkovski, Mirko, 8037 Zürich (CH); Mziaut, Hassan, 01187 Dresden (DE); Schubert, Sandra, 01069 Dresden (DE)
(74) Representative: Vossius & Partner

(57) **Abstract**

The present invention relates to a pharmaceutical composition comprising (a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN; (b) a compound promoting the presence or activity of a protease that cleaves the C-terminus of ICA512, ICA512-TMF or ICA512-CCF or PHOGRIN; (c) a compound promoting the presence or activity of a serine/threonine kinase which phosphorylates β2-syntrophin on serine residue 95; (d) a compound interfering with the presence or activity of a serine/threonine phosphatase which dephosphorylates β2-syntrophin on serine residue 95; (e) a compound promoting the presence or activity of a serine/threonine phosphatase which dephosphorylates β2-syntrophin on serine residue 110; or (f) a compound interfering with the presence or activity of a serine/threonine kinase which phosphorylates β2-syntrophin on serine residue 110.

## Description

The present invention relates to a pharmaceutical composition comprising (a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN; (b) a compound promoting the presence or activity of a protease that cleaves the C-terminus of ICA512, ICA512-TMF or ICA512-CCF or PHOGRIN; (c) a compound promoting the presence or activity of a serine/threonine kinase which phosphorylates β2-syntrophin on serine residue 95; (d) a compound interfering with the presence or activity of a serine/threonine phosphatase which dephosphorylates β2-syntrophin on serine residue 95; (e) a compound promoting the presence or activity of a serine/threonine phosphatase which dephosphorylates β2-syntrophin on serine residue 110; or (f) a compound interfering with the presence or activity of a serine/threonine kinase which phosphorylates β2-syntrophin on serine residue 110. Further, the present invention relates to the use of (a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN; (b) a compound promoting the presence or activity of a protease that cleaves the C-terminus of ICA512, ICA512-TMF, ICA512-CCF or PHOGRIN; (c) a compound promoting the presence or activity of a serine/threonine kinase which phosphorylates β2-syntrophin on serine residue 95; (d) a compound interfering with the presence or activity of a serine/threonine phosphatase which dephosphorylates β2-syntrophin on serine residue 95; (e) a compound promoting the presence or activity of a serine/threonine phosphatase which dephosphorylates β2-syntrophin on serine residue 110; or (f) a compound interfering with the presence or activity of a serine/threonine kinase which phosphorylates β2-syntrophin on serine residue 110 for the preparation of a pharmaceutical composition for the treatment of diabetes.

Several documents are cited throughout the text of this specification. The disclosure content of the documents cited herein (including manufacturer's specifications, instructions, etc.) is herewith incorporated by reference.

Diabetes mellitus is the most common metabolic disorder and its incidence is alarmingly increasing worldwide (Diamond, 2003; Zimmet et al., 2001). Autoimmune destruction of insulin producing β-cells causes type 1 diabetes (Castano and Eisenbarth, 1990; Tisch and McDevitt, 1996), whereas impaired insulin secretion in face of ever-increasing food intake and reduced physical activity leads to type 2 diabetes (Saltiel, 2001). Restoration of β-cell mass and function are therefore major goals in diabetes research.

Each β-cell stores ~10⁴ insulin secretory granules (Bratanova-Tochkova et al., 2002; Rorsman and Renstrom, 2003). Less than 5% of them are readily releasable, i.e. undergo exocytosis during the first phase insulin secretion, which lasts up to 10 minutes after the elevation of blood glucose >5 mM. The remaining >95% of the granules belong to the reserve pool. Recruitment of these granules for exocytosis accounts for the second phase of insulin secretion and requires their priming. This consists of several ATP-, Ca²⁺- and phosphatidylinositol-4,5-bisphosphate [PI(4,5)P2] -dependent steps, which allow secretory vesicles to dock at the plasma membrane and enter the readily releasable pool (Klenchin and Martin, 2000). A critical step in this process is the mobilization of reserve granules. The motor protein kinesin is responsible for the ATP-dependent, microtubule-mediated transport of granules toward the cell surface (Donelan et al., 2002; Varadi et al., 2002), while myosin Va, also an ATPase, drives the movement of insulin granules on cortical actin microfilaments (Ivarsson et al., 2005; Varadi et al., 2005). By sustaining the ATP-dependent refilling of the readily releasable pool, fuel segretagogues such as glucose can greatly amplify insulin secretion relative to agents such as sulphonylureas, which evoke mostly, albeit not exclusively (Eliasson et al., 2003), the exocytosis of pre-existing primed granules by inducing membrane depolarization and Ca²⁺-entry. The dense meshwork of cortical actin filaments, on the other hand, is thought to inhibit insulin secretion by restricting granule mobility and their access to the plasma membrane (Bruun et al., 2000; Li et al., 1994; Tomas et al., 2006). Despite recent progress (Waselle et al., 2003), however, little is known about the dynamic interaction of granules with the actin cytoskeleton. Knowledge of these mechanisms could be exploited to develop novel antidiabetic drugs.

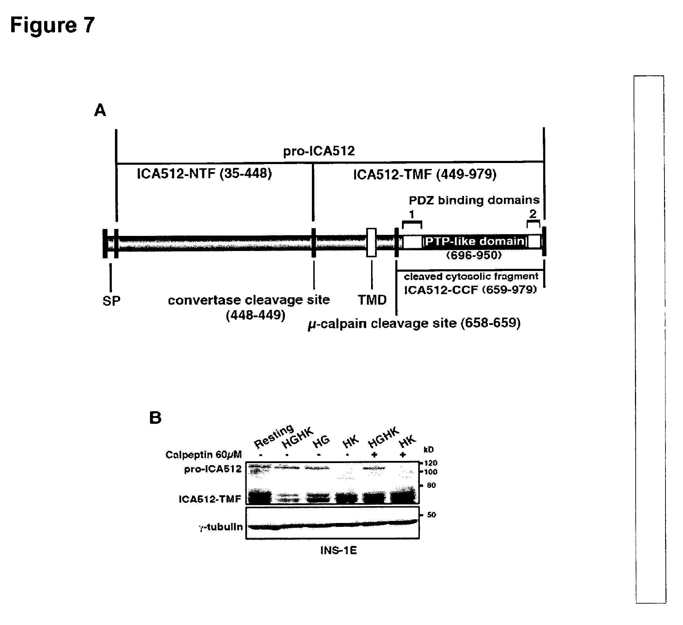

It was recently proposed that islet cell autoantigen 512/IA-2 (ICA512) tethers insulin granules to actin microfilaments (Ort et al., 2000; Ort et al., 2001; Solimena and Gerdes, 2003). ICA512 is an evolutionary conserved member of receptor protein tyrosine phosphatase (PTP) family (Lan et al., 1994; Rabin et al., 1994; Zahn et al., 2001), which lacks phosphatase activity (Drake et al., 2003) and is mostly expressed in neuroendocrine cells (Solimena et al., 1996). The pro-form of ICA512 is a glycoprotein of 110 kDa, whose ectodomain is processed at a consensus cleavage site for furin-like convertases during the maturation of secretory granules (Solimena et al., 1996) (Fig. 7A). This cleavage originates a 65 kDa transmembrane fragment (ICA512-TMF) that in the case of human ICA512 includes amino acids 449-979 (Fig. 7A). The cytoplasmic domain of ICA512-TMF binds to the PDZ domain of β2-syntrophin (Ort et al., 2000), which in turn interacts with cortical actin microfilaments through its binding to utrophin (Albrecht and Froehner, 2002). Glucose stimulation, on the other hand, may induce the dissociation of ICA512-TMF from β2-syntrophin by affecting the phosphorylation of the latter, hence increasing granule mobility (Ort et al., 2001).

Ca²⁺-dependent exocytosis of secretory granules triggers the transient insertion of ICA512-TMF into the plasma membrane and its intracellular cleavage by the Ca²⁺-activated protease calpain-1 (Ort et al., 2001). The resulting cytosolic fragment of ICA512 (ICA512-CCF, amino acids 659-979; Fig. 7A) contains the inactive PTP module flanked by the two PDZ domain interacting regions and enhances granule biogenesis by acting as a decoy phosphatase. Specifically, ICA512-CCF is targeted to the nucleus (Trajkovski et al., 2004) where it binds to tyrosine phosphorylated signal transducer and activator of transcription (STAT)-5 and -3 (Mziaut et al., 2006), thereby preventing their dephosphorylation by conventional tyrosine phosphatases and enhancing their transcription of insulin and other granule genes. Consistent with ICA512 having a role in granule biogenesis and trafficking, knockout of its gene in mice leads to reduced insulin secretion and glucose intolerance (Saeki et al., 2002), whereas its overexpression in mouse insulinoma MIN6 cells increases the number of granules, the content and half-life of insulin as well as its stimulated secretion (Harashima et al., 2005). Valuable approaches to treat diabetes have been described in WO2006/029728 A1. This patent application has for the first time linked that C-terminal fragments of ICA512 are targeted to the nucleus of pancreatic β-cells and stimulate the expression of peptide hormones such as insulin. Due to the ever increasing number of patients suffering from diabetes, it would, however, be valuable to develop alternative approaches to medicaments useful in the treatment of diabetes.

Therefore, the technical problem underlying the present invention the provision of additional compounds useful as ingredients or in the development of medicaments for the treatment of diabetes.

The solution to this technical problem is achieved by providing the embodiments characterized in the claims.

Accordingly, the present invention relates to a pharmaceutical composition comprising
(a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN;
(b) a compound promoting the presence or activity of a protease that cleaves the C-terminus of ICA512, ICA512-TMF or ICA512-CCF or PHOGRIN;
(c) a compound promoting the presence or activity of a serine/threonine kinase which phosphorylates β2-syntrophin on serine residue 95;
(d) a compound interfering with the presence or activity of a serine/threonine phosphatase which dephosphorylates β2-syntrophin on serine residue 95;
(e) a compound promoting the presence or activity of a serine/threonine phosphatase which dephosphorylates β2-syntrophin on serine residue 110; or
(f) a compound interfering with the presence or activity of a serine/threonine kinase which phosphorylates β2-syntrophin on serine residue 110.

In accordance with the present invention, the term "pharmaceutical composition" relates to a composition for administration to a patient, preferably a human patient. The pharmaceutical composition of the invention comprises the compounds recited above, alone or in combination. It may, optionally, comprise further molecules capable of altering the characteristics of the compounds of the invention thereby, for example, stabilizing, modulating and/or activating their function. The composition may be in solid, liquid or gaseous form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. The term "parenteral" as used herein refers to modes of administration, which include intravenous, intramuscular, intraperitoneal, intrastemal, subcutaneous and intraarticular injection and infusion. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

Additionally, a nucleic acid molecule and preferably a DNA molecule encoding said compound(s) (wherein the term "compound" also refers to the mentioned proteins as well as the fragments or derivatives or modifications thereof) may be formulated into a pharmaceutical composition. The nucleic acid molecule is eventually to be introduced into the desired cells. Appropriate formulations include those wherein 10⁶ to 10¹² copies of the DNA molecule, advantageously comprised in an appropriate vector are administered per dose. The vector may be, for example, a phage, plasmid, viral or retroviral vector. Retroviral vectors may be replication competent or replication defective. In the latter case, viral propagation generally will occur only in complementing host/cells.

In accordance with this specification,"ICA512" refers to the mature transmembrane protein "Islet Cell Autoantigen 512" also referred to as "IA-2" or "protein tyrosine phosphatase N"(PTPN) having a level of identity of at least 30%, preferably at least about 35%, more preferred at least 50% and most preferred at least 75% such as 85% with the amino acid sequence displayed in SEQ ID NO.: 1 which reflects the human ICA512 sequence. This sequence is also available as entry # Q16849 from the NCBI protein database. This amino acid sequence, in accordance with the invention, is preferably encoded by the nucleotide sequence of SEQ ID NO.: 2 which is available as entry # L18983 from the NCBI nucleotide database. It is to be understood that the various ICA512 proteins having different degrees of identity and subsumed under the term "ICA512" solely denote naturally occurring proteins from other species that may, in the scientific literature, bear a name different from ICA512. Thus, a mouse ICA512 protein, (NCBI entry #148721) has been identified that bears 86.7% identity with the human sequence at the amino acid level. Similarly, Rattus norvegicus (rat) (NCBI entry #Q63259, 86.82% identity), Danio rerio (zebrafish) (NCBI partial sequence # AF190144.1, 75.97% identity), and C. elegans (NCBI entry # CAB52188, 38.10% identity) proteins have been identified. Further, the invention explicitly includes allelic variants from humans. All these proteins have the following functions in common: 1) they all include a single atypical protein tyrosine phosphatase (PTP) domain in their cytosolic tail that lacks phosphatase activity towards conventional tyrosine phosphatase substrates, 2) they are most abundant in neuroendocrine cells; 3) they are enriched in secretory granules (SGs). They may all be used in accordance with the method of the present invention.

The term "ICA512" also includes derivatives of ICA512 having ICA512 function in accordance with the present invention. The term "derivative of ICA512 having ICA512 function" relates to a protein (the term "protein" in connection with this specification having the same meaning as the term "polypeptide") that is derived from naturally occurring ICA512, e.g. by post-translational modifications that are effected by eukaryotic cells other than the cells the protein naturally occurs in or by lack of post-translational modifications (in case the protein is produced in prokaryotic organisms), by chemical modification, by peptidomimetics etc., to name a few important options. In all these options, the ICA512 function is retained. "ICA512 function" means, in accordance with this invention, to include a PTP like-domain, the capability to be cleaved by µ-calpain, also known as calpain-1, and to sequester a C-terminal portion that retains the capacity to be targeted to the nucleus, and/or to bind proteins of the PIAS (Protein Inhibitor of Activated STAT) family, and/or to instigate or enhance the expression of genes encoding peptide-hormones by modulating the activity of transcription factors, such as Signal Transducer and Activator of Transcription (STATs) as described in WO2006/029778. Included within the term "derivative" are also/thus molecules that have the above identified biological function and that are (semi)synthetically or recombinantly produced without assuming first the naturally occurring structure of ICA512 which is then subsequently modified, e.g. by post-translational modifications that are effected by eukaryotic cells other than the cells the protein naturally occurs in. This is the case if the derivative is a peptidomimetic. Alternatively, the term includes fragments of the full-length protein that are modified as outlined above and that retain the function specified above. Modifications referred to above (in particular post-translational and chemical) include: acetylation, acylation, amidation, ADP-ribosylation, glycosylation, GPI anchor formation, covalent attachment of a lipid or lipid derivative, methylation, myristylation, pegylation, prenylation, phosphorylation, ubiqutination and sumoylation. Derivatives of the above protein include further fusion proteins wherein the two fusion partner may, e.g., be fused via a (flexible) linker such as a glycine linker or a gly-ser linker or wherein the protein is fused to a tag such as His-tag, FLAG-tag, myc-tag etc.

The term "promoting the presence" in general means that a certain level of the protease or the serine/threonine kinase or the serine/threonine phosphatases, all as mentioned above, is maintained in the cells being the target of the pharmaceutical composition of the invention which are preferably pancreatic β-cells and most preferably human pancreatic β-cells. Accordingly, the invention envisages that, in one alternative, an existing level of any of the above recited enzymes in the cell is maintained or essentially maintained, such as maintained to at least 70%, preferably to at least 80%, more preferred to at least 90% and most preferred to at least 95% such as 100%. Important options to maintain an existing level of said enzymes are described in connection with preferred embodiments of the method of the invention further below. In addition, if the cell naturally does not produce any of said enzymes for example due to genetic defects, production may be stimulated by introducing a vector capable of expressing at least one of said enzymes into said cell. Important options how the level of said compounds may be enhanced are discussed in connection with preferred embodiment further below. Further to the above, it is to be understood that the term "enhancing" in connection with the production of any of the enzymes as recited above relates alternatively or both to the enhancement of levels of any of said enzymes already existing in the cytoplasm or in the nucleus and to the *de novo* production thereof in a cell. The level of said compounds required in a cell that is necessary for stimulating expression may vary from cell type to cell type. In any case and as regards pancreatic β-cells, it is preferred that said cells are in a stimulated stage, i.e. secrete said insulin. In the case of pancreatic β-cells, stimulation may be effected by contact with or addition of glucose. It is particularly preferred that the promotion of the presence of either or all of said enzymes includes or corresponds to the promotion of the expression thereof. Also in the other embodiments described in accordance with this invention, "expression" is a preferred embodiment of "presence".

The term "expression" is well known in the art and relates to the transcription and/or translation of a gene, resulting in the generation of a messenger RNA and, optionally, subsequently, its translation into a protein. In accordance with the present invention, it is preferred that in particular the transcription of the gene is stimulated.
The term "promoting the [...] activity" refers, as was explained in conjunction with the term "promotion of the presence" to the essential maintenance or an increase of the activity.

An increase of the activity may be affected, for example, by site-directed mutagenesis and the subsequent testing for an increased activity.

The term "ICA512-TMF" relates to the mature transmembrane form of ICA512. More specifically, ICA512 is released from a precursor, pro-ICA512. Pro-ICA512 is a glycoprotein of 110 kD synthesized in a glucose-regulated fashion. Processing of its ectodomain along the secretory pathway by a furin-like protease converts pro-ICA512 into its mature transmembrane form of 65 kD (ICA512-TMF), which is enriched on secretary granules (SG).

The term "ICA512-CCF" refers to a cleavage product obtained from ICA512-TMF by the catalytic action of a µ-calpain. As mentioned above, this cytosolic fragment contains the inactive PTP domain flanked by the two PDZ interaction regions. The activity of µ-calpain has been described in detail in, for example, WO2006/029728.

The various derivatives and modifications of ICA512 described herein above and also encompassed by the present invention apply mutatis mutandis to ICA512-TMF and ICA512-CCF molecules.

The term "PHOGRIN", also known as IA-2beta or protein tyrosine phosphatase, receptor type, N polypeptide 2 (ptprn2), refers to a paralogue of ICA512 (Kawasaki et al, 1996; Chiang and Flanagan, 1996; Lu et al, 1996). Human PHOGRIN is encoded by SEQ ID NO: 6 and can be retrieved from the NCBI nucleotide database under the entry number NM_002847. The amino acid sequence of human PHOGRIN corresponds to SEQ ID NO: 7 and can be retrieved from the NCBI protein database under the entry number AAC50472. Like ICA512, PHOGRIN lacks phosphatase activity and is mostly expressed in neuroendocrine cells, including neurons and pancreatic β-cells. Comprised by the present invention are also derivatives and modifications of PHOGRIN wherein the derivatives and modifications of PHOGRIN are defined in an analogous manner with regard to the derivatives and modifications described for ICA512 herein above. The term "PHOGRIN" includes in addition molecules corresponding in sequence to ICA512-TMF and ICA512-CCF, in accordance with this invention.

The term "β2-syntrophin" refers to a protein having a level of identity of at least 30%, preferably at least about 35%, more preferred at least 50% and most preferred and least 75% such as 85% with the amino acid sequence displayed in SEQ ID NO.: 4 which reflects the human β2-syntrophin sequence. This sequence is also available as entry Q13425 in the NCBI protein database, whereas e.g. the corresponding mouse sequence (SEQ ID NO: 5) has the entry number Q61235. The amino acid sequence, in accordance with the present invention, is preferably encoded by the nucleotide sequence of SEQ ID NO.: 3 which is available as entry NM_006750 from the NCBI nucleotide database. It is to be understood that the various β2-syntrophin proteins having different degrees of identity and subsumed under the term "β2-syntrophin" solely denote naturally occurring proteins from other species that may, in the scientific literature, bear a name different from β2-syntrophin. β2-syntrophin is a cytosolic adaptor protein that interacts through its PDZ domain to a variety of receptor and signaling molecules and links them to the actin cytoskeleton via the association of its C-terminal domain to the dystrophin related protein utrophin (Albrecht and Froehner 2002). They may all be used in accordance with the method of the present invention.

The term "devoid of the C-terminus" of either of the above-recited molecules ICA512, ICA512-TMF, ICA512-CCF or PHOGRIN relates to molecules wherein the C-terminus has been cleaved by proteases. The C-terminal fragment removed includes, at least in part, the functional domain involved in the binding to the PDZ domain of β2-syntrophin of these molecules. It is important to note that this C-terminus is different from ICA512-CCF which is itself a C-terminal fragment of ICA512-TMF and ICA512, respectively, and obtained by the cleavage of the well-known enzyme µ-calpain.

It has surprisingly been found in accordance with the present invention that various further approaches over those described in WO2006/029728 are available that allow the provision of compounds useful in the treatment of diabetes or useful in the development of lead compounds for the treatment of diabetes. The common inventive principle underlying all the approaches of the present invention is that all of the above-mentioned ingredients of the pharmaceutical composition of the invention directly induce the secretion of insulin from secretary granules without the *de novo* synthesis of insulin. More specifically, in accordance with the present invention, it could be demonstrated that the ICA512-TMF/β2-syntrophin complex regulates the turnover of insulin granules by restraining their motility and exocytosis. In other terms, the experiments underlying the present invention surprisingly established that intracellular cleavage of ICA512-TMF, in addition to granule biogenesis, also regulates insulin secretion. As was further found in accordance with the invention, dissociation of the ICA512-TMF/β2-syntrophin complex following granule exocytosis is further enhanced by the Ca²⁺/calpain-1-induced generation of ICA512-CCF, which independently binds to ICA512-TMF and β2-syntrophin. On the one hand, removal of the C-terminal PDZ binding motif is envisaged in accordance with the invention to further amplify insulin secretion by skewing the interaction of ICA512-CCF towards ICA512-TMF. C-terminal cleavage of each ICA512-TMF molecule in the granule membrane, on the other hand, is envisaged in accordance with the invention to progressively reduce the ability of a vesicle to anchor to the cortical actin cytoskeleton and thus its residence time near the plasma membrane. Such aging process could explain the preferential release of younger versus older granules (Duncan et al., 2003; Solimena and Gerdes, 2003), the latter being progressively displaced by the former from the cell cortex (Duncan et al., 2003). Notably, ICA512 is the only identified protein besides β-tubulin that is cleaved at its very C-terminal cytosolic end. In other terms, following the dissociation of β2-syntrophin from ICA512-TMF or ICA512-CCF the secretory granules move more freely and thus a contact with the cell membrane becomes more likely. Upon the contact of the secretory granules with the cell membrane, a fusion of the secretory granules with the cell membrane becomes possible which results in a release of insulin contained in the secretory granules. Such a fusion will only occur if the cellular calcium channels are open which will in turn only be the case if the glucose concentration in the blood is enhanced. The various proteins or protein fragments mentioned above, all devoid of their C-terminus which is cleaved of by the novel protease do not comprise a functional domain involved in the binding to the PDZ domain of β2-syntrophin. As a consequence the ICA512/β2-syntrophin complex will not be formed or not be maintained with the consequence of an enhancement of insulin secretion. The mimetic of the C-terminus of ICA512 or of PHOGRIN or the mentioned cleavage products thereof function in a similar manner, namely by competing with ICA512/PHOGRIN for binding to β2-syntrophin and thus not allowing the ICA512/β2-syntrophin complex to form or driving it to dissociate.

The PDZ domain of β2-syntrophin is flanked on both sides by a split pleckstrin homology domain, which in the case of β-syntrophin binds to PI(4,5)P2. Generation of PI(4,5)P2 at the plasma membrane is a critical step for the priming and release of secretory granules (Gong et al., 2005; Hay et al., 1995). Binding of PI(4,5)P2 to the pleckstrin-homology domain of β2-syntrophin is envisaged in accordance with the present invention to affect the embedded PDZ domain, and thus represents an additional means to modulate the interaction of ICA512 and the granules with cortical actin.

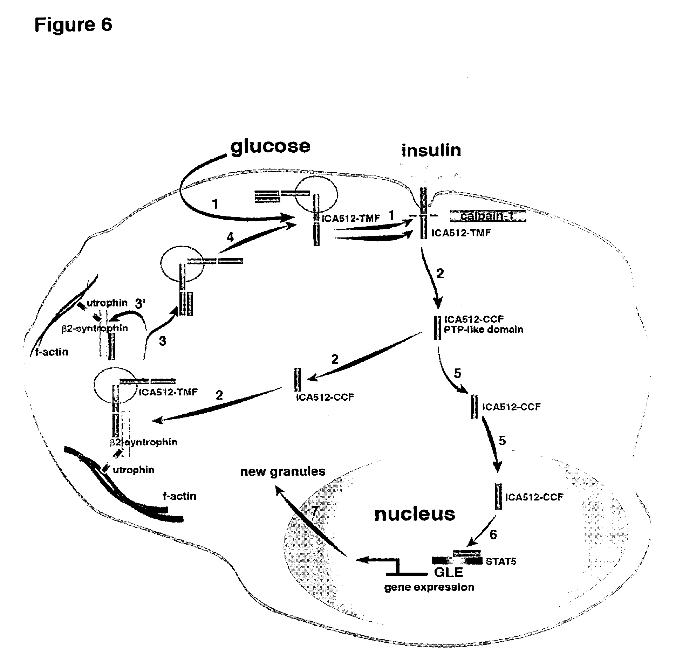

Thus, whereas the applicant does not wish to be bound by any scientific theory, in accordance with the present invention, a model is proposed whereby intracellular cleavage of ICA512-TMF in proportion to granule exocytosis originates a short feedback loop that amplifies insulin secretion by increasing the mobility of granules, and hence their probability to encounter membrane sites competent for exocytosis (Fig. 6). Progressive depletion of granules, in turn, enhances the probability of ICA512-CCF to reach the cell nucleus, thereby promoting the transcription of insulin and other granule genes, and thus granule biogenesis. Further quantitative elaborations of these processes may allow the formulation of algorithms that illustrate how β-cells count granule stores and exocytotic events to dynamically regulate granule biogenesis and glucose homeostasis.

The above discussed experimental data and conclusions therefrom allow in accordance with the present invention to provide all the various novel ingredients of the pharmaceutical composition disclosed in accordance with the present invention. Specifically, since ICA512, ICA512-TMF, ICA512-CCF or PHOGRIN, all devoid of the C-terminus cleaved by the protease such that at least a portion of the PDZ binding/interaction domain is removed, lead to a increased insulin secretion from secretory granules, those compounds are of immediate interest as a pharmaceutical.

Similarly, the promotion of the presence, preferably expression, or of the activity of a protease that cleaves the C-terminus of any of the above-mentioned proteins will in a similar manner lead to a promotion of insulin secretion from secretary granules. In an alternative manner, the promotion of the presence or activity of a serine/threonine kinase phosphorylating β2-synthrophin serine residue 95 will break up the interaction of β2-synthrophin with ICA512-TMF or ICA512-CCF. It is of note that the prior art does not teach or suggest which residue of β2-synthrophin would in fact be phosphorylated. Insofar, the finding that the serine residue 95 is phosphorylated by the serine/threonine kinase must be considered as non-obvious.

An analogous result is achieved by interfering with the presence or activity of a serine/threonine phosphatase which dephosphorylates β2-synthrophin on serine residue 95.

Further, insulin secretion may be promoted by promoting the presence or activity of a serine/threonine phosphatase, which dephosphorylates β2-synthrophin on serine residue 110 or by interfering with the expression or activity of a serine/threonine kinase which phosphorylates β2-synthrophin on serine residue 110. Again, that promotion or interference will lead to an interference of the binding of β2-synthrophin to ICA512-TMF or ICA512-CCF and thus to the dissociation of those molecules which in turn results in the secretion of insulin. Similar to the involvement of serine residue 95, there has been no teaching or suggestion in the prior art that the phosphorylation status of serine residue 110 can be employed in the triggering of insulin secretion. Now that this specification has described the serine residues that need to be phosphorylated or dephosphorylated in order to promote insulin secretion, the enzymes (in addition to CDK5, see below) that effect the phophorylation/dephosphorylation can be identified without further ado by screenings with specific inhibitors and activators pharmacological modulators or RNA interference strategies.

The pharmaceutical composition of the present invention has an enormous advantage over conventional prior art methods that rely on the promotion of insulin secretion. Namely, whereas these prior art methods rely on the promotion of insulin secretion independent of the glucose concentration in the blood and are thus potentially harmful to the patient (in particular in cases where insulin secretion is promoted in patients with lowered glucose concentrations in the blood), the pharmaceutical composition of the present invention secures that insulin secretion is only promoted as a result of increased glucose concentrations in the blood.

Further, the present invention relates to the use of
(a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN;
(b) a compound promoting the presence or activity of a protease that cleaves the C-terminus of ICA512, ICA512-TMF, ICA512-CCF or PHOGRIN;
(c) a compound promoting the presence or activity of a serine/threonine kinase which phosphorylates β2-syntrophin on serine residue 95;
(d) a compound interfering with the presence or activity of a. serine/threonine phosphatase which dephosphorylates β2-syntrophin on serine residue 95;
(e) a compound promoting the presence or activity of a serine/threonine phosphatase which dephosphorylates β2-syntrophin on serine residue 110; or
(f) a compound interfering with the presence or activity of a serine/threonine kinase which phosphorylates β2-syntrophin on serine residue 110
for the preparation of a pharmaceutical composition for the treatment of diabetes.

As mentioned herein above, the pharmaceutical composition to be prepared in accordance with the invention is particularly useful in the treatment of diabetes.

In a preferred embodiment of the pharmaceutical composition of the invention or the use of the invention, said mimetic is a peptidomimetic.
The chemical nature of peptidomimetics is briefly described elsewhere in this specification and is well known in the art. The use of peptidomimetics as compared to other mimetics have some particular advantages. For instance, their conformationally restrained structure allows to minimize binding to non-target receptors and enhance the activity at the desired targets. Through the addition of hydrophobic residues and/or replacement of amide bonds the transport of peptidomimetics cellular membranes can be improved. Furthermore peptidomimetics such as isosters, retro-inverso peptides, cyclic peptides and non-peptidomimetics are less susceptible to degradation by peptidases and other enzymes.

In a further preferred embodiment of the pharmaceutical composition of the invention or the use of the invention, the compound promoting the presence or activity of said protease that cleaves the C-terminus of ICA512, ICA512-TMF, ICA512-CCF or PHOGRIN is an expression vector containing the coding region of said protease in expressible form.

This embodiment of the present invention may be put into practice according to conventional techniques of molecular biology including medical applications thereof such as gene therapy, and requires, optionally after cloning the gene(s) encoding the above protease and insertion of the gene or the coding sequence thereof into a vector, preferably an expression vector, the introduction into a cell. The introduction is accompanied by the expression of said protease from an expression vector. Examples of expression vectors useful in the method of the invention are described herein below.

Advantageously and as stated above, the polynucleotide/coding sequence is introduced into a vector and thus operatively linked to expression control sequences allowing expression in prokaryotic or eukaryotic cells or isolated fractions thereof.
Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells (a less preferred embodiment) comprise, e.g., the *lac, trp* or *tac* promoter, the lacUV5 or the trp promotor in *E. coli,* and examples for regulatory elements permitting expression in eukaryotic host cells (the more preferred embodiment) are the *AOX1* or *GAL1* promoter in yeast or the CMV-(Cytomegalovirus), SV40-, RSV-promoter (Rous sarcoma virus), the gai10 promoter, human elongation factor 1α-promoter, CMV enhancer, CaM-kinase promoter, the Autographa californica multiple nuclear polyhedrosis virus (AcMNPV) polyhedral promoter or the SV40-enhancer or a globin intron in mammalian and other animal cells. Preferred promoters are the natural promoter of the above compounds as well as promoters allowing the tissue specific expression of those compounds such as promoters active in pancreatic β-cells or neuronal cells. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (Invitrogen, as used, inter alia, in the appended examples), pSPORT1 (GIBCO BRL) or pGEMHE (Promega), or prokaryotic expression vectors, such as lambda gt11, pJOE, the pBBR1-MCS - series, pJB861, pBSMuL, pBC2, pUCPKS, pTACT1 or, preferably, the pET vector (Novagen). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (2001) N.Y. and Ausubel, Current Protocols in Molecular Biology, Green Publishing Associates and Wiley Interscience, N.Y. (2001). Alternatively, the polynucleotides or vectors can be reconstituted into liposomes for delivery to target cells.

Preferably, the a vector is a plasmid, cosmid, virus, bacteriophage or another vector used e.g. conventionally in genetic engineering.
Additional non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pA0815, pPIC9K and pPIC3.5K (all Intvitrogen). The polynucleotide referred to above may also be inserted into vectors such that a translational fusion with another polynucleotide is generated. The other polynucleotide may encode a protein which may e.g. increase the solubility and/or facilitate the purifcation of the fusion protein. Non-limiting examples include pET32, pET41, pET43. The vectors may also contain an additional expressible polynucleotide coding for one or more chaperones to facilitate correct protein folding. Suitable bacterial expression hosts comprise e.g. strains derived from BL21 (such as BL21(DE3), BL21(DE3)PlysS, BL21(DE3)RIL, BL21(DE3)PRARE) or Rosetta®.
For vector modification techniques, see Sambrook and Russel (2001), loc. cit. Generally, vectors can contain one or more origins of replication (ori) and inheritance systems for cloning or expression, one or more markers for selection in the host, e.g., antibiotic resistance, and one or more expression cassettes. Suitable origins of replication (ori) include, for example, the Col E1, the SV40 viral and the M 13 origins of replication.
The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the polynucleotide of the invention is operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector of the invention comprises a selectable marker. Examples of selectable markers include neomycin, ampicillin, hygromycin, kanamycin, chloramphenicol resistance and the like. Specifically-designed vectors allow the shuttling of DNA between different hosts, such as bacteria- fungal cells or bacteria-animal cells (e. g. the Gateway® system available at Invitrogen).
An expression vector according to this invention is capable of directing the replication, and the expression, of the polynucleotide and encoded enzyme. Suitable expression vectors which comprise the described regulatory elements are known in the art such as
The nucleic acid molecules encoding the protease as described herein above may be designed for direct introduction or for introduction via liposomes, phage vectors or viral vectors (e.g. adenoviral, retroviral) into the cell. Additionally, baculoviral systems or systems based on vaccinia virus or Semliki Forest Virus can be used as eukaryotic expression system for the nucleic acid molecules of the invention.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression and secretion, the polynucleotide of interest may be ligated between e.g. the PelB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al.1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli,* Streptomyces and *Salmonella typhimurium* cells; fungal cells, such as yeast cells; insect cells such as Drosophila S2 and Spodoptera Sf9 cells or High Five (H5) cells; animal cells such as CHO, COS, 293 and Bowes melanoma cells; and plant cells. Appropriate culture mediums and conditions for the above-described host cells are known in the art.

In a further preferred embodiment of the pharmaceutical composition of the invention or the use of the invention, the compound promoting the presence or activity of said serine/threonine kinase is an expression vector containing the coding region of said kinase in expressible form.
In another preferred embodiment of the pharmaceutical composition of the invention or the use of the invention, the compound promoting the presence or activity of said serine/threonine phosphatase is an expression vector containing the coding region of said phosphatase in expressible form.

Regarding the employment of suitable vectors for expession of said kinase or said phosphatase, the same considerations that have been discussed herein above in connection with the expression of the protease apply.

In a different embodiment, the present invention relates to a method of enhancing insulin secretion from pancreatic β-cells comprising promoting in said β-cells the presence or activity of
(a) (aa) ICA512, ICA512-TMF, ICA512-CCF or PHOGRIN; or
   (ab) ICA512, ICA512-TMF or ICA512-CCF, each devoid of the C-terminus or PHOGRIN devoid of the C-terminus;
(b) a compound promoting the presence or activity of a protease that cleaves the C-terminus of ICA512, ICA512-TMF, ICA512-CCF or PHOGRIN;
(c) a compound promoting the presence or activity of serine/threonine kinase which phosphorylates β2-syntrophin on serine residue 95;
(d) a compound interfering with the presence or activity of serine/threonine phosphatase which dephosphorylates ß2-syntrophin on serine residue 95;
(e) a compound promoting the presence or activity of a phosphatase which dephosphorylates β2-syntrophin on serine residue 110; or
(f) a compound interfering with the presence or activity of a serine/threonine kinase which phosphorylates β2-syntrophin on serine residue 110.

In this embodiment of the invention, the same compounds that are also useful in the pharmaceutical composition of the present invention may be employed. In addition, the presence or activity of naturally occurring compounds ICA512, ICA512-TMF, ICA512-CCF or PHOGRIN or derivatives or modifications thereof as described herein above, may be promoted. In accordance with the present invention, it was surprisingly found, as has been discussed in detail above, that these compounds can also induce insulin secretion by a different mechanism than was described in WO 2006/029728. In other terms, these compounds promote insulin secretion without the prior induction of hormone synthesis in the nucleus of the cell.

This method of the present invention as well as further methods for enhancing insulin secretion from pancreatic β-cells can be carried out *in vivo, ex vivo* or *in vitro.*

In a preferred embodiment of this embodiment of the invention the promotion comprises the stable or transient expression from an exogenously introduced vector of said
(a) (aa) ICA512, ICA512-TMF, ICA512-CCF or PHOGRIN; or
   (ab) ICA512, ICA512-TMF or ICA512-CCF, each devoid of the C-terminus or PHOGRIN devoid of the C-terminus;
(b) compound promoting the presence or activity of a protease that cleaves the C-terminus of ICA512, ICA512-TMF, ICA512-CCF or PHOGRIN;
(c) compound promoting the presence or activity of a serine/threonine kinase which phosphorylates β2-syntrophin on serine residue 95;
(d) compound interfering with the expression or activity of a serine/threonine phosphatases which dephosphorylates β2-syntrophin on serine residue 95;
(e) compound promoting the presence or activity of a serine/threonine phosphatases which dephosphorylates β2-syntrophin on serine residue 110; or
(f) compound interfering with the expression or activity of a serine/threonine kinase which phosphorylates β2-syntrophin on serine residue 110.

Again, as regards the appropriate vector for expression of these proteins, sufficient guidance for the skilled person may be found in the preceding paragraphs and in the documents recited in the preceding paragraphs.

In a preferred embodiment of the pharmaceutical composition, use or method of the invention the promotion comprises reducing degradation or enhancing stability in said β-cells of said
(a) (aa) ICA512, ICA512-TMF, ICA512-CCF or PHOGRIN; or
   (ab) ICA512, ICA512-TMF or ICA512-CCF, each devoid of the C-terminus or PHOGRIN devoid of the C-terminus;
(b) compound promoting the presence or activity of a protease that cleaves the C-terminus of ICA512, ICA512-TMF, ICA512-CCF or PHOGRIN;
(c) compound promoting the presence or activity of a serine/threonine kinase which phosphorylates ß2-syntrophin on serine residue 95;
(d) compound interfering with the presence or activity of a serine/threonine phosphatase which dephosphorylates ß2-syntrophin on serine residue 95;
(e) compound promoting the presence or activity of a serine/threonine phosphatase which dephosphorylates β2-syntrophin on serine residue 110; or said
(f) compound interfering with the presence or activity of a serine/threonine kinase which phosphorylates ß2-syntrophin on serine residue 110.

Reduction of the degradation comprises methods wherein proteins degrading in particular any of the above recited proteinaceous compounds are inhibited such as by RNAi or antisense oligonucleotides. In an alternative embodiment, antibodies to said degrading proteins or antibody fragments or derivatives such as Fab, F(ab')₂ or scFv fragments may be employed; see, for example, Harlow and Lane, "Antibodies, A laboratory manual", CSH Press 1988, Cold Spring Harbor. The goal of this embodiment of the invention is to reduce unspecific degradation, not degradation by µ-calpain, which is, in contrast, desired in order to generate the ICA512-CCF fragment or degradation by the novel protease which specifically cleaves ICA512 and fragments thereof at the C-terminus. Preferably, the enhancement and reduction, respectively, amount to at least 25% of the amount of the compounds, preferably at least 50%, more preferred at least 75% and most preferred at least 90%. In the case of enhancement of the stability, an increase of more than 100% is advantageously envisaged. One option to enhance stability is the generation of a peptidomimetic.

In a particularly preferred embodiment of the pharmaceutical composition of the invention, the use of the invention or the method of the invention the compound interfering with the expression or activity of said phosphatase or of said kinase is, as indicated for some of the particularly preferred embodiments herein above, an siRNA, an shRNA, a ribozyme, a peptide aptamer, a nucleic acid based aptamer, a small molecule or an antibody.

For therapeutic uses, the RNA inactivation by antisense molecules or by ribozymes appears to be implementable. Both classes of compounds can be synthesized chemically or produced in conjunction with a promoter by biological expression *in vitro* or even *in vivo.*

Small interfering RNA (siRNA), sometimes known as short interfering RNA or silencing RNA, are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

SiRNAs have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. SiRNAs can also be exogenously (artificially) introduced into cells to bring about the specific knockdown of a gene of interest. Essentially any gene of which the sequence is known can thus be targeted based on sequence complementarity with an appropriately tailored siRNA.
The double-stranded RNA molecule or a metabolic processing product thereof is capable of mediating target-specific nucleic acid modifications, particularly RNA interference and/or DNA methylation. Preferably at least one RNA strand has a 5'-and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general any RNA molecule suitable to act as siRNA is envisioned in the present invention.
The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

RNAs of the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK). Most conveniently, siRNAs are obtained from commercial RNA oligo synthesis suppliers, which sell RNA-synthesis products of different quality and costs. In general, the RNAs applicable in the present invention are not too difficult to synthesize and are readily provided in a quality suitable for RNAi.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome.
Examples of well-characterized small self-cleaving RNAs are the hammerhead, hairpin, hepatitis delta virus, and in vitro-selected lead-dependent ribozymes. The organization of these small catalysts is contrasted to that of larger ribozymes, such as the group I intron.
The principle of catalytic self-cleavage has become well established in the last 10 years. The hammerhead ribozymes are characterized best among the RNA molecules with ribozyme activity. Since it was shown that hammerhead strucutres can be integrated into heterologous RNA sequences and that ribozyme activity can thereby be transferred to these molecules, it appears that catalytic antisense sequences for almost any target sequence can be created, provided the target sequence contains a potential matching cleavage site.
The basic principle of constructing hammerhead ribozymes is as follows: An interesting region of the RNA, which contains the GUC (or CUC) triplet, is selected. Two oligonucleotide strands, each with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

Aptamers are oligonucleic acid or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications.

More specifically, aptamers can be classified as DNA or RNA aptamers or peptide aptamers. Whereas the former consist of (usually short) strands of oligonucleotides. The latter consist of a short variable peptide domain, attached at both ends to a protein scaffold.

Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers are proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility properties. Currently, the bacterial protein Thioredoxin-A is the most used scaffold protein, the variable loop being inserted within the reducing active site, which is a -Cys-Gly-Pro-Cys- loop in the wild protein, the two cysteins lateral chains being able to form a disulfide bridge. Peptide aptamer selection can be made using different systems, but the most used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications.

Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, etc. are available to scientists with which the half-life of aptamers easily can be increased to the day or even week time scale.

A recent development is the combination of an aptamer recognizing a small compound with a hammerhead ribozyme. The conformational change induced in the aptamer upon binding the target molecule, is supposed to regulate the catalytic function of the ribozyme.

An antibody comprised in a pharmaceutical composition according to the present invention specifically binds to a compound which unfavourably influences the stability or degradation or presence of the naturally occurring compounds as described in detail herein above.
The antibody of the present invention can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. These antibodies can be used, for example, for the immunoprecipitation, affinity purification and immunolocalization of the compounds it binds as well as for the monitoring of the presence and amount of such polypeptides, for example, in cultures of recombinant prokaryotes or eukaryotic cells or organisms. Furthermore, the antibodies are envisaged to be used in the pharmaceutical compositions of the invention.
The antibody of the invention also includes embodiments such as chimeric, single chain and humanized antibodies, as well as antibody fragments, like, inter alia, Fab fragments. Antibody fragments or derivatives further comprise F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for the compounds described above. Also, transgenic animals may be used to express humanized antibodies specific for these compounds. Most preferably, the antibody of this invention is a monoclonal antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of the compounds described above (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, amongst others, viruses or plasmid vectors. Once the antibody according to the present invention has been obtained by the methods of the invention, the antibody itself or the DNA encoding it can be sequenced providing for the information to recombinantly produce the antibody of the invention in small or large scale. Methods of the production of a recombinant antibody are known to the person skilled in the art.
The antibody described in the context of the invention is capable to specifically bind/interact with the compounds described above. The term "specifically binding/interacting with" as used in accordance with the present invention means that the antibody does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest and thus to be antibodies in accordance with this invention. Corresponding methods are described e.g. in Harlow and Lane, 1988 and 1999, loc cit.

A small molecule according to the present invention can be organic or inorganic and has a molecular weight of up to 2000 Daltons, preferably not more than 1000 Daltons, most preferably not more than 800 Daltons.

Further, the present invention relates to a method of identifying a compound useful in the enhancement of insulin secretion from pancreatic β-cells or useful as a lead compound for developing a compound useful in the enhancement of insulin secretion from pancreatic β-cells comprising contacting a compound or a plurality of compounds suspected to be useful in the enhancement of insulin secretion from pancreatic β-cells or useful as a lead compound for developing a compound useful in the enhancement of insulin secretion from pancreatic β-cells with a mixture of (a) ICA512, ICA512-TMF, ICA512-CCF and/or PHOGRIN; and (b) β2-syntrophin and/or the PDZ domain of β2-syntrophin *in vitro* under conditions that would allow binding of ICA512, ICA512-TMF, ICA512-CCF and/or PHOGRIN to β2-syntrophin and/or the PDZ domain of β2-syntrophin in the absence of the compound or the plurality of compounds and assessing binding of ICA512, ICA512-TMF, ICA512-CCF and/or PHOGRIN to β2-syntrophin and/or the PDZ domain of β2-syntrophin wherein an interference of binding of ICA512, ICA512-TMF, ICA512-CCF and/or PHOGRIN to β2-syntrophin and/or the PDZ domain of β2-syntrophin in the presence of said compound or said plurality of compounds is indicative of said compound or at least one member of said plurality of compounds being useful in the enhancement of insulin secretion from pancreatic β-cells or useful as a lead compound for developing a compound useful in the enhancement of insulin secretion from pancreatic β-cells.

Contacting of the test compound(s) will be done, for example, under conditions that typically or preferably resemble or are physiological conditions such as conditions achieved by incubation in physiological saline. If more than one compound is contacted with the cell and a number of compounds tests positive, it is advantageous to repeat the experiment by incubating the cell with only one compound at the time in order to identify the most promising candidate(s). In so far, a direct relation between the compound tested and its capability to enhance secretion of insulin can be established. The inclusion of an assessment step in the absence of a test compound is useful as a control.

The assessment can be established by a variety of means. For example, the binding of β2-syntrophin to ICA512-TMF (or PHOGRIN) can be measured employing the experimental set-ups described in the appended examples.

Said method of identification can be accomplished in a high-throughput manner. Robotic equipment for that purpose is known in the art and available from a number of suppliers. Cells are normally grown in wells of plates containing arrays of 96, 384, 1536 or more wells. Transfer of the well-plates from incubators, addition of test compounds, optional washing steps as well as determining the read-out is performed in an automated fashion without requiring user interference using hundreds of thousands to millions of compounds in days to weeks.

Once a compound of interest has been identified, it can be further developed into a pharmaceutical agent such as by reducing its toxicity, prolonging shelf life and so on.

It is preferred in accordance with the present invention that said compound is a member of a library of compounds.

Consequently, in this and other embodiments pertaining to identification methods, the agent to be screened can be contained in libraries of small molecules, such as organic or inorganic small molecules which may be commercially available. In addition, libraries comprising antibodies or functional fragments or derivatives thereof (i.e. fragments or derivatives maintaining the binding specificity of the original antibody) may be used as a starting point in the screening process. Also, libraries of aptamers such as peptide aptamers might be employed. The skilled artisan is of course free to use any other starting point of desired compounds for use in the screen assays described throughout the specification. Suitable libraries are commercially available, for example from ChemBridge Corp., San Diego, USA.

The present invention relates in a further embodiment to a method of identifying a compound useful in the treatment of diabetes or useful as a lead compound for developing a compound useful in the treatment of diabetes comprising *in silico* assessing a crystal of ICA512 or PHOGRIN or a crystal of a fragment of ICA512 or PHOGRIN comprising the PTP domain for a molecular structure fitting into the binding pocket of the PTP domain.

The successful identification of a protein structure is reliant on the production of high quality crystals. This requires the use of extremely pure and homogenous protein. Purity and homogeneity can be checked by methods known in the art, e.g. electrophoresis or mass spectrometry. Furthermore, in general, the more folded the protein is, i.e. the higher the ration of folded secondary structure elements and unfolded patches, the more likely crystallization will occur. Folding of a protein can be checked e.g. with interaction studies with a known interaction partner by e.g. isothermal titration calorimetry or native gel electrophoresis methods. The proportion of secondary structure elements in the protein can be estimated using circular dichroism spectroscopy. Monodispersity, another requirement for protein crystallization, can be checked with a dynamic light scattering device. Depending on the time it will take the protein to crystallize, stability requirements may differ for each protein. The skilled person will thus understand that every protein provides its own crystallization challenge and specific protein groups, such as membrane proteins, are notoriously difficult to crystallize.

Crystal growth will occur when molecules are brought into a thermodynamically unstable state called supersaturation, achieved through the gradual removal of solvent. Nucleation, growth, and growth cessation are the three steps of crystal growth and each may require a change in experimental conditions to produce the best quality crystal.
Every protein crystallization process is hence designed and set up under a variety of conditions, adjusting experimental parameters to identify optimal crystallization conditions. These can include temperature, pH, precipitants, additives, protein concentration, and expression and purification conditions. Furthermore, the protein sample can be modified prior or during the crystallization experiment by adding modifying enzymes such as e.g. proteases which will digest the protein preferably at the respective target sites exposed to the solvent. This means that preferably parts of the protein with low organisation state and thus potentially inhibiting crystallization will be removed from the protein.
For structural determination using X-ray diffraction, not all crystals produced will diffract sufficiently. Thus, the crystallization process may have to be revisited; surface residues may have to be modified or crystallization conditions changed.

A crystallization experiment can be done in various ways. The most commonly applied methods are the hanging-drop, the sitting-drop or the micro-batch approach, all well-known to the person skilled in the art.

The surprising finding underlying this embodiment of the invention is the fact that it is the PTP domain which is involved in the interaction of, e.g. ICA512-TMF and β2-syntrophin and thus leads to the enhancement of the secretion of insulin from pancreatic β-cells. It is of note that the PTP domain has not been correlated in the art with any possible implication in insulin secretion or the treatment of diabetes.

Further, the present invention relates to a method of identifying a compound useful for enhancing insulin secretion from pancreatic β-cells or useful as a lead compound for developing a compound useful enhancing insulin secretion from pancreatic β-cells comprising *in silico* assessing a crystal of ICA512 or PHOGRIN or a crystal of a fragment of ICA512 or PHOGRIN comprising the PTP domain for a molecular structure fitting into the binding pocket of the PTP domain.

A method of identifying a compound useful for enhancing insulin secretion from pancreatic β-cells or useful as a lead compound for developing a compound useful for enhancing insulin secretion from pancreatic β-cells comprising *in silico* assessing a crystal of β2-syntrophin or a crystal of a fragment of β2-syntrophin comprising the PDZ domain for a molecular structure fitting into the binding pocket of the PDZ domain constitutes another embodiment of the present invention.

In a preferred embodiment of the method of the invention, said method further comprises *in silico* or *in vitro* assessing whether (a) the compound fitting into the binding pocket of the PTP domain interferes with binding of said domain or of ICA512, ICA-TMF, ICA-CCF or PHOGRIN with β2-syntrophin; or (b) the compound fitting into the binding pocket of the PDZ domain interferes with binding of said domain or of β2-syntrophin with ICA512, ICA-TMF, ICA-CCF or PHOGRIN.

Preferred in accordance with the present invention is further a method that additionally comprises the step of testing the compound identified *in vivo, in vitro or ex vivo* for the desired capacity.
This additional step will allow significant insights in the *in vivo* applicability of the compound that tested positive in an in vitro assay. Parameters like toxicity, half-life etc. that have to be tested in pre-clinical trials may be assessed in this step. Suitable (non-human) animals foremost include laboratory mice and rats such as available from Charles River Laboratories etc.

Additionally, the invention envisages that the method comprises the further step of improvement or of refining the pharmacological properties of the identified agent (in the following also referred to as "compound" or "drug"), by the method as described herein above, said method comprising the optionally the steps of said methods and:
(1) identification of the binding sites of the compound and the interacting protein by site-directed mutagenesis or chimeric protein studies;
(2) molecular modeling of both the binding site of the compound and the binding site of the interacting protein; and
(3) modification of the compound to improve its binding specificity for the interacting protein.
All techniques employed in the various steps of the method of the invention are conventional or can be derived by the person skilled in the art from conventional techniques without further ado. Thus, biological assays based on the herein identified nature of the interacting compound may be employed to assess the specificity or potency of the drugs wherein the increase of one or more activities of the interacting compound may be used to monitor said specificity or potency. Steps (1) and (2) can be carried out according to conventional protocols. A protocol for site directed mutagenesis (if the compound is a nucleic acid or a protein encoded by a nucleic acid) is described in Ling MM, Robinson BH. (1997) Anal. Biochem. 254: 157-178. The use of homology modeling in conjunction with site-directed mutagenesis for analysis of structure-function relationships is reviewed in Szklarz and Halpert (1997) Life Sci. 61:2507-2520. Chimeric proteins are generated by ligation of the corresponding DNA fragments via a unique restriction site using the conventional cloning techniques described in Sambrook (1989), loc. cit.. A fusion of two DNA fragments that results in a chimeric DNA fragment encoding a chimeric protein can also be generated using the gateway-system (Life technologies), a system that is based on DNA fusion by recombination. A prominent example of molecular modeling is the structure-based design of compounds binding to HIV reverse transcriptase that is reviewed in Mao, Sudbeck, Venkatachalam and Uckun (2000). Biochem. Pharmacol. 60: 1251-1265.
For example, identification of the binding site of said drug by site-directed mutagenesis and chimerical protein studies can be achieved by modifications in the primary sequence of the interacting protein that affect the drug affinity; this usually allows to precisely map the binding pocket for the drug.
As regards step (2), the following protocols may be envisaged: Once the effector site for drugs has been mapped, the precise residues interacting with different parts of the drug can be identified by combination of the information obtained from mutagenesis studies (step (1)) and computer simulations of the structure of the binding site provided that the precise three-dimensional structure of the drug is known (if not, it can be predicted by computational simulation, e.g. with the programme SWISS-MODEL (http://swissmodel.expasy.org/SWISS-MODEL.html), or the programme MODELLER (http://salilab.org/modeller/) in case a three dimensional structure of a homologous protein is available). If said drug is itself a peptide, it can be also mutated to determine which residues interact with other residues in the (poly)peptide of interest.
Finally, in step (3) the drug can be modified to improve its binding affinity or its potency and specificity. If, for instance, there are electrostatic interactions between a particular residue of the interacting protein of interest and some region of the drug molecule, the overall charge in that region can be modified to increase that particular interaction.
Identification of binding sites may be assisted by computer programs. Thus, appropriate computer programs can be used for the identification of interactive sites of a putative inhibitor and the (poly)peptide by computer assisted searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. Modifications of the drug can be produced, for example, by peptidomimetics and other inhibitors can also be identified by the synthesis of peptidomimetic combinatorial libraries through successive chemical modification and testing the resulting compounds. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of activators of the expression of PTB can be used for the design of peptidomimetic activators (Rose, Biochemistry 35 (1996), 12933-12944; Rutenber, Bioorg. Med. Chem. 4 (1996), 1545-1558).

In accordance with the above, in a preferred embodiment of the method of the invention said pharmacological properties of the identified compound is further improved or refined by peptidomimetics.

The method of the invention can further include modifying a compound identified, improved of refined by the method as described herein above as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbonic acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophylic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidinesor combinations thereof; said method optionally further comprising the steps of the above described methods.
The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The most commonly used tool to model biological systems is molecular dynamics. Virtual screening is a computational technique to find novel drug candidates. Data from virtual screening can be used to develop predictive models in order to optimize ADMET (adsorption, distribution, metabolism, elimination, toxicology) properties of the candidate molecules. Quantitative structure-activity relationships (QSAR) represent an attempt to correlate structural or property descriptors of compounds with activities. These physicochemical descriptors, which include parameters to account for hydrophobicity, topology, electronic properties, and steric effects, are determined empirically or, more recently, by computational methods. Activities used in QSAR include chemical measurements and biological assays. Computer-assisted drug design (CADD), also called computer-assisted molecular design (CAMD), represents more recent applications of computers as tools in the drug design process. In most current applications of CADD, attempts are made to find a ligand (the putative drug) that will interact favourably with a target protein. Binding of ligand to the receptor may include hydrophobic, electrostatic, and hydrogen-bonding interactions. In addition, solvation energies of the ligand and receptor site also are important because partial to complete desolvation must occur prior to binding.
Once potential drugs have been identified by the methods described above, other molecular modelling techniques may then be applied. For example, geometry optimization may be used to "relax" the structures and to identify low energy orientations of drugs in receptor sites. Molecular dynamics may assist in exploring the energy landscape, and free energy simulations can be used to compute the relative binding free energies of a series of putative drugs.

The invention moreover contemplates in another preferred embodiment a method wherein the compound identified and optionally further modified as indicated above is formulated into a pharmaceutical composition.

In a different preferred embodiment of the invention, the method comprises chemically synthesizing (a) the compound fitting into the binding pocket of the PTP domain and *in silico* or *in vitro* interfering with the binding of the PTP domain or of ICA512, ICA-TMF, ICA-CCF or PHOGRIN to β2-syntrophin; or (b) the compound fitting into the binding pocket of the PDZ domain and *in silico* or *in vitro* interfering with the binding of the PDZ domain or β2-syntrophin with ICA512, ICA-TMF, ICA-CCF or PHOGRIN.

In a further preferred embodiment of the pharmaceutical composition of the invention, the use of the invention or the method of the invention said kinase is CDK5 (Rosales and Lee, 2006; Angelo et al, 2006) .

In a further preferred embodiment of the pharmaceutical composition, use or method of the invention for enhancing insulin secretion, said compound is a small molecule, a peptide, an antibody, a peptide aptamer, a nucleic acid aptamer, a glucose metabolite, an anticalin, an siRNA, an shRNA, a ribozyme, an antisense nucleic acid molecule or a mimetic, preferably a peptidomimetic.

SiRNA, shRNA, antisense nucleic acid molecules and ribozymes only come into play if the molecule to be interfered with is a nucleic acid molecule such as an mRNA molecule. Antisense nucleic acid molecules may be DNA or RNA molecules.

The term "antibody", as used in accordance with the present invention includes antibody fragments or derivatives, such as Fab' framents, F(ab₂)', scFvs, humanized or chimeric antibodies and has been described in more detail above.

Peptide aptamers are small molecule drugs that inhibit intracellular protein interactions (Hoppe-Seyler F, Crnkovic-Mertens I, Tomai E, Butz K. 2004. Peptide aptamers: specific inhibitors of protein function. Curr Mol Med. 2004 Aug;4(5):529-38) and have been further described above. Anticalins are engineered proteins with antibody-like ligand-binding functions derived from natural lipocalins as a scaffold (Skerra A. 2001 'Anticalins': a new class of engineered ligand-binding proteins with antibody-like properties. J Biotechnol. 74:257-75).

Further, the present invention relates to a glucose metabolite or a structurally related compound/derivative thereof for the preparation of a pharmaceutical composition for the treatment of diabetes.
As has been described herein above, one surprising finding in the present invention is that it is the PTP domain which is involved in the interaction of, e.g. ICA512-TMF and β2-syntrophin and thus leads to the enhancement of the secretion of insulin from pancreatic β-cells.
The requirement of the glucose metabolite or structurally related compound/derivative is thus to fit into the catalytically inactive pocket of the PTB of ICA512, PHOGRIN or functional fragments thereof to modulate their activity such that their interaction with β2-syntrophin is reduced or abolished.
The term "derivative" as used in accordance with the present invention relates to a compound derived from glucose metabolites in a way that they fit into the catalytically inactive pocket of the PTB of ICA512, PHOGRIN or functional analogues thereof. Thus, any chemical modification to a glucose metabolite, in particular to glyceraldehyde-3-phosphate, dihydroxyacetonephosphate or 1,3-diphosphoglycerate which conserves or enhances the binding properties/binding strength of the respective molecule to said catalytically inactive pocket falls within the scope of the present invention. Methods to test whether a compound falls under the scope of the present invention are disclosed in the present invention and described in detail above and in the appended examples.

In a preferred embodiment of the use of the invention said glucose metabolite is glyceraldehyde-3-phosphate.

In a most preferred embodiment of the invention, said diabetes is type-2 diabetes.

The figures show:

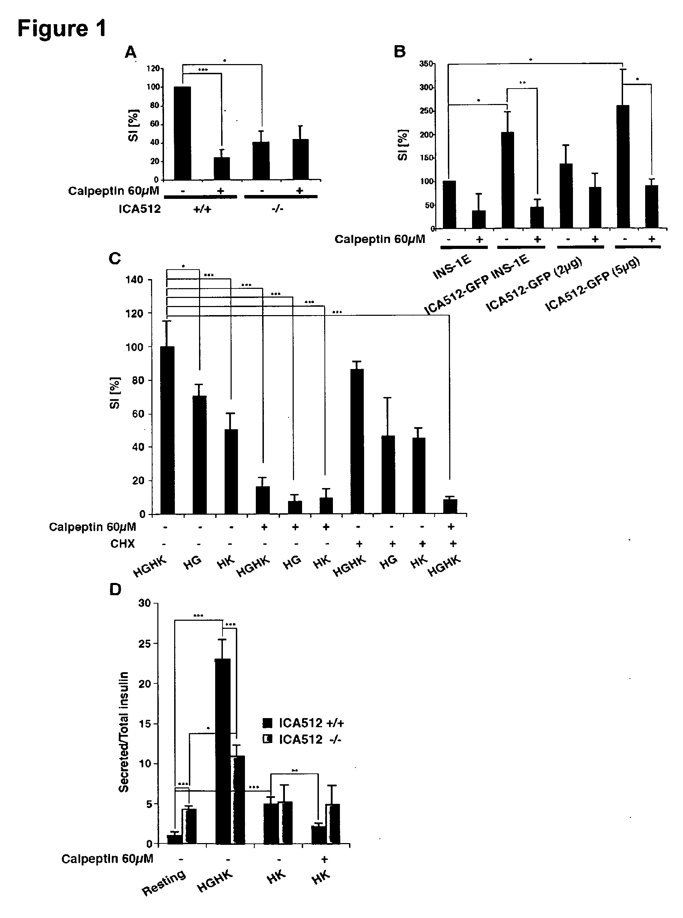

**Figure 1****. Calpain-1-mediated cleavage of ICA512 regulates insulin secretion. (A)** Insulin secretion stimulation index (SI) of pancreatic islets from *ICA512*^{+/+} or *ICA512*^{-/-} mice treated with (+) or without (-) 60µM Calpeptin. The SI of *ICA512*^{+/+} islets was equaled to 100%. **(B)** Sl of INS-1E cells non-transfected, or transfected stably or transiently with 2 or 5µg *ICA512-GFP,* and then treated or untreated with 60µM Calpeptin. The Sl of non-transfected INS-1E cells was equaled to 100%. **(C)** SI of INS- 1E cells stimulated with high glucose (25 mM, HG), high potassium (55 mM, HK), or both (HGHK). Cells were treated with or without 60µM Calpeptin, 100µM Cycloheximide (CHX), or both. The Sl of cells stimulated with HGHK was equaled to 100%. **(D)** Ratio of secreted vs. total insulin of *ICA512*^{+/+} (black columns) or *ICA512*^{-/-} (gray columns) mouse islets kept at rest or stimulated with HGHK or HK and treated with or without 60µM Calpeptin. The ratio in resting, untreated *ICA512*^{+/+} islets, was equaled to 1. Error bars show mean +/- SD. All the results are from at ≥3 independent experiments except (D) which is from 2 independent experiments performed in triplicate. Single asterisk (*): p<0.05, double asterisks (**): p<0.01, triple asterisks (***): p<0.005.

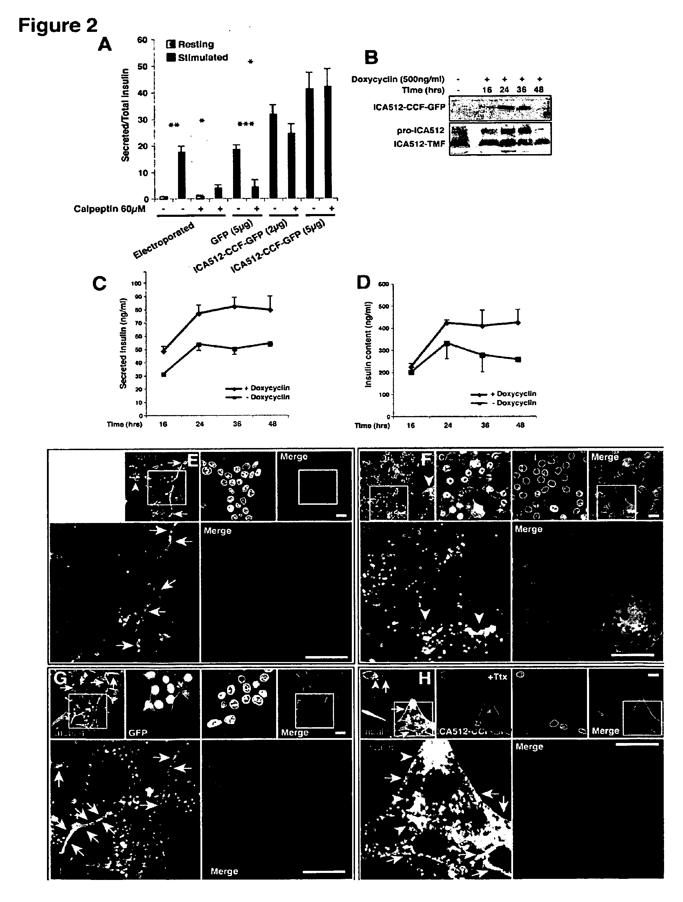

**Figure 2****. ICA512-CCF stimulates insulin secretion. (A)** Ratio of secreted vs. total insulin from INS-1E cells non-transfected, or transiently transfected with 5µg *GFP* or 2
   or 5 µg *ICA512-CCF-GFP* plasmids. Cells were kept at rest or stimulated with HGHK and treated with or without 60µM Calpeptin. The ratio in electroporated, resting INS-1E cells was equaled to 1. **(B)** Western blottings with anti-GFP (top) or anti-ICA512 (bottom) antibodies on total protein extracts from INS-r3 cells transiently transfected with tetracycline inducible *ICA512-CCF-GFP.* Cells were treated with or without 500ng/ml Doxycyclin for various time intervals as indicated. **(C** and **D)** Insulin secretion (C) and content (D) of INS-r3 cells treated as in (B). Results in (C) and (D) are from 3 independent experiments, each in triplicate. **(E-H)** Confocal Z-sections (0.5 µm) of INS-1E cells immunolabeled with anti-insulin antibody (pseudored). Nuclei were counterstained with DAPI (pseudoblue). Arrows and arrowheads point to the accumulation of insulin immunoreactivity in proximity of the plasma membrane and the perinuclear region, respectively. In (**F-H**) cells were transiently transfected with 5µg *ICA512-CCF-GFP* **(F, H)** or *GFP* **(G)** (pseudogreen). In **(H)** cells were co-transfected with *tetanus toxin light chain* as described (Trajkovski et al., 2004). Bars in (**E-H**): 10 µm. Error bars in (A), (C), and (D) show mean +/- SD. The results are from ≥ 3 independent experiments performed in triplicate. (*) p<0.05, (**) p<0.01, (***) p<0.005.

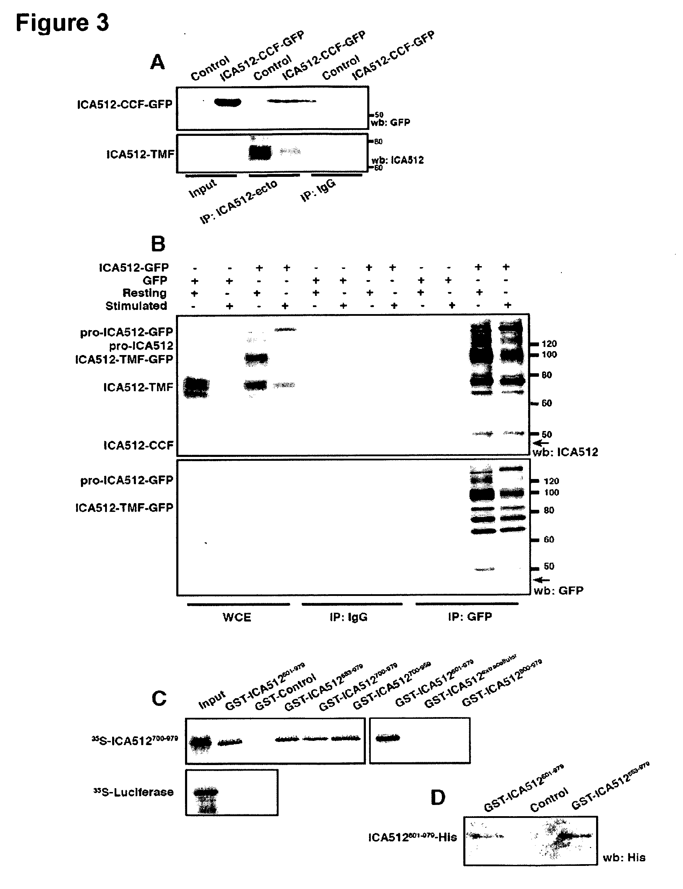

**Figure 3****. ICA512-CCF dimerizes with ICA512-TMF. (A)** Western blottings with mouse anti-GFP (top) or anti-ICA512 (bottom) antibodies on total protein extracts (lanes 1 and 2), or on immunoprecipates (IP) with rabbit anti-ICA512ecto (lanes 3 and 4) or control (lanes 5 and 6) immunoglobulins from INS-1E transfected or not with 5µg *ICA512-CCF-GFP.* **(B)** Western blottings with mouse anti-ICA512-HM1 (Mziaut et al., 2006) (top) or anti-GFP (bottom) antibodies on cytosolic protein extracts (lanes 1 to 4) or immunoprecipitates with goat control (lanes 5 to 8) or anti-GFP (lanes 9 to 12) immunoglobulins from resting or HGHK-stimulated INS-1E cells. Cells were either nontransfected or transfected with 5µg *ICA512-GFP.* **(C)** SDS-PAGE and autoradiography of in-vitro transcribed and translated 35S-ICA512700-979 (top) or 35S-luciferase (bottom) pulled down with glutathione sepharose beads coupled to 1µg of the following recombinant proteins: GST-ICA512601-979, GST-ICA512663-979, GST-ICA512700-979, GSTICA512700-959, GST-ICA512extracellular, GST-ICA512800-979 or GST. The input of 35SICA512700-979 and 35S-luciferase for the pull-down assays is shown in lanes **1**. **(D)** Western blotting with anti-His antibodies of bacterially expressed ICA512601-979-His pulled down with glutathione sepharose beads coupled to 1 µg of GST-ICA512601-979, GST-ICA512663-979 or GST.

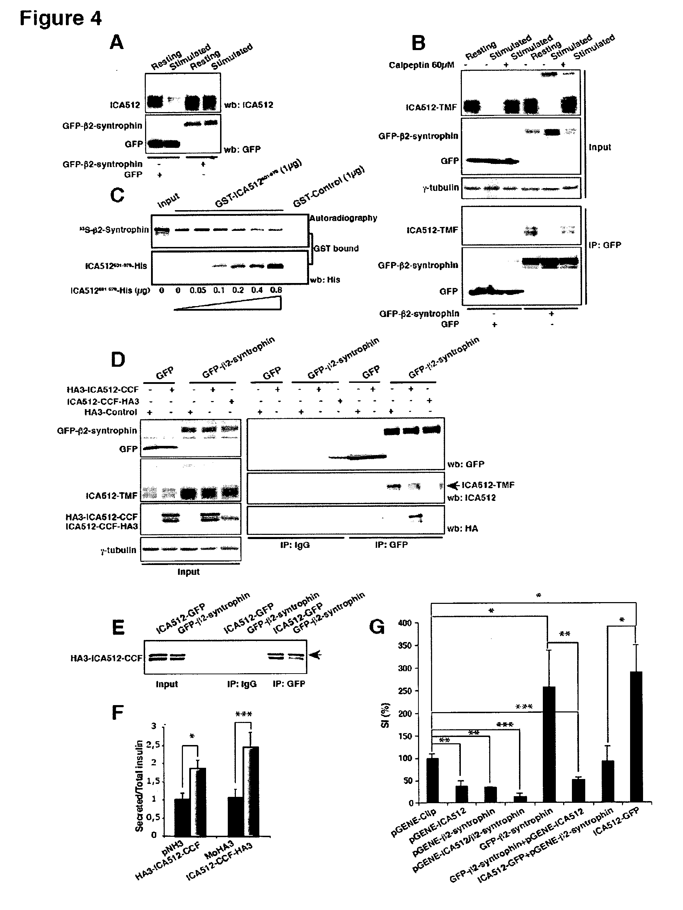

**Figure 4****. ICA512-CCF dissociates ICA512-TMF from β2-syntrophin. (A)** Western blottings with mouse anti-ICA512 (top panel) or anti-GFP (middle and bottom pannels) antibodies on total protein extracts from resting or HGHK-stimulated INS-1 cells stably transfected with *GFP* or *GFP-β2-syntrophin.* **(B)** Western blottings with mouse anti-ICA512 (first and fourth panels from the top), anti-GFP (second and last panels from the top), or anti-□-tubulin (third panel from the top) antibodies on total protein extracts (first three panels from the top) or immunoprecipitates with anti-GFP antibodies from resting or HGHK-stimulated INS-1E cells. Cells were stably transfected with *GFP* or *GFP- β2-syntrophin* and treated with or without 60µM Calpeptin. **(C)** SDS-PAGE and autoradiography of in-vitro transcribed and translated 35S- β2-syntrophin (top panel), and western blottings with anti-His antibodies of bacterially expressed ICA512601-979-His (bottom panel), pulled down with glutathione sepharose beads coupled to 1 µg GSTICA512601-979. **(D)** Western blottings with anti-GFP (top panels), anti-ICA512 (second panels from the top), and anti-HA (third panels from the top) or β-tubulin (bottom left panel) antibodies on total protein extracts (input, left panels) or immunoprecipitates (IP) with goat control (lanes 6-10) or anti-GFP (lanes 11-15) immunoglobulins (right panels) from INS-1E cells stably co-transfected stably with *GFP* or *GFP- β2-syntrophin* and transiently with 5µg *HA3-ICA512-CCF, ICA512-CCF-HA3* or control *HA3* vectors. **(E)** Western blotting with the anti-HA antibody on total protein extracts (lanes 1 and 2); or immunoprecipitates with goat control (lanes 3 and 4) and anti-GFP (lanes 5 and 6) immunoglobulins from INS-1E cells transfected stably with *GFP- β2-syntrophin* or *ICA512-GFP* and transiently with 5µg *HA3-ICA512-CCF.* **(F)** Ratio of secreted vs. total insulin from INS-1E cells transiently transfected with 5 µg *HA3-ICA512-CCF, ICA512-CCF-HA3,* or the control *pNH3* and *MoHA3* vectors used for the generation of the Hatagged *ICA512-CCF* constructs. The ratio of cells transfected with *pNH3* and *MoHA3* was equaled to 1. **(G)** SI of insulin secretion from INS-1E cells transiently transfected with vectors for the individual or combined knockdown of *ICA512 (pGENE-ICA512*), *β2-syntrophin* (*pGENE- β2-syntrophin*), or the control *pGENE-Clip.* Cells were cotransfected with 5µg *GFP-β2-syntrophin* or *ICA512-GFP,* as indicated. The Sl in cells transfected with *pGENE-Clip* was equaled to 100%. The results are from ≥ 3 independent experiments performed in triplicate. (*) p<0.05, (**) p<0.01, (***) p<0.005.

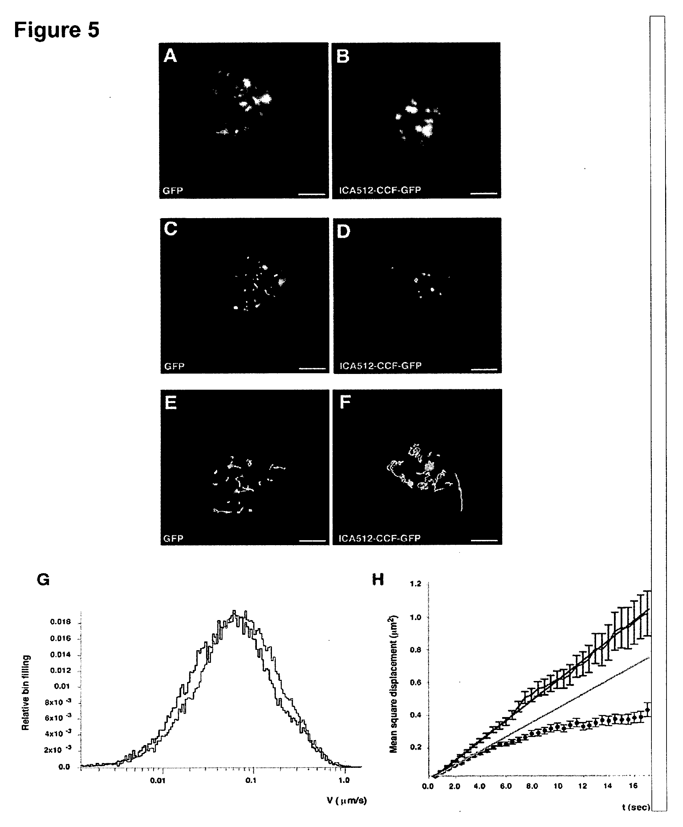

**Figure 5****. ICA512-CCF increases granule motility. (A-F)** Frames from TIRFM movies tracking the motion of CgB-mRFP1⁺ granules in resting INS-1 cells expressing GFP (A, C, E) or ICA512-CCF-GFP (B, D, F). (A and B) show the raw images, while (C and D) show the tracked granules, as recognized by the Motiontracking II software. In (E and F) the motion of individual CgB-mRFP1⁺ granules tracked for 50 sec. is displayed with a blue line. (**G** and **H**) Mean speed velocity (G) and square displacement (H) of CgBmRFP1⁺ granules in resting INS-1 cells expressing GFP (black traces) or ICA512-CCFGFP (red traces). In (G) the histogram bins were sampled uniformly in the logarithmic scale to better present the distribution difference at low speed. The sum of all bins equals 1 for both histograms. In (H) the green and blue lines show the weighted linear fit of the 3 initial mean square displacement points. Bars: 5 µm.
**Figure 6****. Regulation of granule turnover by ICA512.** A schematic representation of a model illustrating how stimuli inducing the release of insulin (such as glucose, 1) regulate granule turnover through the calpain-1-mediated cleavage of ICA512-TMF at the plasma membrane. The resulting cleaved cytoplasmic fragment, termed ICA512-CCF (2), disrupts the ICA512-TMF/β2-syntrophin complex by binding to either component of this complex (3 and 3'). The consequential displacement of granules from cortical actin increases their mobility and likelihood to approach sites competent for exocytosis (4). Progressive depletion of granules, increases the probability of ICA512-CCF to reach the nucleus (5), thereby increasing the transcription of granule genes by enhancing the activity of STAT-5 (6), as previously shown (Trajkovski et al., 2004), and thus granule biogenesis (7). For graphic reasons granules tethered to cortical actin filaments via the ICA512-TMF/ β2-syntrophin complex appear distant from the plasma membrane, although in reality they are located in very close proximity to it.
**Figure 7. (A)** Schematic domain representation of human ICA512. **(B)** Western blotting with the anti-ICA512-HM1 antibody on total protein extracts from resting or stimulated INS-1E cells as in Fig. 1d, treated with or without 60µM Calpeptin. The migration of standard molecular weight (kDa) proteins is indicated on the right.

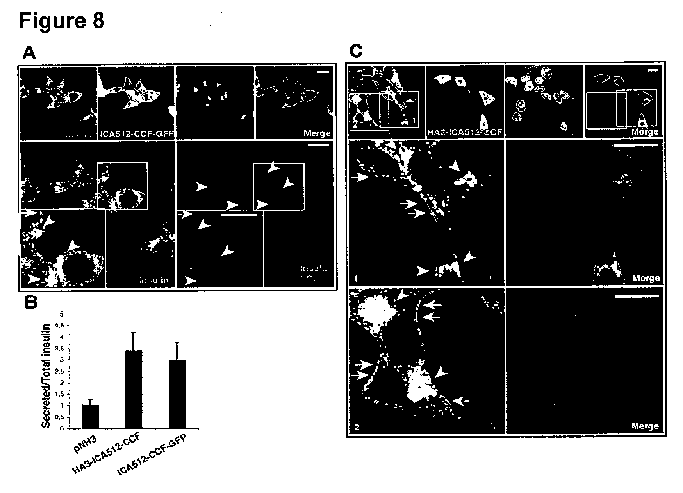

**Figure 8. (A)** Confocal Z-sections (0.5 µm) of INS-1E cells transiently transfected with 5µg *ICA512-CCF-GFP* (pseudogreen) double immunostained with antiinsulin (pseudored) and anti-TGN38 (pseudoblue) antibodies. (**B**) Ratio of secreted vs. total insulin of INS-1E cells transfected with *pNH3, HA3-ICA512-CCF* or *ICA512-CCFGFP.* The ratio in cells transfected with *pNH3* was equaled to 1. **(C)** Confocal Z-sections (0.5 µm) of INS-1E cells transiently transfected with 5µg *HA3-ICA512-CCF* immunolabeled with an anti-insulin antibody (pseudored). The nuclei are counterstained with DAPI (pseudoblue). In (A) and (C) the granules near the plasma membrane and the Golgi complex region are indicated with arrows and arrowheads, respectively. Bars: 10 µm.

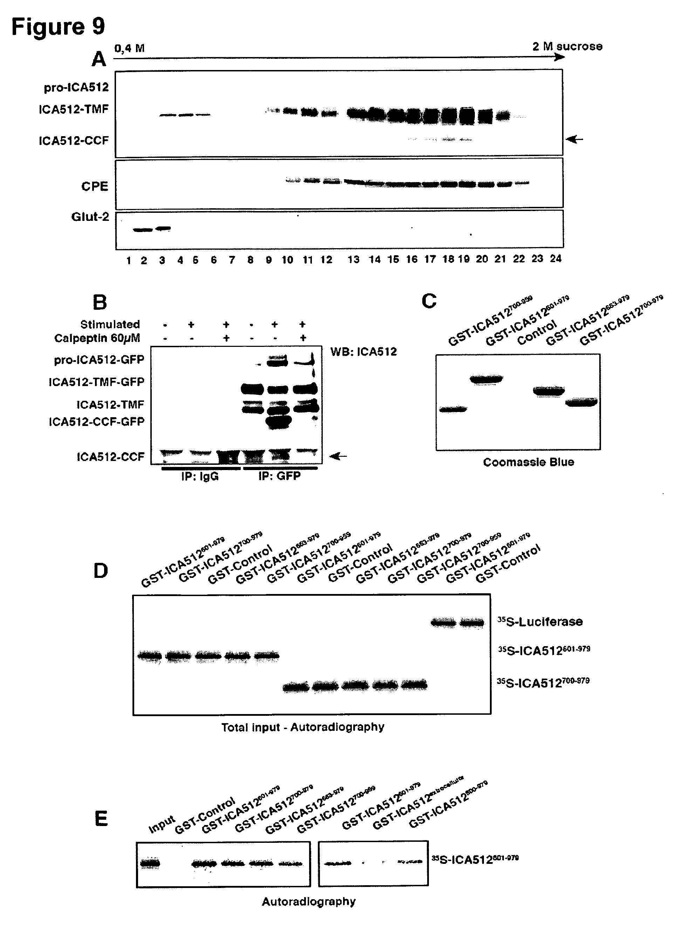

**Figure 9. (A)** Western blottings with anti-ICA512-HM1, anti-CPE and anti-Glut2 antibodies on INS-1E cell fractions separated on a continuous 0,4-2M sucrose density gradient. The arrows point to the band with the expected mobility of ICA512-CCF. **(B)** Western blotting with anti-ICA512-HM1 on immunoprecipitates (IP) btainedwith goat control (lanes 1-3), or anti-GFP immunoglobulins from total protein extracts of resting or HGHK-stimulated INS-1E cells transfected with or without 5µg *ICA512-GFP,* and treated with or without 60µM Calpeptin. The arrow points to co-immunoprecipitated endogenous ICA512-CCF. (**C**) Coomassie Blue staining of bacterially expressed GSTICA512601-979, GST-ICA512663-979, GST-ICA512700-979, GST-ICA512700-959. (**D**) SDSPAGE and autoradiography of in-vitro transcribed and translated 35S-ICA512601-979, ICA512700-979, and 35S-Luciferase used as starting material in figure 3c. (**E**) SDS-PAGE and autoradiography of in-vitro transcribed and translated 35S-ICA512601-979 pulled down with glutathione sepharose beads coupled to **1** µg GST-ICA512601-979, GST-ICA512663-979, GST-ICA512700-979, GST-ICA512700-959, GST-ICA512extracellular, GST-ICA512800-979, or GST alone. The starting material for the pulldown is shown in lane 1.

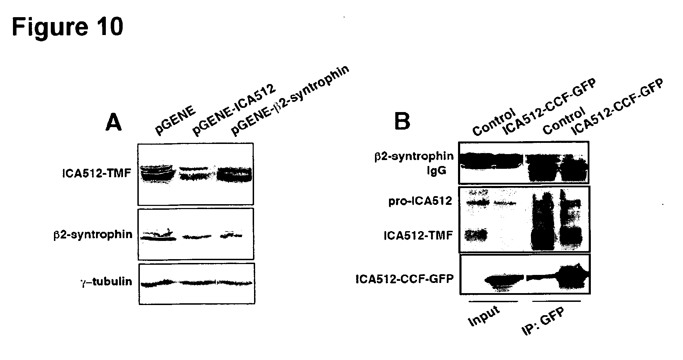

**Figure 10. (A)** Western blottings with anti-ICA512 (top panel), anti-ß2-syntrophin (middle panel), or anti-β-tubulin (botton panel) antibodies on total protein extracts from INS-1E cells transiently transfected with pGENE, pGENE-ICA512, or pGENE-β2-syntrophin as in figure 4g. **(B)** Western blottings with anti-β2-syntrophin (top panel), anti-ICA512 (middle panel), or anti-GFP (bottom panel) antibodies on total protein extracts (lanes 1 and 2), and immunoprecipitates obtained with anti-GFP (lanes 3 and 4) antibodies from INS-1 cells stably transfected with ICA512-CCF-GFP.

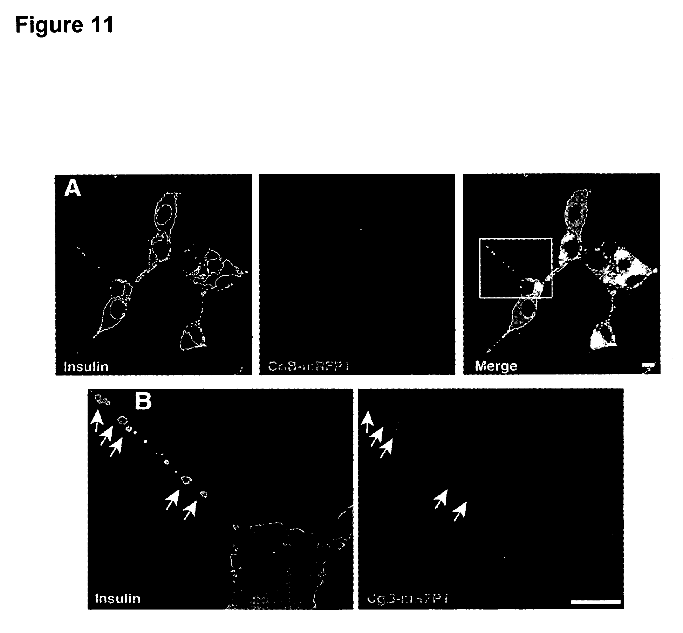

**Figure 11****. (A)** Co-localization of CgB-mRFP1 (pseudored) and insulin (pseudogreen) in stable CgB-mRFP1 INS-1 cells. The area in the inset is magnified in **(B)**. Arrows point to granules positive for insulin and CgB-mRFP1. Nuclei were counterstained with DAPI (blue). Bars: 5µm.

### The examples illustrate the invention

### Example 1: ICA512 cleavage regulates exocytosis

The calpain inhibitor calpeptin reduces the cleavage of ICA512-TMF in stimulated INS-1 cells (Ort et al., 2001; Trajkovski et al., 2004) and also in the more glucose responsive INS-1E cells (Merglen et al., 2004) (Fig. 7B). Calpeptin also inhibited insulin secretion by ≥70% from INS-1 cells (Ort et al., 2001), INS-1E cells (Fig. 1B, bars 1 and 2) and from purified mouse pancreatic islets (Fig. 1A, bars 1 and 2) following stimulation with high glucose (25 mM, HG) for 120 or 90 minutes, respectively. Consistent with previous studies (Saeki et al., 2002), the insulin stimulation index of *ICA512*-/- islets was reduced by 58% ±8.7 compared to *ICA512*+/+ islets (Fig. 1A, bars 1 and 3). Strikingly, calpeptin did not further decrease insulin secretion from *ICA512-*/*-* islets (Fig. 1A, bar 4), pointing to the calpain-1-mediated cleavage of ICA512-TMF being involved in regulation of insulin secretion. Accordingly, both stable and transient overexpression of ICA512-GFP in INS-1E cells enhanced their insulin release in a dose-dependent and calpeptin-sensitive fashion (Fig. 1B). Since calpeptin did not inhibit the increase of pro-ICA512 which is observed within 2 hours following stimulation (Mziaut et al., 2006) ( Fig. 7B), and which results from the activation of post-transcriptional mechanisms (Knoch et al., 2004), it seems likely that cleavage of ICA512-TMF promotes insulin secretion independently from its role on gene transcription and granule biogenesis. Indeed, block of protein synthesis with cycloheximide (Fig. 1C, bars 7-10), unlike calpeptin (Fig. 1C, bars 4-6 and 10), did not significantly reduce insulin secretion following stimulation with HG, high potassium (55 mM, HK) or both. Finally, depolarization of β-cells with HK alone, which triggers granule exocytosis and cleavage of ICA512-TMF by inducing Ca2+-entry (Ort et al., 2001; Trajkovski et al., 2004) (Fig. 7B), but not the biosynthesis of preproinsulin (Steiner et al., 2001) and pro-ICA512 (Ort et al., 2001; Trajkovski et al., 2004) (Fig. 7B), increased -5 fold insulin secretion from *ICA512*+/+ islets (Fig. 1D, bars 1 and 5), but not from ICA512-/- islets (Fig. 1D, bars 2 and 6). Moreover, this increment was calpeptin-sensitive in ICA512+/+ islets (Fig. 1D, bars 5 and 7), but not in *ICA512*-/- islets (Fig. 1D, bars 6 and 8). Taken together, these results demonstrate that cleavage of ICA512-TMF is a key event for regulating the Ca2+- dependent exocytosis of insulin granules.

### Example 2: ICA512-CCF directly affects secretion

Next, we evaluated the direct effect of ICA512-CCF on insulin secretion. Overexpression of ICA512-CCF fused to green fluorescent protein (ICA512-CCF-GFP) (Fig.2A, bars 2, 7, and 9), but not GFP (Fig. 2A, bars 2 and 5) increased stimulated insulin release in a concentration dependent fashion. Strikingly, calpeptin inhibited stimulated insulin secretion by 77% ± 5.3 and 75% ± 13.0 from control (Fig. 2A, bar 4) and *GFP*INS-1E cells (Fig. 2A, bars 5 and 6), respectively, while it had only a modest (Fig. 2A, bars 7 and 8), or no effect (Fig. 2A, bar 9 and 10) in *ICA512-CCF-GFP*-INS-1E cells, hence indicating that ICA512-CCF enhances granule exocytosis directly, and not through the regulation of proteins whose activity on secretion is calpeptin-sensitive. To further investigate its relationship with insulin secretion, ICA512-CCF was expressed in the INSr3 cells (Wang and lynedjian, 1997) in a tetracycline-inducible fashion. ICA512-CCF GFP was first detectable 16 hours after its induction with doxycyclin, and maximally expressed after 24 hours (Fig. 2B). Its appearance correlated at first with decreased levels of endogenous ICA512-TMF (Fig. 2B, lane 2) followed by increased pro-ICA512 and ICA512-TMF levels (Fig. 2B, lane 3). In parallel, insulin secretion increased by 65% ± 6.54 (Fig. 2C, point 1), before the increment in insulin content (Fig. 2D, point 1 and 2), indicating that the direct effect of ICA512-CCF on secretion precedes its role on granule biogenesis.

ICA512-CCF-GFP dramatically altered the intracellular distribution of insulin secretory granules (Fig. 2E and F). In INS-1E cells expressing ICA512-CCF-GFP, but not GFP alone, insulin immunoreactivity was reduced at the cell periphery (Fig. 2F), where granules typically accumulate (Fig. 2E and G, arrows), while it was increased throughout the cells and especially in the Golgi region (Fig. 2F, arrowheads, and Fig. 8A). The depletion of peripheral granules in *ICA512-CCF-GFP-*INS-1E cells was prevented by the co-transfection of tetanus toxin light chain, which blocks granule exocytosis (Fig. 2H, arrows), but not with an inactive tetanus toxin light chain mutant (not shown). Similar effects were observed when expressing ICA512-CCF N-terminally tagged with a triple HA epitope (HA3-ICA512-CCF). HA3-ICA512-CCF was also enriched in the nucleus (Fig. 8C), and stimulated insulin secretion slightly more than ICA512-CCF-GFP (Fig. 8B). Moreover, its expression increased insulin immunoreactivity in the Golgi region (Fig. 8C, arrowheads), while decreasing it at the periphery (Fig. 8C, arrows). Depletion of cortical granules and accumulation of insulin along the secretory pathway are consistent with ICA512-CCF having a dual role in enhancing granule exocytosis while promoting their biogenesis.

### Example 3: ICA512-CCF and ICA512-TMF homodimerize

We then attempted to identify how ICA512-CCF induces granule exocytosis. Separation of INS-1E cell extracts by sucrose density gradients revealed that fractions enriched in the granule markers ICA512-TMF and carboxypeptidase H contained a minor protein reactive with an antibody against ICA512-CCF (Mziaut et al., 2006) and the expected size for ICA512-CCF of -36 kDa (Fig. 9A, arrow). Since the ICA512 cytoplasmic domain can form homodimers in vitro and in transfected fibroblasts (Gross et al., 2002), ICA512-CCF could potentially bind through ICA512-TMF to granule membranes. Supporting this hypothesis, ICA512-CCF-GFP was selectively coimmunoprecipitated using an antibody against the ICA512-TMF ectodomain (Solimena et al., 1996), but not with control IgG (Fig. 3A). Likewise, endogenous ICA512-CCF was immunoprecipitated with the anti-GFP antibody from stimulated, but not from resting (Fig. 3B) or calpeptin-treated Fig. 9B, arrow) INS-1E cells expressing ICA512-GFP. On the other hand, we neither detected pro-ICA512 nor ICA512-TMF homodimers.

To map the dimerization domain, the cytoplasmic (ICA512601-979) and PTP (ICA512700-979) portions of ICA512 and firefly luciferase as a control protein were *in vitro* transcribed and translated in the presence of 35S-methionine and used for pull-down assays (Fig. 3C, and Fig. 9D and 9E) with various recombinant GST-ICA512 fusion proteins (Fig. 9C). Both 35S-ICA512601-979 and 35SICA512700-979 bound similarly to GST-ICA512601-979, GST-ICA512663-979, GST-ICA512700-979 and GST-ICA512700-959, but not to GST-ICA512800-979, which lacks part of the PTP domain, or GST-ICA512extracellular, while the control protein 35S-Luciferase, did not heterodimerize with GST-ICA512601-979 (Fig. 3C, lower panel). These data indicate that the PTP domain without its flanking N- and C-terminal PDZ domain binding regions is necessary and sufficient for ICA512 homodimerization (Fig. 3C and Fig. 9E). Moreover, bacterially expressed ICA512601-979 tagged with a 6-histidine epitope (ICA512601-979-His) did also bind to GST-ICA512601-979 and GST-ICA512663-979 (Fig. 3D), thus demonstrating that ICA512 dimerization does not dependent on other proteins.

### Example 4: ICA512-CCF displaces ICA512-TMF from β2-syntrophin

We subsequently investigated whether ICA512-CCF affects the interaction of ICA512-TMF with β2-syntrophin (Ort et al., 2000; Ort et al., 2001) by binding to either protein. Additional evidence of the close relationship between β2-syntrophin and ICA512-TMF was that overexpression of the former fused to GFP increased the levels of the latter, both in resting and stimulated cells (Fig. 4A, lanes 3 and 4). Conversely, knockdown of β2-syntrophin by RNAi reduced the expression of ICA512-TMF and vice versa (Fig. 10A). Also consistent with preliminary findings (Ort et al.,2001) ICA512-TMF was co-immunoprecipitated with GFP-β2-syntrophin from extracts of resting (Fig. 4B, lane 4), but not stimulated (Fig. 4B, lane 5), INS-1E cells. However, ICA512-TMF was still co-immunoprecipated with GFP- β2-syntrophin upon calpeptin treatment of stimulated cells (Fig. 4B, lane 6), hence suggesting that ICA512-CCF modulates the dissociation of ICA512-TMF from β2-syntrophin. To test this hypothesis,we analyzed how homodimerization of ICA512 affects its binding to 35S- β2-syntrophin by pull-down assays. These analyses clearly showed that increased binding of ICA512601-979-His to GST-ICA512601-979 (Fig. 3C, bottom) proportionally decreased the interaction of GST-ICA512601-979 with 35S- β2-syntrophin (Fig.3C, top). To further test this scenario within INS-1E cells, ICA512-CCF either N-terminally (HA3-ICA512-CCF) or C-terminally (ICA512-CCF-HA3) tagged with a triple HA epitope was co-expressed with GFP-β2-syntrophin or GFP (Fig. 4D, input). Remarkably, by immunoblotting with the anti-HA antibody HA3-ICA512-CCF appeared as a close doublet (Fig. 4D, lower middle panel, lanes 2 and 4), suggesting that within cells its C-terminus which includes the binding site for the PDZ of β2-syntrophin (Fig. 8A) is proteolytically removed (Ort et al., 2000). This cleavage could be precluded by fusion of the C-terminus with a tag, as in ICA512-CCF-HA3 and ICA512-CCF-GFP, which always appeared as single bands by immunoblotting (Fig. 4D, lower middle panel, lane 5 and Fig. 3A, top panel, lanes 2 and 4). As we expected, the association of ICA512-TMF with GFP-β2-syntrophin was strongly reduced upon expression of HA3-ICA512-CCF (Fig. 4D, middle panel, lanes 13 and 14), and even more so by ICA512-CCF-HA3, whose C-terminal tag conceivably hampers its interaction with GFP-β2-syntrophin (Fig. 4D, middle panel, lane 15). Co-immunoprecipitations with an antibody directed against the ectodomain of ICA512-TMF further indicated that the interaction of endogenous β2- syntrophin with ICA512-TMF is also reduced upon expression of ICA512-CCF-GFP relatively to GFP alone (Fig. 10A, top panel, lanes 3 and 4).

Unlike ICA512-TMF (Fig. 4E, lane 5), GFP-β2-syntrophin interacted preferentially with the higher as compared to the lower of the two HA3-ICA512-CCF species (Fig. 4D, bottom panel, lane 14 and Fig. 4E, lane 6). This result can be explained by considering that the lower HA3-ICA512-CCF species, despite the likely removal of the C-terminal binding site for β2-syntrophin, still contains a PDZ binding site N-terminally to the PTP domain (Ort et al., 2000) (Fig. 7A). By favoring the interaction with ICA512-TMF over β2-syntrophin, the C-terminal cleavage of ICA512-CCF could represent a novel mechanism for promoting the mobilization of granules. Accordingly, ICA512-CCF-HA3, which did not bind β2-syntrophin and induced a stronger dissociation of the ICA512-TMF/β2-syntrophin complex (Fig. 4D, lane 15), enhanced insulin secretion more than HA3-ICA512-CCF (Fig. 4F). Corroborating the role of the ICA512-TMF/ β2-syntrophin complex in regulating the retention of granules and their exocytosis, knockdown of β2-syntrophin reduced the insulin stimulation index to 34% ± 1.0 compared to control cells (Fig. 4G, bar 3). As expected (Harashima et al., 2005; Saeki et al., 2002), knockdown of ICA512 also decreased stimulated insulin secretion (Fig. 4G, bar 2), which was further diminished upon knockdown of both proteins (Fig. 4G, bar 4). Conversely, overexpression of GFP-β2-syntrophin increased the insulin stimulation index by 258% ±80 compared to control cells (Fig. 4G, bar 5) and most importantly, this increment was abolished upon knockdown of ICA512 (Fig. 4G, bar 6). Likewise, knockdown of β2-syntrophin reduced stimulated insulin secretion from ICA512-GFP over-expressing cells (Fig. 4G, bars 7 and 8), altogether demonstrating that the two proteins act on the same pathway.

### Example 5: ICA512-CCF increases granule mobility

In β-cells exposed to low (<5mM) glucose levels granules display mainly short oscillatory movements (<2-3 vesicle diameter), whereas stimulatory glucose concentrations (>5 mM) allow the detection of granules making excursions of several micrometers (Pouli et al., 1998), hence having a greater probability to approach the plasma membrane and fuse with it. Accordingly, we used total internal reflection fluorescence microscopy to test in living INS-1 cells whether ICA512-CCF affects the mobility of granules near (≤200 nm) the plasma membrane. For this purpose INS-1 cells stably transfected with the granule cargo *chromogranin B* tagged with monomeric red fluorescent protein 1 (Campbell et al., 2002) (*CgB-mRFP1*) were transiently cotransfected with *GFP* (Fig. 5A, C, E) or *ICA512-CCFGFP* (Fig. 5B, D, F). Co-localization of CgB-mRFP1 with insulin granules was verified by confocal microscopy (Fig. 11). Computer-assisted analysis with the Motiontracking II software (Rink et al., 2005), which allows automated particle tracking, revealed that the mean speed of CgB-mRFP1+ granules in resting *ICA512-CCF-GFPlCgB-mRFP1-*INS1 cells was significantly increased relative to resting *GFP*/*CgB-mRFP1-*INS1 (0.113 µm sec-1 vs. 0.069 µm sec-1, p=0.003) (Fig. 5G). To gain further insight into the impact of ICA512-CCF on the trajectory of cortical granules, the averaged mean square displacement of CgB-mRFP1+ vesicles was plotted against time. In *ICA512-CCF-GFPICgB-mRFP1-*INS1 cells the mean-square displacement of CgB mRFP1+ granules showed an almost linear dependency (Fig. 5H), which is characteristic of vesicles whose motion is not constrained (Huet et al., 2006). This pattern arose from frequent and random changes in the direction of the granules. Conversely, in *GFPICgBmRFP1-*INS1 cells (Fig. 5h, black trace) the mean-square displacement of CgB-mRFP1+ granules showed a linear dependency for <4 sec before curve abatement, which is characteristic of vesicles caged by the neighboring cytoskeleton network. This evidence directly proves that ICA512-CCF enhances the motility of granules by breaking their link to the cytoskeleton.

### Example 6: Materials and Methods

**Islet isolations and cell cultures.** Pancreatic islets were isolated from 15-17 weeks old *ICA512-*/*-* mice (Mziaut et al., 2006) and wildtype littermates as described (Gotoh et al., 1985) and then kept for 24 h in culture prior to the experiment. INS-1-derived INS-1E (Merglen et al., 2004) and tetracycline-inducible INS-r3 (Wang and lynedjian, 1997) cell lines were gifts from C. Wollheim (University of Geneva, Geneva, Switzerland) and were grown as described (Asfari et al., 1992). Wildtype and transfected INS-1E cells and pancreatic islets were incubated for 60 minutes in resting buffer (0 mM glucose and 5mM KCI) and then for 105 minutes in fresh resting or stimulating buffer (25 mM glucose and 55 mM KCI) as described (Ort et al., 2001; Trajkovski et al., 2004). Calpeptin (Calbiochem, La Jolla, CA) was added in the last 15 minutes of the pre-incubation, and then until cells were harvested.

**cDNA constructs.** The following constructs were described: ICA512-GFP, ICA512-CCF-GFP and ICA512-HA3 (Trajkovski et al., 2004); GST-ICA512601-979, GSTICA512663-979, GST-ICA512700-959, GST-ICA512700-979, GST-ICA512extracellular and GSTICA512800-979 (Ort et al., 2000), ICA512-His (Ort et al., 2001), GFP-β2-syntrophin (Kachinsky et al., 1999). ICA512-CCF-HA3 and HA3-ICA512-CCF were generated by subcloning the cDNA encoding residues 659-979 of human ICA512 as a Kpnl-Xbal insert into pMoHa3 (gift from M. Suchanek and C. Thiele, Max Planck Institute of Cell Biology and Genetics, Dresden, Germany) or as a Bglll-EcoR1 insert into pN3HA (Clontech, Palo Alto, CA), respectively. For tetracycline inducible expression of ICA512-CCF-GFP, the corresponding cDNA (Trajkovski et al., 2004) was subcloned as a BamHl/Xbal insert between the tet-operator-tk-minimal promoter and the SV40 polyA in a vector derived from the tet-tk-luc plasmid (Anastassiadis et al., 2002). Subsequently the tet-tk-lCA512-CCF-GFP-pA cassette was cloned between chicken β-globin insulators in a vector kindly provided by K Anastassiadis (Biotec TUD, Dresden, Germany). The mouse cDNA of the granule cargo chromogranin B (CgB; gift from W. Huttner, Max Planck Institute of Cell Biology and Genetics, Dresden, Germany) was fused in frame at the 5'-end of monomeric Red Fluorescent Protein (mRFP1) cDNA (Planetgene, Menlo Park, CA). All clones were generated using conventional procedures and verified by DNA sequencing.

**Cell Transfections.** INS-1E cells were electroporated as described (Trajkovski et al., 2004). The generation of stable GFP- and ICA512-CCF-GFP INS-1 cell clones was described (Trajkovski et al., 2004). INS-r3 cells expressing the reverse tetracyclinedependent transactivator were transiently transfected with ICA512-CCF-GFP in the tettk-luc-derived plasmid. The expression of ICA512-CCF-GFP was induced by exposure to 500ng/ml doxycyclin for 16 and 40 hours. CgB-mRFP1 INS-1 clones were selected for puromycin resistance, and further screened by fluorescence microscopy and immunoblotting for expression of the transgene.

**Cell extracts and western blottings.** Purified mouse islets and INS-1E cells were washed with ice cold PBS, and extracted in lysis buffer (10 mM Tris-HCI at pH 8.0, 140 mM NaCl, 1% Triton X-100, 1mM EDTA, 1mM PMSF, 1% phosphatase inhibitors (Calbiochem), and 1% protease inhibitor cocktail (Sigma, St. Louis, MO) at 4 °C. Protein measurements and immunoblottings were performed as described (Trajkovski et al., 2004) using the following primary antibodies: mouse monoclonals antibodies mICA512 (Hermel et al., 1999), mICA512-HM1 (Mziaut et al., 2006), anti-β-tubulin (Sigma) and anti-GFP (Clontech); rabbit polyclonal anti-glut2 (Alpha Diagnostics International, St Antonio, TX), anti-ICA512ecto (Solimena et al., 1996) anti-carboxypeptidase E/H (CPE) (Chemicon, Temecula, CA), goat anti-GFP (gift from David Drechsel, Max Planck Institute of Cell Biology and Genetics, Dresden, Germany). Chemiluminescence was developed and quantified as described (Trajkovski et al., 2004).

**Sucrose density gradient fractionations.** Fractionations of INS-1E cells on continuous sucrose density gradients from 0.4-2 M sucrose were performed as described (lezzi et al., 1999; Solimena et al., 1996). Fractions were separated by SDS-PAGE and analyzed by immunoblotting.

**Immunoprecipitations.** INS-1E cells were transiently or stably transfected with one or more of the following constructs: *GFP, ICA512-CCF-GFP, GFP-* β*2-syntrophin, ICA512-CCF-HA3, HA3-ICA512-CCF.* Protein extracts in 20 mM Tris-HCl at pH 8.0, 200 mM NaCl, 1% Triton X-100, 0.1% SDS, 1mM EDTA, 1mM PMSF, 1% phosphatase inhibitors (Calbiochem) and 1% protease inhibitor cocktail (Sigma) were incubated overnight at 4 °C with one of the following antibodies: goat anti-GFP, rabbit anti ICA512ecto, goat or rabbit IgGs (Sigma), followed by incubation with protein G sepharose (GE Healthcare Biosciences, Uppsala, Sweden) for 90 minutes. The beads were then washed 10 times, and loaded on 8-10% SDS-PAGE followed by western blotting with one of the following mouse antibodies: anti-ICA512, anti-GFP (Clontech, Mountain View, CA), anti- β2-syntrophin (a gift from S. Froehner and M. Adams, University of Washington, Seattle), anti-HA and anti-β-tubulin (Sigma).

**Pulldown Assays.** ICA512601-979, ICA512700-979 or β2-syntrophin in pET28a (Ort et al., 2000) were in vitro transcribed and translated with the T7/coupled transcriptiontranslation system (Promega, Madison, WI). GST-ICA512601-979, GST-ICA512663-979,
GST-ICA512700-959, GST-ICA512700-979, GST-ICA512extracellular, GST-ICA512800-979 and ICA512-His were expressed in bacteria as described (Mziaut et al., 2006; Ort et al., 2001). 35S-methionine-labeled ICA512601-979, ICA512700-979 or β2-syntrophin were incubated overnight at 4 °C in GST binding buffer (240 mM NaCl, 50 mM Tris, pH 8, 0.25% NP-40, 0.15% SDS, 1 mM DTT, and 1 mM PMSF) with GST-fusion proteins or GST alone coupled to glutathione-sepharose beads (Amersham Biosciences). Beads were washed 10 times with binding buffer and eluted with 10 mM of reduced glutathione. Eluted proteins were subjected to SDS-PAGE and analyzed by autoradiography.

**Immunocytochemistry.** Labeling of transfected or non-transfected INS-1E cells was performed as described (Trajkovski et al., 2004). The following primary and secondary antibodies were used: mouse anti-insulin (Sigma), rabbit anti-TGN38 (BD Transduction Laboratories, San Jose, CA), Alexa568-conjugated goat-anti-mouse or Alexa488- conjugated-goat-anti-rabbit IgGs (Molecular Probes, Eugene, OR). Nuclei were counterstained with DAPI (Sigma). Images of 0.5 µm optical sections were acquired with an inverted confocal microscope LSM Meta 405nm (Zeiss, Göttingen, Germany).

**RNA interference.** For knockdown of rat ICA512 and β2-syntrophin the pGENE-CLIP U1 cassette system (Promega) was used according to the manufacturer's instruction to clone the following oligonucleotides: ICA512 shRNA oligo1, 5'-TCTCGCGCCATCATTCGAAACAATTCAAGAGATTGTTTCGAATGATGGCGCCT-3'; ICA512 shRNA oligo2, 5'-TCTCGCAGTACAAGCAGATGTAATTCAAGAGATTACATCTGCTTGTACTGCCT-3'; β2-syntrophin shRNA oligo1: 5'-TCTCGTAGCTATCCACACCAACATATTCAAGAGATATGTTGGTGTGGATAGCTAC CT-3'; 5'-CTGCAGGTAGCTATCCACACCAACATATCTCTTGAATATGTTGGTGTGGATAGCT AC-3'. Single or double transfections of INS-1E cells with pGENE-ICA512 and pGENE- β2-syntrophin by electroporation were performed as described (Trajkovski et al., 2004), using 4µg or 3µg of each plasmid for single or double transfection, respectively. Cells were stimulated and harvested 4 days after transfection and knockdown of ICA512 and β2-syntrophin was determined by real-time PCR and western blotting, as described Trajkovski et al., 2004).

**Insulin RIA.** Insulin secretion was assessed as a ratio between insulin in the medium and the cell insulin content. After stimulation, 25-30 purified pancreatic islets were resuspended in fresh resting or stimulating medium and sonicated for 4 minutes. INS-1E cells were extracted overnight in 3:1:0.06 volumes of ethanol, H2O and 37% HCI at -20 °C. Following centrifugation, insulin in islet cells and in the medium was measured with the Sensitive Rat Insulin RIA Kit (Linco Research, St. Charles, MO). The insulin stimulation index was calculated as follows: secreted/total insulin in stimulated conditions vs. secreted/total insulin in resting conditions.

**TIRFM.** Images of INS-1 cells stably expressing mouse CgB-mRFP1 were acquired with a Roper Scientific MicroMAX:512BFT charge-coupled device camera with a Zeiss Axiovert 200M microscope equipped with an Argon laser and a Zeiss 100x/1.45 N.A. Plan-FLUAR objective and a 1.6X optovar. The microscope was outfitted with a dualport TIRF condenser (Till Photonics, Gräfelfing, Germany). Prior to imaging, cells grown in an open chamber were incubated in resting media and transferred onto a thermostat-controlled (37 oC) stage. CgB-mRFP1+ granules in GFP+ cells were visualized by excitation of mRFP1 at 488 nm using filter sets for tetramethylrhodamine isothiocyanate emission (Chroma Technology Corp, Rockingham, VT). Images were collected at the speed of 2 frames second -1 with an exposure time of 100 ms, for a total of 4 minutes (480 frames) each movie.

Automated image analysis was performed with the Motiontracking II software as described (Rink et al., 2005). The background of nonvesicular fluorescence was subtracted and the granules were fitted by analytical function as described (Rink et al., 2005). The accuracy of granule center definition was estimated from the mean square displacement, i.e. the average of squared displacements, and found equal to 60 nm. The jitter of center definition was suppressed by smoothing the tracks with sliding window (±2 frames). The total number of tracked CgB-mRFP1+ granules in GFP+ and ICA512-CCF-GFP+cells was 779 and 888, respectively. The speed was measured between every two sequential frames. The total number of speed measurements in GFP+ and ICA512-CCF-GFP+ cells was 13,332 and 14,828 respectively.

**Statistics and graphics.** Statistical analyses were performed as previously described (Mziaut et al., 2006; Trajkovski et al., 2004). Error bars show standard deviations from at least three independent experiments. Histograms were prepared with Microsoft Excel (Microsoft, Redmond, WA).

### References

Albrecht, D.E., and Froehner, S.C. (2002). Syntrophins and dystrobrevins: defining the dystrophin scaffold at synapses. Neurosignals 11, 123-129.
Anastassiadis, K., Kim, J., Daigle, N., Sprengel, R., Scholer, H.R., and Stewart, A.F. (2002). A predictable ligand regulated expression strategy for stably integrated transgenes in mammalian cells in culture. Gene 298, 159-172.
Angelo M., Plattner F., Giese, K.P. (2006). Cyclin-dependent kinase 5 in synaptic plasticity, learning and memory. J Neurochem. 99(2), 353-370.
Asfari, M., Janjic, D., Meda, P., Li, G., Halban, P.A., and Wollheim, C.B. (1992). Establishment of 2-mercaptoethanol-dependent differentiated insulin-secreting cell lines. Endocrinology 130, 167-178.
Bratanova-Tochkova, T.K., Cheng, H., Daniel, S., Gunawardana, S., Liu, Y.J., Mulvaney-Musa, J., Schermerhorn, T., Straub, S.G., Yajima, H., and Sharp, G.W. (2002). Triggering and augmentation mechanisms, granule pools, and biphasic insulin secretion. Diabetes 51 Suppl 1, S83-90.
Bruun, T.Z., Hoy, M., and Gromada, J. (2000). Scinderin-derived actin-binding peptides inhibit Ca(2+)- and GTPgammaS-dependent exocytosis in mouse pancreatic beta-cells. Eur J Pharmacol 403, 221-224.
Cai, T., Fukushige, T., Notkins, A.L., and Krause, M. (2004). lnsulinoma-Associated Protein IA-2, a Vesicle Transmembrane Protein, Genetically Interacts with UNC-31/CAPS and Affects Neurosecretion in Caenorhabditis elegans. J Neurosci 24, 3115-3124.
Campbell, R.E., Tour, O., Palmer, A.E., Steinbach, P.A., Baird, G.S., Zacharias, D.A., and Tsien, R.Y. (2002). A monomeric red fluorescent protein. Proc Natl Acad Sci U S A 99, 7877-7882.
Castano, L., and Eisenbarth, G.S. (1990). Type-I diabetes: a chronic autoimmune disease of human, mouse, and rat. Annu Rev Immunol 8, 647-679.
Chiang, M.K. and Flanagan, J.G. (1996). PTP-NP, a new member of the receptor protein tyrosine phosphatase family, implicated in development of nervous system and pancreatic endocrine cells. Development. 122(7), 2239-2250.
Diamond, J. (2003). The double puzzle of diabetes. Nature 423, 599-602.
Donelan, M.J., Morfini, G., Julyan, R., Sommers, S., Hays, L., Kajio, H., Briaud, I., Easom, R.A., Molkentin, J.D., Brady, S.T., and Rhodes, C.J. (2002). Ca2+-dependent dephosphorylation of kinesin heavy chain on beta-granules in pancreatic beta-cells. Implications for regulated beta-granule transport and insulin exocytosis. J Biol Chem 277, 24232-24242.
Drake, P.G., Peters, G.H., Andersen, H.S., Hendriks, W., and Moller, N.P. (2003). A novel strategy for the development of selective active-site inhibitors of the protein tyrosine phosphatase-like proteins islet-cell antigen 512 (IA-2) and phogrin (IA-2beta). Biochem J 373, 393-401.
Duncan, R.R., Greaves, J., Wiegand, U.K., Matskevich, I., Bodammer, G., Apps, D.K., Shipston, M.J., and Chow, R.H. (2003). Functional and spatial segregation of secretory vesicle pools according to vesicle age. Nature 422, 176-180.
Elbashir S.M., Harborth J., Lendeckel W., Yalcin A., Weber K., Tuschl T. (2001). Duplexes of 21-nucleotide RNAs mediate RNA interference in cultured mammalian cells. Nature. 411 (6836), 494-498.
Eliasson, L., Ma, X., Renstrom, E., Barg, S., Berggren, P.O., Galvanovskis, J., Gromada, J., Jing, X., Lundquist, I., Salehi, A., et al. (2003). SUR1 regulates PKA-independent cAMP-induced granule priming in mouse pancreatic B-cells. J Gen Physiol 121, 181-197.
Gong, L.W., Di Paolo, G., Diaz, E., Cestra, G., Diaz, M.E., Lindau, M., De Camilli, P., and Toomre, D. (2005). Phosphatidylinositol phosphate kinase type I gamma regulates dynamics of large dense-core vesicle fusion. Proc Natl Acad Sci U S A 102, 5204-5209.
Gotoh, M., Maki, T., Kiyoizumi, T., Satomi, S., and Monaco, A.P. (1985). An improved method for isolation of mouse pancreatic islets. Transplantation 40, 437-438.
Gross, S., Blanchetot, C., Schepens, J., Albet, S., Lammers, R., den Hertog, J., and Hendriks, W. (2002). Multimerization of the protein-tyrosine phosphatase (PTP)-like insulin-dependent diabetes mellitus autoantigens IA-2 and IA-2beta with receptor PTPs (RPTPs). Inhibition of RPTPalpha enzymatic activity. J Biol Chem 277, 48139-48145.
Harashima, S., Clark, A., Christie, M.R., and Notkins, A.L. (2005). The dense core transmembrane vesicle protein IA-2 is a regulator of vesicle number and insulin secretion. Proc Natl Acad Sci U S A 102, 8704-8709.
Hay, J.C., Fisette, P.L., Jenkins, G.H., Fukami, K., Takenawa, T., Anderson, R.A., and Martin, T.F. (1995). ATP-dependent inositide phosphorylation required for Ca(2+)-activated secretion. Nature 374, 173-177.
Hermel, J.M., Dirkx, R., Jr., and Solimena, M. (1999). Post-translational modifications of ICA512, a receptor tyrosine phosphatase-like protein of secretory granules. Eur J Neurosci 11, 2609-2620.
Huet, S., Karatekin, E., Tran, V.S., Fanget, I., Cribier, S., and Henry, J.P. (2006). Analysis of transient behavior in complex trajectories: application to secretory vesicle dynamics. Biophys J 91, 3542-3559.
lezzi, M., Escher, G., Meda, P., Charollais, A., Baldini, G., Darchen, F., Wollheim, C.B., and Regazzi, R. (1999). Subcellular distribution and function of Rab3A, B, C, and D isoforms in insulin-secreting cells. Mol Endocrinol 13, 202-212. Ivarsson, R., Jing, X., Waselle, L., Regazzi, R., and Renstrom, E. (2005). Myosin 5a controls insulin granule recruitment during late-phase secretion. Traffic 6, 1027-1035. Kachinsky, A.M., Froehner, S.C., and Milgram, S.L. (1999). A PDZ-containing scaffold related to the dystrophin complex at the basolateral membrane of epithelial cells. J Cell Biol 145, 391-402.
Kawasaki, E., Hutton, J.C. and Eisenbarth, G.S. (1996). Molecular cloning and characterization of the human transmembrane protein tyrosine phosphatase homologue, phogrin, an autoantigen of type 1 diabetes. Biochem Biophys Res Commun. 227(2), 440-447.
Kawasaki, E., Eisenbarth, G.S., Wasmeier, C. and Hutton, J.C. (1996). Autoantibodies to protein tyrosine phosphatase-like proteins in type I diabetes. Overlapping specificities to phogrin and ICA512/IA-2. Diabetes 45(10), 1344-1349.
Klenchin, V.A., and Martin, T.F. (2000). Priming in exocytosis: attaining fusioncompetence after vesicle docking. Biochimie 82, 399-407.
Knoch, K.P., Bergert, H., Borgonovo, B., Saeger, H.D., Altkruger, A., Verkade, P., and Solimena, M. (2004). Polypyrimidine tract-binding protein promotes insulin secretory granule biogenesis. Nat Cell Biol 6, 207-214.
Lan, M.S., Lu, J., Goto, Y., and Notkins, A.L. (1994). Molecular cloning and identification of a receptor-type protein tyrosine phosphatase, IA-2, from human insulinoma. DNA Cell Biol 13, 505-514.
Li, G., Rungger-Brandle, E., Just, I., Jonas, J.C., Aktories, K., and Wollheim, C.B. (1994). Effect of disruption of actin filaments by Clostridium botulinum C2 toxin on insulin secretion in HIT-T15 cells and pancreatic islets. Mol Biol Cell 5, 1199-1213.
Loyet, K.M., Kowalchyk, J.A., Chaudhary, A., Chen, J., Prestwich, G.D., and Martin, T.F. (1998). Specific binding of phosphatidylinositol 4,5-bisphosphate to calciumdependent activator protein for secretion (CAPS), a potential phosphoinositide effector protein for regulated exocytosis. J Biol Chem 273, 8337-8343.
Lu J., Li Q., Xie H., Chen Z.J., Borovitskaya A.E., Maclaren N.K., Notkins A.L., Lan M.S. (1996). Identification of a second transmembrane protein tyrosine phosphatase, IA-2beta, as an autoantigen in insulin-dependent diabetes mellitus: precursor of the 37-kDa tryptic fragment. Proc Natl Acad Sci USA. 93(6), 2307-2311.
Merglen, A., Theander, S., Rubi, B., Chaffard, G., Wollheim, C.B., and Maechler, P. (2004). Glucose sensitivity and metabolism-secretion coupling studied during two-year continuous culture in INS-1E insulinoma cells. Endocrinology 145, 667-678.
Mziaut, H., Trajkovski, M., Kersting, S., Ehninger, A., Altkruger, A., Lemaitre, R.P., Schmidt, D., Saeger, H.D., Lee, M.S., Drechsel, D.N., et al. (2006). Synergy of glucose and growth hormone signalling in islet cells through ICA512 and STAT5. Nat Cell Biol 8, 435-445.
Ort, T., Maksimova, E., Dirkx, R., Kachinsky, A.M., Berghs, S., Froehner, S.C., and Solimena, M. (2000). The receptor tyrosine phosphatase-like protein ICA512 binds the PDZ domains of beta2-syntrophin and nNOS in pancreatic beta-cells. Eur J Cell Biol 79, 621-630.
Ort, T., Voronov, S., Guo, J., Zawalich, K., Froehner, S.C., Zawalich, W., and Solimena, M. (2001). Dephosphorylation of beta2-syntrophin and Ca2+/mu-calpain-mediated cleavage of ICA512 upon stimulation of insulin secretion. Embo J 20, 4013-4023.
Pouli, A.E., Emmanouilidou, E., Zhao, C., Wasmeier, C., Hutton, J.C., and Rutter, G.A. (1998). Secretory-granule dynamics visualized in vivo with a phogrin-green fluorescent protein chimaera. Biochem J 333 (Pt 1), 193-199.
Rabin, D.U., Pleasic, S.M., Shapiro, J.A., Yoo-Warren, H., Oles, J., Hicks, J.M., Goldstein, D.E., and Rae, P.M. (1994). Islet cell antigen 512 is a diabetes-specific islet autoantigen related to protein tyrosine phosphatases. J Immunol 152, 3183-3188.
Rink, J., Ghigo, E., Kalaidzidis, Y., and Zerial, M. (2005). Rab conversion as a mechanism of progression from early to late endosomes. Cell 122, 735-749.
Rorsman, P., and Renstrom, E. (2003). Insulin granule dynamics in pancreatic beta cells. Diabetologia 46, 1029-1045.
Rosales J.L. and Lee K.Y. (2006). Extraneuronal roles of cyclin-dependent kinase 5. Bioessays 10, 1023-1034.
Saeki, K., Zhu, M., Kubosaki, A., Xie, J., Lan, M.S., and Notkins, A.L. (2002). Targeted disruption of the protein tyrosine phosphatase-like molecule IA-2 results in alterations in glucose tolerance tests and insulin secretion. Diabetes 51, 1842-1850.
Saltiel, A.R. (2001). New perspectives into the molecular pathogenesis and treatment of type 2 diabetes. Cell 104, 517-529.
Solimena, M., Dirkx, R., Jr., Hermel, J.M., Pleasic-Williams, S., Shapiro, J.A., Caron, L., and Rabin, D.U. (1996). ICA 512, an autoantigen of type I diabetes, is an intrinsic membrane protein of neurosecretory granules. Embo J 15, 2102-2114.
Solimena, M., and Gerdes, H.H. (2003). Secretory granules: and the last shall be first. Trends Cell Biol 13, 399-402.
Steiner, D.F., Chan, S.J., and Rubenstein, A.H. (2001). Biosynthesis of insulin, In Handbook of Physiology. Section 7: The Endocrine System, L. S. Jefferson, A. D. Cherrington, and H. M. Goodman, eds. (New York: Oxford University Press, Inc.), pp. 49-78.
Tisch, R., and McDevitt, H. (1996). Insulin-dependent diabetes mellitus. Cell 85, 291-297.
Tomas, A., Yermen, B., Min, L., Pessin, J.E., and Halban, P.A. (2006). Regulation of pancreatic beta-cell insulin secretion by actin cytoskeleton remodelling: role of gelsolin and cooperation with the MAPK signalling pathway. J Cell Sci 119, 2156-2167.
Trajkovski, M., Mziaut, H., Altkruger, A., Ouwendijk, J., Knoch, K.P., Muller, S., and Solimena, M. (2004). Nuclear translocation of an ICA512 cytosolic fragment couples granule exocytosis and insulin expression in {beta}-cells. J Cell Biol 167, 1063-1074.
Varadi, A., Ainscow, E.K., Allan, V.J., and Rutter, G.A. (2002). Involvement of conventional kinesin in glucose-stimulated secretory granule movements and exocytosis in clonal pancreatic beta-cells. J Cell Sci 115, 4177-4189.
Varadi, A., Tsuboi, T., and Rutter, G.A. (2005). Myosin Va transports dense core secretory vesicles in pancreatic MIN6 beta-cells. Mol Biol Cell 16, 2670-2680.
Wang, H., and lynedjian, P.B. (1997). Modulation of glucose responsiveness of insulinoma beta-cells by graded overexpression of glucokinase. Proc Natl Acad Sci U S A 94, 4372-4377.
Waselle, L., Coppola, T., Fukuda, M., lezzi, M., El-Amraoui, A., Petit, C., and Regazzi, R. (2003). Involvement of the Rab27 binding protein Slac2c/MyRIP in insulin exocytosis. Mol Biol Cell 14, 4103-4113.
Zahn, T.R., Macmorris, M.A., Dong, W., Day, R., and Hutton, J.C. (2001). IDA-1, a Caenorhabditis elegans homolog of the diabetic autoantigens IA-2 and phogrin, is expressed in peptidergic neurons in the worm. J Comp Neurol 429, 127-143.
Zimmet, P., Alberti, K.G., and Shaw, J. (2001). Global and societal implications of the diabetes epidemic. Nature 414, 782-787.

## Claims

1. A pharmaceutical composition comprising
(a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN;
(b) a compound promoting the presence or activity of a protease that cleaves the C-terminus of ICA512, ICA512-TMF or ICA512-CCF or PHOGRIN;
(c) a compound promoting the presence or activity of a serine/threonine kinase which phosphorylates β2-syntrophin on serine residue 95;
(d) a compound interfering with the presence or activity of a serine/threonine phosphatase which dephosphorylates β2-syntrophin on serine residue 95;
(e) a compound promoting the presence or activity of a serine/threonine phosphatase which dephosphorylates β2-syntrophin on serine residue 110; or
(f) a compound interfering with the presence or activity of a serine/threonine kinase which phosphorylates β2-syntrophin on serine residue 110.

2. Use of
(a) ICA512, ICA512-TMF or ICA512-CCF, each devoid of the C-terminus or PHOGRIN devoid of the C-terminus or of a mimetic of the C-terminus of ICA512, ICA512-TMF, ICA512-CCF or PHOGRIN;
(b) a compound promoting the presence or activity of a protease that cleaves the C-terminus of ICA512, ICA512-TMF, ICA512-CCF or PHOGRIN;
(c) a compound promoting the presence or activity of a serine/threonine kinase which phosphorylates β2-syntrophin on serine residue 95;
(d) a compound interfering with the presence or activity of a. serine/threonine phosphatase which dephosphorylates β2-syntrophin on serine residue 95;
(e) a compound promoting the presence or activity of a serine/threonine phosphatase which dephosphorylates β2-syntrophin on serine residue 110; or
(f) a compound interfering with the presence or activity of a serine/threonine kinase which phosphorylates β2-syntrophin on serine residue 110
for the preparation of a pharmaceutical composition for the treatment of diabetes.

3. The pharmaceutical composition of claim 1 or the use of claim 2 wherein said mimetic is a peptidomimetic.

4. The pharmaceutical composition of claim 1 or the use of claim 2 wherein the compound promoting the presence or activity of said protease that cleaves the C-terminus of ICA512, ICA512-TMF, ICA512-CCF or PHOGRIN is an expression vector containing the coding region of said protease in expressible form.

5. The pharmaceutical composition of claim 1 or the use of claim 2 wherein the compound promoting the presence or activity of said serine/threonine kinase is an expression vector containing the coding region of said kinase in expressible form.

6. The pharmaceutical composition of claim 1 or the use of claim 2 wherein the compound promoting the presence or activity of said serine/threonine phosphatase is an expression vector containing the coding region of said phosphatase in expressible form.

7. A method of enhancing insulin secretion from pancreatic β-cells comprising promoting in said β-cells the presence or activity of
(a) (aa) ICA512, ICA512-TMF, ICA512-CCF or PHOGRIN; or
(ab) ICA512, ICA512-TMF or ICA512-CCF, each devoid of the C-terminus or PHOGRIN devoid of the C-terminus;
(b) a compound promoting the presence or activity of a protease that cleaves the C-terminus of ICA512, ICA512-TMF, ICA512-CCF or PHOGRIN;
(c) a compound promoting the presence or activity of serine/threonine kinase which phosphorylates β2-syntrophin on serine residue 95;
(d) a compound interfering with the presence or activity of serine/threonine phosphatase which dephosphorylates β2-syntrophin on serine residue 95;
(e) a compound promoting the presence or activity of a phosphatase which dephosphorylates β2-syntrophin on serine residue 110; or
(f) a compound interfering with the presence or activity of a serine/threonine kinase which phosphorylates ß2-syntrophin on serine residue 110.

8. The method of claim 7 wherein the promotion comprises the stable or transient expression from an exogenously introduced vector of said
(a) (aa) ICA512, ICA512-TMF, ICA512-CCF or PHOGRIN; or
(ab) ICA512, ICA512-TMF or ICA512-CCF, each devoid of the C-terminus or PHOGRIN devoid of the C-terminus;
(b) compound promoting the presence or activity of a protease that cleaves the C-terminus of ICA512, ICA512-TMF, ICA512-CCF or PHOGRIN;
(c) compound promoting the presence or activity of a serine/threonine kinase which phosphorylates β2-syntrophin on serine residue 95;
(d) compound interfering with the expression or activity of a serine/threonine phosphatase which dephosphorylates ß2-syntrophin on serine residue 95;
(e) compound promoting the presence or activity of a serine/threonine phosphatase which dephosphorylates β2-syntrophin on serine residue 110; or
(f) compound interfering with the expression or activity of a serine/threonine kinase which phosphorylates β2-syntrophin on serine residue 110.

9. The pharmaceutical composition of any one of claims 1 to 6, the use of any one of claims 2 to 6 or the method of claim 7 wherein the promotion comprises reducing degradation or enhancing stability in said β-cells of said
(a) (aa) ICA512, ICA512-TMF, ICA512-CCF or PHOGRIN; or
(ab) ICA512, ICA512-TMF or ICA512-CCF, each devoid of the C-terminus or PHOGRIN devoid of the C-terminus;
(b) compound promoting the presence or activity of a protease that cleaves the C-terminus of ICA512, ICA512-TMF, ICA512-CCF or PHOGRIN;
(c) compound promoting the presence or activity of a serine/threonine kinase which phosphorylates β2-syntrophin on serine residue 95;
(d) compound interfering with the presence or activity of a serine/threonine phosphatase which dephosphorylates β2-syntrophin on serine residue 95;
(e) compound promoting the presence or activity of a serine/threonine phosphatase which dephosphorylates β2-syntrophin on serine residue 110; or said
(f) compound interfering with the presence or activity of a serine/threonine kinase which phosphorylates β2-syntrophin on serine residue 110.

10. The pharmaceutical composition of any one of claims 1 to 6 and 9, the use of any one of claims 2 to 6 and 9 or the method of any one of claims 7 to 9 wherein the compound interfering with the presence or activity of said phosphatase or of said kinase is an siRNA, an shRNA, a ribozyme, a peptide aptamer, a nucleic acid based aptamer, a small molecule or an antibody.

11. A method of identifying a compound useful in the enhancement of insulin secretion from pancreatic β-cells or useful as a lead compound for developing a compound useful in the enhancement of insulin secretion from pancreatic β-cells comprising contacting a compound or a plurality of compounds suspected to be useful in the enhancement of insulin secretion from pancreatic β-cells or useful as a lead compound for developing a compound useful in the enhancement of insulin secretion from pancreatic β-cells with a mixture of (a) ICA512, ICA512-TMF, ICA512-CCF and/or PHOGRIN; and (b) β2-syntrophin and/or the PDZ domain of β2-syntrophin *in vitro* under conditions that would allow binding of ICA512, ICA512-TMF, ICA512-CCF and/or PHOGRIN to β2-syntrophin and/or the PDZ domain of β2-syntrophin in the absence of the compound or the plurality of compounds and assessing binding of ICA512, ICA512-TMF, ICA512-CCF and/or PHOGRIN to β2-syntrophin and/or the PDZ domain of β2-syntrophin wherein an interference of binding of ICA512, ICA512-TMF, ICA512-CCF and/or PHOGRIN to β2-syntrophin and/or the PDZ domain of β2-syntrophin in the presence of said compound or said plurality of compounds is indicative of said compound or at least one member of said plurality of compounds being useful in the enhancement of insulin secretion from pancreatic β-cells or useful as a lead compound for developing a compound useful in the enhancement of insulin secretion from pancreatic β-cells.

12. A method of identifying a compound useful in the treatment of diabetes or useful as a lead compound for developing a compound useful in the treatment of diabetes comprising *in silico* assessing a crystal of ICA512 or PHOGRIN or a crystal of a fragment of ICA512 or PHOGRIN comprising the PTP domain for a molecular structure fitting into the binding pocket of the PTP domain.

13. A method of identifying a compound useful for enhancing insulin secretion from pancreatic β-cells or useful as a lead compound for developing a compound useful enhancing insulin secretion from pancreatic β-cells comprising *in silico* assessing a crystal of ICA512 or PHOGRIN or a crystal of a fragment of ICA512 or PHOGRIN comprising the PTP domain for a molecular structure fitting into the binding pocket of the PTP domain.

14. A method of identifying a compound useful for enhancing insulin secretion from pancreatic β-cells or useful as a lead compound for developing a compound useful enhancing insulin secretion from pancreatic β-cells comprising *in silico* assessing a crystal of β2-syntrophin or a crystal of a fragment of β2-syntrophin comprising the PDZ domain for a molecular structure fitting into the binding pocket of the PDZ domain.

15. The method of any one of claims 12 to 14 further comprising *in silico* or *in vitro* assessing whether (a) the compound fitting into the binding pocket of the PTP domain interferes with binding of said domain or of ICA512, ICA-TMF, ICA-CCF or PHOGRIN with β2-syntrophin; or (b) the compound fitting into the binding pocket of the PDZ domain interferes with binding of said domain or of β2-syntrophin with ICA512, ICA-TMF, ICA-CCF or PHOGRIN.

16. The method of claim 15 further comprising chemically synthesizing (a) the compound fitting into the binding pocket of the PTP domain and *in silico* or *in vitro* interfering with the binding of the PTP domain or of ICA512, ICA-TMF, ICA-CCF or PHOGRIN to β2-syntrophin; or (b) the compound fitting into the binding pocket of the PDZ domain and *in silico* or *in vitro* interfering with the binding of the PDZ domain or β2-syntrophin with ICA512, ICA-TMF, ICA-CCF or PHOGRIN.

17. The pharmaceutical composition of any one of claims 1 to 6, the use of any one of claims 1 to 6 or the method of any one of claims 7 to 16 wherein said kinase is CDK5.

18. The pharmaceutical composition, use or method of any of the preceding claims wherein said compound is a small molecule, a peptide, and antibody, a peptide aptamer, a nucleic acid aptamer, a glucose metabolite, an anticalin, an siRNA, an shRNA, a ribozyme, an antisense nucleic acid molecule or a mimetic, preferably a peptidomimetic..

19. Use of a glucose metabolite or a derivative thereof for the preparation of a pharmaceutical composition for the treatment of diabetes.

20. The use of claim 19 wherein said glucose metabolite is glyceraldehyde-3-phosphate.

21. The pharmaceutical composition, use or method of any of the preceding claims wherein said diabetes is type-2 diabetes.


SEQUENCE LISTING

<110> Technische Universität Dresden

<120> Pharmaceutical composition for increasing insulin secretion from
pancreatic ß-cells

<130> N1475 EP

<140> EP 07 00 8880.2
<141> 2007-05-02

<160> 11

<170> PatentIn version 3.3

<210> 1
<211> 979
<212> PRT
<213> Homo sapiens

<400> 1

Met Arg Arg Pro Arg Arg Pro Gly Gly Leu Gly Gly Ser Gly Gly Leu
1               5                   10                  15

Arg Leu Leu Leu Cys Leu Leu Leu Leu Ser Ser Arg Pro Gly Gly Cys
            20                  25                  30

Ser Ala Val Ser Ala His Gly Cys Leu Phe Asp Arg Arg Leu Cys Ser
        35                  40                  45

His Leu Glu Val Cys Ile Gln Asp Gly Leu Phe Gly Gln Cys Gln Val
    50                  55                  60

Gly Val Gly Gln Ala Arg Pro Leu Leu Gln Val Thr Ser Pro Val Leu
65                  70                  75                  80

Gln Arg Leu Gln Gly Val Leu Arg Gln Leu Met Ser Gln Gly Leu Ser
                85                  90                  95

Trp His Asp Asp Leu Thr Gln Tyr Val Ile Ser Gln Glu Met Glu Arg
            100                 105                 110

Ile Pro Arg Leu Arg Pro Pro Glu Pro Arg Pro Arg Asp Arg Ser Gly
        115                 120                 125

Leu Ala Pro Lys Arg Pro Gly Pro Ala Gly Glu Leu Leu Leu Gln Asp
    130                 135                 140

Ile Pro Thr Gly Ser Ala Pro Ala Ala Gln His Arg Leu Pro Gln Pro
145                 150                 155                 160

Pro Val Gly Lys Gly Gly Ala Gly Ala Ser Ser Ser Leu Ser Pro Leu

                165                 170                 175

Gln Ala Glu Leu Leu Pro Pro Leu Leu Glu His Leu Leu Leu Pro Pro
            180                 185                 190

Gln Pro Pro His Pro Ser Leu Ser Tyr Glu Pro Ala Leu Leu Gln Pro
        195                 200                 205

Tyr Leu Phe His Gln Phe Gly Ser Arg Asp Gly Ser Arg Val Ser Glu
    210                 215                 220

Gly Ser Pro Gly Met Val Ser Val Gly Pro Leu Pro Lys Ala Glu Ala
225                 230                 235                 240

Pro Ala Leu Phe Ser Arg Thr Ala Ser Lys Gly Ile Phe Gly Asp His
                245                 250                 255

Pro Gly His Ser Tyr Gly Asp Leu Pro Gly Pro Ser Pro Ala Gln Leu
            260                 265                 270

Phe Gln Asp Ser Gly Leu Leu Tyr Leu Ala Gln Glu Leu Pro Ala Pro
        275                 280                 285

Ser Arg Ala Arg Val Pro Arg Leu Pro Glu Gln Gly Ser Ser Ser Arg
    290                 295                 300

Ala Glu Asp Ser Pro Glu Gly Tyr Glu Lys Glu Gly Leu Gly Asp Arg
305                 310                 315                 320

Gly Glu Lys Pro Ala Ser Pro Ala Val Gln Pro Asp Ala Ala Leu Gln
                325                 330                 335

Arg Leu Ala Ala Val Leu Ala Gly Tyr Gly Val Glu Leu Arg Gln Leu
            340                 345                 350

Thr Pro Glu Gln Leu Ser Thr Leu Leu Thr Leu Leu Gln Leu Leu Pro
        355                 360                 365

Lys Gly Ala Gly Arg Asn Pro Gly Gly Val Val Asn Val Gly Ala Asp
    370                 375                 380

Ile Lys Lys Thr Met Glu Gly Pro Val Glu Gly Arg Asp Thr Ala Glu
385                 390                 395                 400

Leu Pro Ala Arg Thr Ser Pro Met Pro Gly His Pro Thr Ala Ser Pro
                405                 410                 415

Thr Ser Ser Glu Val Gln Gln Val Pro Ser Pro Val Ser Ser Glu Pro

420                 425                 430

Pro Lys Ala Ala Arg Pro Pro Val Thr Pro Val Leu Leu Glu Lys Lys
        435                 440                 445

Ser Pro Leu Gly Gln Ser Gln Pro Thr Val Ala Gly Gln Pro Ser Ala
    450                 455                 460

Arg Pro Ala Ala Glu Glu Tyr Gly Tyr Ile Val Thr Asp Gln Lys Pro
465                 470                 475                 480

Leu Ser Leu Ala Ala Gly Val Lys Leu Leu Glu Ile Leu Ala Glu His
                485                 490                 495

Val His Met Ser Ser Gly Ser Phe Ile Asn Ile Ser Val Val Gly Pro
            500                 505                 510

Ala Leu Thr Phe Arg Ile Arg His Asn Glu Gln Asn Leu Ser Leu Ala
        515                 520                 525

Asp Val Thr Gln Gln Ala Gly Leu Val Lys Ser Glu Leu Glu Ala Gln
    530                 535                 540

Thr Gly Leu Gln Ile Leu Gln Thr Gly Val Gly Gln Arg Glu Glu Ala
545                 550                 555                 560

Ala Ala Val Leu Pro Gln Thr Ala His Ser Thr Ser Pro Met Arg Ser
                565                 570                 575

Val Leu Leu Thr Leu Val Ala Leu Ala Gly Val Ala Gly Leu Leu Val
            580                 585                 590

Ala Leu Ala Val Ala Leu Cys Val Arg Gln His Ala Arg Gln Gln Asp
        595                 600                 605

Lys Glu Arg Leu Ala Ala Leu Gly Pro Glu Gly Ala His Gly Asp Thr
    610                 615                 620

Thr Phe Glu Tyr Gln Asp Leu Cys Arg Gln His Met Ala Thr Lys Ser
625                 630                 635                 640

Leu Phe Asn Arg Ala Glu Gly Pro Pro Glu Pro Ser Arg Val Ser Ser
                645                 650                 655

Val Ser Ser Gln Phe Ser Asp Ala Ala Gln Ala Ser Pro Ser Ser His
            660                 665                 670

Ser Ser Thr Pro Ser Trp Cys Glu Glu Pro Ala Gln Ala Asn Met Asp

675                 680                 685

Ile Ser Thr Gly His Met Ile Leu Ala Tyr Met Glu Asp His Leu Arg
    690                 695                 700

Asn Arg Asp Arg Leu Ala Lys Glu Trp Gln Ala Leu Cys Ala Tyr Gln
705                 710                 715                 720

Ala Glu Pro Asn Thr Cys Ala Thr Ala Gln Gly Glu Gly Asn Ile Lys
                725                 730                 735

Lys Asn Arg His Pro Asp Phe Leu Pro Tyr Asp His Ala Arg Ile Lys
            740                 745                 750

Leu Lys Val Glu Ser Ser Pro Ser Arg Ser Asp Tyr Ile Asn Ala Ser
        755                 760                 765

Pro Ile Ile Glu His Asp Pro Arg Met Pro Ala Tyr Ile Ala Thr Gln
    770                 775                 780

Gly Pro Leu Ser His Thr Ile Ala Asp Phe Trp Gln Met Val Trp Glu
785                 790                 795                 800

Ser Gly Cys Thr Val Ile Val Met Leu Thr Pro Leu Val Glu Asp Gly
                805                 810                 815

Val Lys Gln Cys Asp Arg Tyr Trp Pro Asp Glu Gly Ala Ser Leu Tyr
            820                 825                 830

His Val Tyr Glu Val Asn Leu Val Ser Glu His Ile Trp Cys Glu Asp
        835                 840                 845

Phe Leu Val Arg Ser Phe Tyr Leu Lys Asn Val Gln Thr Gln Glu Thr
    850                 855                 860

Arg Thr Leu Thr Gln Phe His Phe Leu Ser Trp Pro Ala Glu Gly Thr
865                 870                 875                 880

Pro Ala Ser Thr Arg Pro Leu Leu Asp Phe Arg Arg Lys Val Asn Lys
                885                 890                 895

Cys Tyr Arg Gly Arg Ser Cys Pro Ile Ile Val His Cys Ser Asp Gly
            900                 905                 910

Ala Gly Arg Thr Gly Thr Tyr Ile Leu Ile Asp Met Val Leu Asn Arg
        915                 920                 925

Met Ala Lys Gly Val Lys Glu Ile Asp Ile Ala Ala Thr Leu Glu His

930                 935                 940

Val Arg Asp Gln Arg Pro Gly Leu Val Arg Ser Lys Asp Gln Phe Glu
945                 950                 955                 960

Phe Ala Leu Thr Ala Val Ala Glu Glu Val Asn Ala Ile Leu Lys Ala
                965                 970                 975

Leu Pro Gln

<210>  2
<211>  3613
<212>  DNA
<213>  Homo sapiens

<400>  2
cagcccctct ggcaggctcc cgccagcgtc gctgcggctc cggcccggga gcgagcgccc      60

ggagctcgga aagatgcggc gcccgcggcg gcctgggggt ctcgggggat ccgggggtct     120

ccggctgctc ctctgcctcc tgctgctgag cagccgcccg gggggctgca gcgccgttag     180

tgcccacggc tgtctatttg accgcaggct ctgctctcac ctggaagtct gtattcagga     240

tggcttgttt gggcagtgcc aggtgggagt ggggcaggcc cggccccttt tgcaagtcac     300

ctccccagtt ctccaacgct tacaaggtgt gctccgacaa ctcatgtccc aaggattgtc     360

ctggcacgat gacctcaccc agtatgtgat ctctcaggag atggagcgca tccccaggct     420

tcgcccccca gagccccgtc caagggacag gtctggcttg gcacccaaga gacctggtcc     480

tgctggagag ctgcttttac aggacatccc cactggctcc gcccctgctg cccagcatcg     540

gcttccacaa ccaccagtgg gcaaaggtgg agctggggcc agctcctctc tgtcccctct     600

gcaggctgag ctgctcccgc ctctcttgga gcacctgctg ctgcccccac agcctcccca     660

cccttcactg agttacgaac ctgccttgct gcagccctac ctgttccacc agtttggctc     720

ccgtgatggc tccagggtct cagagggctc cccagggatg gtcagtgtcg gccccctgcc     780

caaggctgaa gcccctgccc tcttcagcag aactgcctcc aagggcatat ttggggacca     840

ccctggccac tcctacgggg accttccagg gccttcacct gcccagcttt ttcaagactc     900

tgggctgctc tatctggccc aggagttgcc agcacccagc agggccaggg tgccaaggct     960

gccagagcaa gggagcagca gccgggcaga ggactcccca gagggctatg agaaggaagg    1020

actaggggat cgtggagaga agcctgcttc cccagctgtg cagccagatg cggctctgca    1080

gaggctggcc gctgtgctgg cgggctatgg ggtagagctg cgtcagctga cccctgagca    1140

gctctccaca ctcctgaccc tgctgcagct actgcccaag ggtgcaggaa gaaatccggg    1200

aggggttgta aatgttggag ctgatatcaa gaaaacaatg gaggggccgg tggagggcag    1260

agacacagca gagcttccag cccgcacatc ccccatgcct ggacacccca ctgccagccc    1320

tacctccagt gaagtccagc aggtgccaag ccctgtctcc tctgagcctc ccaaagctgc 1380

cagaccccct gtgacacctg tcctgctaga gaagaaaagc ccactgggcc agagccagcc 1440

cacggtggca ggacagccct cagcccgccc agcagcagag gaatatggct acatcgtcac 1500

tgatcagaag cccctgagcc tggctgcagg agtgaagctg ctggagatcc tggctgagca 1560

tgtgcacatg tcctcaggca gcttcatcaa catcagtgtg gtgggaccag ccctcacctt 1620

ccgcatccgg cacaatgagc agaacctgtc tttggctgat gtgacccaac aagcagggct 1680

ggtgaagtct gaactggaag cacagacagg gctccaaatc ttgcagacag gagtgggaca 1740

gagggaggag gcagctgcag tccttcccca aactgcgcac agcacctcac ccatgcgctc 1800

agtgctgctc actctggtgg ccctggcagg tgtggctggg ctgctggtgg ctctggctgt 1860

ggctctgtgt gtgcggcagc atgcgcggca gcaagacaag gagcgcctgg cagccctggg 1920

gcctgagggg gcccatggtg acactacctt tgagtaccag gacctgtgcc gccagcacat 1980

ggccacgaag tccttgttca accgggcaga gggtccaccg gagccttcac gggtgagcag 2040

tgtgtcctcc cagttcagcg acgcagccca ggccagcccc agctcccaca gcagcacccc 2100

gtcctggtgc gaggagccgg cccaagccaa catggacatc tccacgggac acatgattct 2160

ggcatacatg gaggatcacc tgcggaaccg ggaccgcctt gccaaggagt ggcaggccct 2220

ctgtgcctac caagcagagc caaacacctg tgccaccgcg cagggggagg gcaacatcaa 2280

aaagaaccgg catcctgact tcctgcccta tgaccatgcc cgcataaaac tgaaggtgga 2340

gagcagccct tctcggagcg attacatcaa cgccagcccc attattgagc atgaccctcg 2400

gatgccagcc tacatagcca cgcagggccc gctgtcccat accatcgcag acttctggca 2460

gatggtgtgg gagagcggct gcaccgtcat cgtcatgctg accccgctgg tggaggatgg 2520

tgtcaagcag tgtgaccgct actggccaga tgagggtgcc tccctctacc acgtatatga 2580

ggtgaacctg gtgtcggagc acatctggtg cgaggacttt ctggtgcgga gcttctacct 2640

gaagaacgtg cagacccagg agacgcgcac gctcacgcag ttccacttcc tcagctggcc 2700

ggcagagggc acaccggcct ccacgcggcc cctgctggac ttccgcagga aggtgaacaa 2760

gtgctaccgg ggccgctcct gccccatcat cgtgcactgc agtgatggtg cggggaggac 2820

cggcacctac atcctcatcg acatggtcct gaaccgcatg gcaaaaggag tgaaggagat 2880

tgacatcgct gccaccctgg agcatgtccg tgaccagcgg cctggccttg tccgctctaa 2940

ggaccagttt gaatttgccc tgacagccgt ggcggaggaa gtgaatgcca tcctcaaggc 3000

cctgccccag tgagaccctg gggccccttg gcgggcagcc cagcctctgt ccctctttgc 3060

ctgtgtgagc atctctgtgt acccactcct cactgcccca ccagccacct cttgggcatg 3120

ctcagccctt cctagaagag tcaggaaggg aaagccagaa ggggcacgcc tgcccagcct 3180

cgcatgccag agcctggggc atcccagagc ccagggcatc ccatgggggt gctgcagcca 3240

ggaggagagg aaaggacatg ggtagcaatt ctacccagag ccttctcctg cctacattcc 3300

ctggcctggc tctcctgtag ctctcctggg gttctgggag ttccctgaac atctgtgtgt 3360

gtccccctat gctccagtat ggaagaatgg ggtggagggt cgccacaccc ggctccccct 3420

gcttctcagc cccgggcctg cctctgactc acacttgggc gctctgccct ccctggcctc 3480

acgcccagcc tggtcccacc accctcccac catgcgctgc tcaacctctc tccttctggc 3540

gcaagagaac atttctagaa aaaactactt ttgtaccagt gtgaataaag ttagtgtgtt 3600

gtctgtgcag ctg 3613

<210> 3
<211> 9771
<212> DNA
<213> Homo sapiens

<400> 3
gccgaggctg cctgactgga atgagggtag ctgcggcgac tgcggcggct ggagcggggc 60

cggccatggc ggtgtggacg cgggccacca aagcggggct ggtggagctg ctcctgaggg 120

agcgctgggt ccgagtggtg gccgagctga gcggggagag cctgagcctg acgggcgacg 180

ccgccgcggc cgagctggag cccgctctgg gacccgcggc cgccgccttc aacggcctcc 240

caaacggcgg cggcgcgggc gactcgctgc ccgggagccc aagccgcggc ctggggcccc 300

cgagcccgcc ggcgccgcct cggggccccg cgggtgaggc gggcgcgtcg ccgcccgtgc 360

gccgggtgcg ggtggtgaag caagaggcgg gcggcctggg catcagcatc aagggcggcc 420

gcgagaaccg gatgccgatc ctcatctcca agatcttccc cgggctggct gccgaccaga 480

gccgggcgct gcggctgggc gacgccatcc tgtcggtgaa cggcaccgac ctgcgccagg 540

ccacccacga ccaggccgtg caggcgctga agcgcgcggg caaggaggtg ctgctggagg 600

tcaagttcat ccgagaagta acaccatata tcaagaagcc atcattagta tcagatctgc 660

cgtgggaagg tgcagccccc cagtcaccaa gctttagtgg cagtgaggac tctggttcgc 720

caaaacacca gaacagcacc aaggacagga agatcatccc tctcaaaatg tgctttgctg 780

ctagaaacct aagcatgccg gatctggaaa acagattgat agagctacat tctcctgata 840

gcaggaacac gttgatccta cgctgcaaag atacagccac agcacactcc tggttcgtag 900

ctatccacac caacataatg gctctcctcc cacaggtgtt ggctgaactc aacgccatgc 960

ttggggcaac cagtacagca ggaggcagta aagaggtgaa gcatattgcc tggctggcag 1020

aacaggcaaa actagatggt ggaagacagc aatggagacc tgtcctcatg gctgtgactg 1080

agaaggattt gctgctctat gactgtatgc cgtggacaag agatgcctgg gcgtcaccat 1140

gccacagcta cccacttgtt gccaccaggt tggttcattc tggctccgga tgtcgatccc 1200

cctcccttgg atctgacctt acatttgcta ccaggacagg ctctcgacag ggcattgaga 1260

tgcatctctt cagggtggag acacatcggg atctgtcatc ctggaccagg atacttgttc 1320
agggttgcca tgctgctgct gagctgatca aggaagtctc tctaggctgc atgttaaatg 1380
gccaagaggt gaggcttact attcactatg aaaatgggtt caccatctca agggaaaatg 1440
gaggctccag cagcatattg taccgctacc cctttgaaag gctgaagatg tctgctgatg 1500
atggcatccg aaatctatac ttggattttg gtggtcccga gggagaactg accatggacc 1560
tgcactcttg tccgaagccg attgtatttg tgttgcacac gtttttatcg gccaaagtca 1620
ctcgtatggg actgcttgta tgagcaacaa aaaatcagaa aagagccttg actgtcacaa 1680
gaaatatttc cacctcaaaa aaaaaaaagc acaaaaagaa actctttgct ctccttcagc 1740
acagtgcctt cccaaggacc tgcaaataac tgctgaacca taaccgtgtt taaagaagag 1800
cctacctttc acagtctacc ttggccagat attctagcac tctaaaaggc tccaaaatga 1860
agctgttgat ttattctcat ttcaaaatat ggtttatgag taattaggtt tatttctact 1920
gctaaaaaga atggctcatt gttttctttt tttttcattg ttttcatttt tagacataat 1980
caaataccca agcattgttt tctttttctt ctccctgtag gatttgtgtt gtgcttagcc 2040
ttgatctctt gttcagaaca attgtccctt cccaggtgct agcttgcagc agcctgttgc 2100
ttcctgagca ttccagatag gtcgtatgtc ttggaattta ctgtcctcac tagctccttc 2160
caccatagga agtccttgaa ttgtatcacc aaagggttgt tccatttgcc ctggatctcc 2220
ctctatctca gcctggccat cacttcccac cttatcccaa gctttgccac tgtctctagg 2280
acttagaact tgtaaatttg atttgccttg ccttctactt cctttctagt ggtgactgag 2340
ttgaaaagca agttggaagc cactgctttt aaagaaaagt tttgcttggt ttaggttttt 2400
ccaattttat ttttgttagg agccagggga taagatttat tttaaatgaa aatcctcttc 2460
ctaggttaag ccattttttt tattgcttac caaatgtgcc tttggttagg gttagtggag 2520
ctttattagt cactatttga ataactggaa atcactgcca tttccaggag atcacctatc 2580
ctagttttga ggaatagtca ttgaggtggg tttccttaat ggcatctctt ctagtaggca 2640
gatctgtttg aacaagataa atcctaaaga gccagcctgc ttgaggagta gacttggtgg 2700
gtgaagccag caattccgca caaacgtcat gttgaattgt tttgggctgc ccaaaaacaa 2760
caggatgcag catcctggct gtggcctggc cctttgtggc agcagtggca tggacaagca 2820
actgctaatt cgagacttac tattggcttc acagcacacc ctacagtgag cagggtgatg 2880
agaccgtgga acagctacct gcttttggtt aagattgcca gatatcatta ggacacagta 2940
gcagcaagaa tgctgatttt gtagtgttag aaaatgaact cagcttgttt ttcctaattt 3000
tgaaggtggt atatatctgc aaacatttaa aatggtataa tatcatataa aatgtcaaaa 3060
ttagcacagt ccctggatct caaagaatag gtaatattga cttggatgtt ctttatgctc 3120
tctgaagaaa ggtctaggga aagttcttgt ttgcttgatt gatcaagagt gccacggaag 3180

aatgtgagcg ctgtggaggg tggaggaggg tgtcacttca gatggggcag gcacaggggg 3240
agaaaagaag agaaaaacac acctgtgtgc aaaatgagct atttggtttc agtgcggcct 3300
gaccgatggg gaggacccat gagcagagag agttctaggt caacattgta actccattgt 3360
cctccttcac ccataagtca gtcctccttc ctcattacag tgaaaccaac aatatgcaga 3420
ggttggggag agatgcctgt tcttccccca gcttcatata attgtcaaag ttccaaaaat 3480
gaattatctg aactgcttaa aactggaaat taaaatctag acaagaatat gtctttcaag 3540
agctttttat gttttgaaag ttcataaacc taagattggc tccatctgag ataaattttc 3600
ttttcttttt ttttttgaga cggagtctcg ttctgtctcc caggctggag tgcagtggca 3660
tgatctcggc tcactgcaac ctccacctcc taggttcaag tgattctcct gagtagctgg 3720
ggttatagct acccaccacc acacccagct aatttttgta tttttagtag aggcaggttt 3780
tcaccatgtt ggctaggctg gtcttgaact cccgacctca agtgatccac ctgccttggc 3840
ctcccaaagt gctgggatta taggtgtgag cccccgtgcc tggcccacaa taaattttca 3900
tttcagagaa ggaaaaaaat ctcttgaacg tccagaaatg atttctggaa tcagaatttc 3960
aatatgtaat accctcaggt aggcaatctt gaaccctcta tgcagtgaga gcattattaa 4020
tatgagatgt tgaatgatgc ctttacagac ttcagcagca aagccatttg gttgaggttt 4080
tctgtgttaa tgtgaaaagc taagccactg agatttgcag gggagcttct gagccaccct 4140
gcttgttggt tatatgagga tgtaaaatag gtggaattaa aatgtgagca ggaaaggcaa 4200
ggcatgtgtg cattttcact cattttggtc aaggttaaac tgacatgtat agcactgagg 4260
tctgtagggt tcttaatatc ttttgagtct tgctgtgaaa tgaaagtgtg ggaggtttcc 4320
tttgtccatt agcagcccca agaaccagaa cccttttgct gcttttctta catacctaac 4380
agctctccag tcatgatgac caaggttgtt cttcaatcaa atgtgtttgt gggattttca 4440
gtccgcaaat gaagtgctct ctaatgaatg ggacaccatg ataaatatgt atttatattt 4500
agatgccaaa gtatggcaaa ttatttccaa atgataacta caaatgggaa ttttcgatat 4560
tctacctttt ttatagaacc agctcacttt tcatttcttt ttcattttga attaagaaaa 4620
ttgttgagga tgtggtgggt tccagtgtgt ggaatggaaa ggaaactgca gaatagtgtc 4680
tgctccccat tcagagggac tgcttcctgt gccccccaga cccggggctt cgacagcttc 4740
tccacattcc acacagatgc ctaggagcag cgagttggta tatgaaaagt ctcccacctt 4800
ttctcctaaa acttctctcc tttctctcca taaaaagaaa aggaaaggaa caaaagaaaa 4860
acattcagtt tttctttttc tgaaaaaggt aagtcctttc ctgaagtcat caaatgaaac 4920
attatctgga aattagtttc taatgttgta tatgaagaaa tacttaaata taagttcctg 4980
cagtatttat tagatagttg taactgtaaa ctcacctccc tagtagataa gagtttcagg 5040
ttaaatactg gaacatatat aggcagtcaa aaatactact ttaaatgtca ttcacctatt 5100

ttaaagccat gttttagcac tttttaggcc aaagaaggtc tgatagtgcc tgtttttatg 5160
ttctgtactc tcacaaactt tgttactcaa aattattgca tggcaggaga gattggatta 5220
tttatttctt atatttttat aagtaaaaaa atctttctaa acaacaaata cctaacatta 5280
ttactgattg ttttcctaat ttatcctcct aagttgaatg gtaacaaagc ttttccagct 5340
gaatgaatgc acttagctga taaaccagaa tttgttcttt tttttccttc tttttttttt 5400
gagacaggtt ctcactctgt caccgaggtt ggagtgcagt ggtatgatca tagctgactg 5460
cagcctcaac cttctgggct caaatgatcc tttcacctca gcctcctgag tagctgggac 5520
cacaggtgtg tgccaccaca cctggctaat ttttatggat ttttttttct tttttttttt 5580
tcgccatgtt gcccaggctg gtcttgaact cttggcactc aagcagtcct tctgcctcag 5640
cctcccaaag tgctgggatt acagatgtga gccactgcac ctggccccag aaattatgct 5700
taaaaaaaaa attaagctgt gtggaatctg gacaaggcta tgtttctcct agaaacaggt 5760
tttgctgtaa atggttttga tgggcgaaag tgtgagaaaa ggttataata gattcttttt 5820
tttaattttg tgagaaattc caggtcttct tgcttaactt atatgcatgt ggatatattt 5880
gttttcaaag ccatggaaga atctctgttc atatatttta atgcaaatta ccttgtatgc 5940
tgctatgcaa attctattaa aaggcctacc tgcttaagaa ttaaacattt ttgaaacttt 6000
cagggaagac ctgtagactc aagcactttc tctgcttttt tgtatcttat cttggacact 6060
tatgctgaat tatgttctta ttattttcac tggttataag ctattccttg tacataatat 6120
ttaaactatc caaatatcca gcatacattt ccatgtggct gccatcggct gttgatgcca 6180
tgaatgaaat ggctggttag aaagccaaag gtcttctttt ttcaattcct aatgaataag 6240
taaaatgcca gatctctttg gtctcaaagc aaattgctct gatcagatca aatatctatg 6300
taatgaaaag agacttactt aaatttggga ttctgcttaa actttaagtt gaatttgact 6360
gtagtcattc attcttttta tatatctctg tcaggtatga cattaattac caggtggtat 6420
tatactctac tttgagtttg gacatcactt tcagttaaat ttctgttgtt attgtggctt 6480
tttttttttt ttaaccccac tgaagatgga agtagagcag tctgccatct cctgctgttc 6540
tttctctgtg ccccctaatt gcttggtaat ctaaaattat tctgactttc cacatactgt 6600
ctttaaaaac caattgtagc aatctgcccg cctccaccct ttaagctaaa tctttcactg 6660
tgctggatac atagacactc gataaatgtg tgatgatagt tctaaaataa ttatgctata 6720
tatcagggtt ctgtattttt gagcaatatc attattccta tgctgtgatg ttaaatggaa 6780
tttattttac actagtaata gactgcttaa gtggctgatt tgaaaccttt ctctaaaaaa 6840
acaaaaacat ctggcagatg agtgacacaa attcattctc ttaaaggata gaatttcaag 6900
ttaaggataa ggcacaataa ataggcacat agaggacatc tgtttttgtt tttggttttg 6960
gttttgtttt tttttgtctc tctgactagc taaactgcca actagaagaa tggttgttat 7020

gtagtaactc agaagaatct tttaataagt ttcttctttt gtttcaaaac aaacttgagc 7080
ttcatttctt gccacctcta ttctttcttc ccacgttggg tgactgaact gatgcttatt 7140
ctagttttag aagaggatcc acatttaatg taaggcataa atatgccttt tgcaaattat 7200
tgcttgttct ccagtgaaca tttgatcata ttcaacagaa atccaccata taaaccataa 7260
tccttgaatt gcccctttta aaatagattg gttaaaacca aaaacctata agagagaaat 7320
gatatttcat tgtattgtag gtttgggaat cctggttaga tttataattt ctggccgggt 7380
gcggtggctc atgcctgtat tcccagtact ttgggagccc aaggtgggtg gatcacctga 7440
ggtaaggagt ttgagaccag cctggccaac atggtgaaac cccatctcta ctaaaaatac 7500
aaaaagttag ctgggcatgg tggtgcgcgc ctgtggtccc agctactcgg gaggctgagg 7560
tgggagaatc ccttgaaccc aggaggcaga agttgcagtg agctgagatc atgccattgc 7620
actccagcct gggcaacaga gcgagacttc atctccaaaa aaagaaaaag aagaaaaaga 7680
tttataattt ctgagtcccc taagtccctt tctcagtttg caataatctt acgtccatgc 7740
tattgacact aattttgaaa ctggaaagct aattaatgac atattcatgt gttctttcat 7800
ctaataaaca tttattaatg tatagttcta caccaggcat tctgaagggt ccaaaagaaa 7860
gttcctgccc tctagtagca cataatccgt tatttctcag cacaccatgc catctgttta 7920
tcattcagca gttaagtaat tcatcacttc atatttttac caaagtatat ttacaagtct 7980
gtttatctgg atctggcaaa ctggaaaatg tttgaaggga tactcaggct ttgatccatg 8040
ccaacatgaa aggactccaa gcacatttaa gtaggctggt cttttcatca tgcatgattt 8100
tttttttagt gcaaatttag actttagttt gggaaagaga acagtataca gtatcctttg 8160
taagataaat cacaacaaca aaaattaagt tttattgcac aaagtagata aatgagaaag 8220
tctttcttaa aaaatgcttc tttaactgta acagcaaaac taaatgcata ttttatcaac 8280
cccttatttc taattgctgt attcatttcc acccttaaac tatttaagca tttcatctgc 8340
tgaaagtcct tatatacatg gtagacggaa tatttctcca ttaatgatag atcaaaatga 8400
agatatttaa aaattaacaa aatcaggcca aggcaggagg atcactttag cccaggaggt 8460
ccagaccagc ctgggcaaca cagcgggacc ccgactccat ttaaaaaatt aaaaaaaaaa 8520
aaaaaacttt taacatttaa aaaataaaaa ttaacaaaat ttcacttatt ccaggacacg 8580
ctggcatttg gactcaatga aaagggcacc taaagaaaat aaggctgact gaatgttttc 8640
cataattttc acacaataac agtccctttc tatccagctt gccttccatt tatctctagg 8700
gttagctttt caggcaacat ccttggtcat tgcccagaaa gtacctgagc tatcagtgat 8760
tggaatggca caggaaaccg aatcacatgg gtgccctccc cttggttttc aagtatcttg 8820
gagttgtgca caaaaattag gtcatgcctt cagtgtcttg ttctttaaac ctaccctttg 8880
acaatcaggt gctaatgatt gtatactatt aaaaccagca cataagtatt gtaaatgtgt 8940

gttcctccta ggttggaaga aatgtctttc cttctatctg ggtcctgtta aagcgggtgt 9000

cagttgtgtc ttttcacctc gatttgtgaa ttaatagaat tggggggaga ggaaatgatg 9060

atgtcaatta agtttcaggt ttggcatgat catcattctc gatgatattc tcactttgtc 9120

gcaaatctgc ccttatcgta agaacaagtt tcagaatttt ccctccacta tacgactcca 9180

gtattatgtt tacaatccat tggatgagtg cagcattata agaccttggt gcccagaaaa 9240

atctgtcctt tttggtacca aacctgaggt cttttggaag ataatgtaga aaaccactac 9300

ctattgaagg cctgttttgg ctaatctgtg caaactctga tgatacctgc ttatgtggat 9360

tcttttccac actgctttca tttttaagta taaagactta gaaaactaga ataatgcttt 9420

tacaaataat taaaagtatg tgatgttctg ggttttttcc ttctttttag aaccctgtat 9480

ttaaacaagc cttcttttta agtcttgttt gaaatttaag tctcagatct tctggatacc 9540

aaatcaaaaa cccaacgcgt aaaacagggc agtatttgtg ttcctaattt taaaaagctt 9600

tatgtatact ctataaatat agatgcataa acaacacttc cccttgagta gcacatcaac 9660

atacagcatt gtacattaca atgaaaatgt gtaacttaag ggtattatat atataaatac 9720

atatatacct ttgtaacctt tatactgtaa ataaaaaagt tgctttagtc a 9771

<210> 4
<211> 540
<212> PRT
<213> Homo sapiens

<400> 4

Met Arg Val Ala Ala Ala Thr Ala Ala Ala Gly Ala Gly Pro Ala Met
1               5                   10                  15

Ala Val Trp Thr Arg Ala Thr Lys Ala Gly Leu Val Glu Leu Leu Leu
            20                  25                  30

Arg Glu Arg Trp Val Arg Val Val Ala Glu Leu Ser Gly Glu Ser Leu
        35                  40                  45

Ser Leu Thr Gly Asp Ala Ala Ala Ala Glu Leu Glu Pro Ala Leu Gly
    50                  55                  60

Pro Ala Ala Ala Ala Phe Asn Gly Leu Pro Asn Gly Gly Gly Ala Gly
65                  70                  75                  80

Asp Ser Leu Pro Gly Ser Pro Ser Arg Gly Leu Gly Pro Pro Ser Pro
                85                  90                  95

Pro Ala Pro Pro Arg Gly Pro Ala Gly Glu Ala Gly Ala Ser Pro Pro
            100                 105                 110

Val Arg Arg Val Arg Val Val Lys Gln Glu Ala Gly Gly Leu Gly Ile
        115                 120                 125

Ser Ile Lys Gly Gly Arg Glu Asn Arg Met Pro Ile Leu Ile Ser Lys
    130                 135                 140

Ile Phe Pro Gly Leu Ala Ala Asp Gln Ser Arg Ala Leu Arg Leu Gly
145                 150                 155                 160

Asp Ala Ile Leu Ser Val Asn Gly Thr Asp Leu Arg Gln Ala Thr His
                165                 170                 175

Asp Gln Ala Val Gln Ala Leu Lys Arg Ala Gly Lys Glu Val Leu Leu
            180                 185                 190

Glu Val Lys Phe Ile Arg Glu Val Thr Pro Tyr Ile Lys Lys Pro Ser
        195                 200                 205

Leu Val Ser Asp Leu Pro Trp Glu Gly Ala Ala Pro Gln Ser Pro Ser
    210                 215                 220

Phe Ser Gly Ser Glu Asp Ser Gly Ser Pro Lys His Gln Asn Ser Thr
225                 230                 235                 240

Lys Asp Arg Lys Ile Ile Pro Leu Lys Met Cys Phe Ala Ala Arg Asn
                245                 250                 255

Leu Ser Met Pro Asp Leu Glu Asn Arg Leu Ile Glu Leu His Ser Pro
            260                 265                 270

Asp Ser Arg Asn Thr Leu Ile Leu Arg Cys Lys Asp Thr Ala Thr Ala
        275                 280                 285

His Ser Trp Phe Val Ala Ile His Thr Asn Ile Met Ala Leu Leu Pro
    290                 295                 300

Gln Val Leu Ala Glu Leu Asn Ala Met Leu Gly Ala Thr Ser Thr Ala
305                 310                 315                 320

Gly Gly Ser Lys Glu Val Lys His Ile Ala Trp Leu Ala Glu Gln Ala
                325                 330                 335

Lys Leu Asp Gly Gly Arg Gln Gln Trp Arg Pro Val Leu Met Ala Val
            340                 345                 350

Thr Glu Lys Asp Leu Leu Leu Tyr Asp Cys Met Pro Trp Thr Arg Asp
        355                 360                 365

Ala Trp Ala Ser Pro Cys His Ser Tyr Pro Leu Val Ala Thr Arg Leu
    370                 375                 380

Val His Ser Gly Ser Gly Cys Arg Ser Pro Ser Leu Gly Ser Asp Leu
385                 390                 395                 400

Thr Phe Ala Thr Arg Thr Gly Ser Arg Gln Gly Ile Glu Met His Leu
                405                 410                 415

Phe Arg Val Glu Thr His Arg Asp Leu Ser Ser Trp Thr Arg Ile Leu
            420                 425                 430

Val Gln Gly Cys His Ala Ala Ala Glu Leu Ile Lys Glu Val Ser Leu
        435                 440                 445

Gly Cys Met Leu Asn Gly Gln Glu Val Arg Leu Thr Ile His Tyr Glu
    450                 455                 460

Asn Gly Phe Thr Ile Ser Arg Glu Asn Gly Gly Ser Ser Ser Ile Leu
465                 470                 475                 480

Tyr Arg Tyr Pro Phe Glu Arg Leu Lys Met Ser Ala Asp Asp Gly Ile
                485                 490                 495

Arg Asn Leu Tyr Leu Asp Phe Gly Gly Pro Glu Gly Glu Leu Thr Met
            500                 505                 510

Asp Leu His Ser Cys Pro Lys Pro Ile Val Phe Val Leu His Thr Phe
        515                 520                 525

Leu Ser Ala Lys Val Thr Arg Met Gly Leu Leu Val
    530                 535                 540

<210>  5
<211>  520
<212>  PRT
<213>  Mus musculus

<400>  5

Met Ala Val Trp Thr Arg Ala Thr Lys Ala Gly Leu Val Glu Leu Leu
1               5                   10                  15

Leu Arg Glu Arg Trp Val Arg Val Val Ala Glu Leu Ser Gly Glu Ser
            20                  25                  30

Leu Ser Leu Thr Gly Asp Ala Ala Ala Val Glu Pro Glu Pro Pro Ala
        35                  40                  45

Ala Ala Phe Asn Gly Leu Pro Asn Gly Gly Gly Gly Glu Ser Leu Pro

50                  55                  60

Gly Ser Pro Asn Arg Gly Leu Gly Pro Pro Ser Pro Pro Ala Pro Pro
65                  70                  75                  80

Arg Gly Pro Ala Gly Glu Ala Ser Ala Ser Pro Pro Val Arg Arg Val
                85                  90                  95

Arg Val Val Lys Gln Glu Ala Gly Gly Leu Gly Ile Ser Ile Lys Gly
            100                 105                 110

Gly Arg Glu Asn Arg Met Pro Ile Leu Ile Ser Lys Ile Phe Pro Gly
        115                 120                 125

Leu Ala Ala Asp Gln Ser Arg Ala Leu Arg Leu Gly Asp Ala Ile Leu
    130                 135                 140

Ser Val Asn Gly Thr Asp Leu Arg Gln Ala Thr His Asp Gln Ala Val
145                 150                 155                 160

Gln Ala Leu Lys Arg Ala Gly Lys Glu Val Leu Leu Glu Val Lys Phe
                165                 170                 175

Ile Arg Glu Val Thr Pro Tyr Ile Lys Lys Pro Ser Leu Val Ser Asp
            180                 185                 190

Leu Pro Trp Glu Gly Ala Ser Pro Gln Ser Pro Ser Phe Ser Gly Ser
        195                 200                 205

Glu Asp Ser Gly Ser Pro Lys His Gln Asn Thr Thr Lys Asp Arg Lys
    210                 215                 220

Val Ile Pro Leu Lys Met Cys Phe Ala Ala Arg Asn Leu Ser Met Pro
225                 230                 235                 240

Asp Leu Glu Asn Arg Leu Ile Glu Leu His Ser Pro Asp Ser Arg Asn
                245                 250                 255

Thr Leu Ile Leu Arg Cys Lys Asp Thr Ala Thr Ala His Ser Trp Phe
            260                 265                 270

Val Ala Ile His Thr Asn Ile Met Ala Leu Leu Pro Gln Val Leu Ala
        275                 280                 285

Glu Leu Asn Ala Met Leu Gly Ala Thr Ser Thr Ala Gly Gly Ser Lys
    290                 295                 300

Glu Val Lys His Ile Ala Trp Leu Ala Glu Gln Ala Lys Leu Asp Gly

305                 310                 315                 320

Gly Arg Gln Gln Trp Arg Pro Val Leu Met Ala Val Thr Glu Lys Asp
                325                 330                 335

Leu Leu Leu Tyr Asp Cys Met Pro Trp Thr Arg Asp Ala Trp Ala Ser
            340                 345                 350

Pro Cys His Ser Tyr Pro Leu Val Ala Thr Arg Leu Val His Ser Gly
        355                 360                 365

Ser Gly Cys Arg Ser Pro Ser Leu Gly Ser Asp Leu Thr Phe Ala Thr
    370                 375                 380

Arg Thr Gly Ser Arg Gln Gly Ile Glu Met His Leu Phe Arg Val Glu
385                 390                 395                 400

Thr His Arg Asp Leu Ser Thr Trp Thr Arg Ile Leu Val Gln Gly Cys
                405                 410                 415

His Ala Ala Ala Glu Leu Ile Lys Glu Val Ser Leu Gly Cys Thr Leu
            420                 425                 430

Ser Gly Gln Glu Val Arg Phe Thr Val His Tyr Glu His Gly Phe Thr
        435                 440                 445

Ile Thr Arg Asp Asn Gly Gly Ala Ser Ser Ile Leu Tyr Arg Tyr Pro
    450                 455                 460

Phe Glu Arg Leu Lys Met Ser Ala Asp Asp Gly Ile Arg Asn Leu Tyr
465                 470                 475                 480

Leu Asp Phe Gly Gly Pro Glu Gly Glu Leu Thr Met Asp Leu His Ser
                485                 490                 495

Cys Pro Lys Pro Ile Val Phe Val Leu His Thr Phe Leu Ser Ala Lys
            500                 505                 510

Val Thr Arg Met Gly Leu Leu Val
        515                 520

<210>  6
<211>  4767
<212>  DNA
<213>  Homo sapiens

<400>  6
gcctcccgcc gcctcccgcg cggccatgga ctgagcgccg ccggccaggc cgcggggatg      60

gggccgccgc tcccgctgct gctgctgcta ctgctgctgc tgccgccacg cgtcctgcct     120

gccgcccctt cgtccgtccc ccgcggccgg cagctcccgg ggcgtctggg ctgcctgctc 180
gaggagggcc tctgcggagc gtccgaggcc tgtgtgaacg atggagtgtt tggaaggtgc 240
cagaaggttc cggcaatgga cttttaccgc tacgaggtgt cgcccgtggc cctgcagcgc 300
ctgcgcgtgg cgttgcagaa gctttccggc acaggtttca cgtggcagga tgactatact 360
cagtatgtga tggaccagga acttgcagac ctcccgaaaa cctacctgag gcgtcctgaa 420
gcatccagcc cagccaggcc ctcaaaacac agcgttggca gcgagaggag gtacagtcgg 480
gagggcggtg ctgccctggc caacgccctc cgacgccacc tgcccttcct ggaggccctg 540
tcccaggccc cagcctcaga cgtgctcgcc aggacccata cggcgcagga cagacccccc 600
gctgagggtg atgaccgctt ctccgagagc atcctgacct atgtggccca cacgtctgcg 660
ctgacctacc ctcccgggcc ccggacccag ctccgcgagg acctcctgcc gcggaccctc 720
ggccagctcc agccagatga gctcagccct aaggtggaca gtggtgtgga cagacaccat 780
ctgatggcgg ccctcagtgc ctatgctgcc cagaggcccc cagctccccc cggggagggc 840
agcctggagc cacagtacct tctgcgtgca ccctcaagaa tgcccaggcc tttgctggca 900
ccagccgccc cccagaagtg gccttcacct ctgggagatt ccgaagaccc ctccagcaca 960
ggcgatggag cacggattca taccctcctg aaggacctgc agaggcagcc ggctgaggtg 1020
aggggcctga gtggcctgga gctggacggc atggctgagc tgatggctgg cctgatgcaa 1080
ggcgtggacc atggagtagc tcgaggcagc cctgggagag cggccctggg agagtctgga 1140
gaacaggcgg atggccccaa ggccaccctc cgtggagaca gctttccaga tgacggagtg 1200
caggacgacg atgatagact ttaccaagag gtccatcgtc tgagtgccac actcgggggc 1260
ctcctgcagg accacgggtc tcgactctta cctggagccc tcccctttgc aaggcccctc 1320
gacatggaga ggaagaagtc cgagcaccct gagtcttccc tgtcttcaga agaggagact 1380
gccggagtgg agaacgtcaa gagccagacg tattccaaag atctgctggg gcagcagccg 1440
cattcggagc ccggggccgc tgcgtttggg gagctccaaa accagatgcc tgggccctcg 1500
aaggaggagc agagccttcc agcgggtgct caggaggccc tcagcgacgg cctgcaattg 1560
gaggtccagc cttccgagga agaggcgcgg ggctacatcg tgacagacag agaccccctg 1620
cgccccgagg aaggaaggcg gctggtggag gacgtcgccc gcctcctgca ggtgcccagc 1680
agtgcgttcg ctgacgtgga ggttctcgga ccagcagtga ccttcaaagt gagcgccaat 1740
gtccaaaacg tgaccactga ggatgtggag aaggccacag ttgacaacaa agacaaactg 1800
gaggaaacct ctggactgaa aattcttcaa accggagtcg ggtcgaaaag caaactcaag 1860
ttcctgcctc ctcaggcgga gcaagaagac tccaccaagt tcatcgcgct caccctggtc 1920
tccctcgcct gcatcctggg cgtcctcctg gcctctggcc tcatctactg cctccgccat 1980
agctctcagc acaggctgaa ggagaagctc tcgggactag ggggcgaccc aggtgcagat 2040

gccactgccg cctaccagga gctgtgccgc cagcgtatgg ccacgcggcc accagaccga 2100
cctgagggcc cgcacacgtc acgcatcagc agcgtctcat cccagttcag cgacgggccg 2160
atccccagcc cctccgcacg cagcagcgcc tcatcctggt ccgaggagcc tgtgcagtcc 2220
aacatggaca tctccaccgg ccacatgatc ctgtcctaca tggaggacca cctgaagaac 2280
aagaaccggc tggagaagga gtgggaagcg ctgtgcgcct accaggcgga gcccaacagc 2340
tcgttcgtgg cccagaggga ggagaacgtg cccaagaacc gctccctggc tgtgctgacc 2400
tatgaccact cccgggtcct gctgaaggcg gagaacagcc acagccactc agactacatc 2460
aacgctagcc ccatcatgga tcacgacccg aggaaccccg cgtacatcgc cacccaggga 2520
ccgctgcccg ccaccgtggc tgacttttgg cagatggtgt gggagagcgg ctgcgtggtg 2580
atcgtcatgc tgacacccct cgcggagaac ggcgtccggc agtgctacca ctactggccg 2640
gatgaaggct ccaatctcta ccacatctat gaggtgaacc tggtctccga gcacatctgg 2700
tgtgaggact tcctggtgag gagcttctat ctgaagaacc tgcagaccaa cgagacgcgc 2760
accgtgacgc agttccactt cctgagttgg tatgaccgag gagtcccttc ctcctcaagg 2820
tccctcctgg acttccgcag aaaagtaaac aagtgctaca ggggccgttc ttgtccaata 2880
attgttcatt gcagtgacgg tgcaggccgg agcggcacct acgtcctgat cgacatggtt 2940
ctcaacaaga tggccaaagg tgctaaagag attgatatcg cagcgaccct ggagcacttg 3000
agggaccaga gacccggcat ggtccagacg aaggagcagt ttgagttcgc gctgacagcc 3060
gtggctgagg aggtgaacgc catcctcaag gcccttcccc agtgagcggc agcctcaggg 3120
gcctcagggg agcccccacc ccacggatgt tgtcaggaat catgatctga ctttaattgt 3180
gtgtcttcta ttataactgc atagtaatag ggcccttagc tctcccgtag tcagcgcagt 3240
ttagcagtta aaagtgtatt tttgtttaat caaacaataa taaagagaga tttgtggaaa 3300
aatccagtta cgggtggagg ggaatcggtt catcaatttt cacttgctta aaaaaaatac 3360
tttttcttaa agcacccgtt caccttcttg gttgaagttg tgttaacaat gcagtagcca 3420
gcacgttcga ggcggtttcc aggaagagtg tgcttgtcat ctgccacttt cgggagggtg 3480
gatccactgt gcaggagtgg ccggggaagc tggcagcact cagtgaggcc gcccggcaca 3540
caaggcacgt ttggcatttc tctttgagag agtttatcat tgggagaagc cgcggggaca 3600
gaactgaacg tcctgcagct tcggggcaag tgagacaatc acagctcctc gctgcgtctc 3660
catcaacact gcgccgggta ccatggacgg ccccgtcagc cacacctgtc agcccaagca 3720
gagtgattca ggggctcccc gggggcagac acctgtgcac cccatgagta gtgcccactt 3780
gaggctggca ctcccctgac ctcacctttg caaagttaca gatgcacccc aacattgaga 3840
tgtgttttta atgttaaaat attgatttct acgttatgaa aacagatgcc cccgtgaatg 3900
cttacctgtg agataaccac aaccaggaag aacaaatctg ggcattgagc aagctatgag 3960

ggtccccggg agcacacgaa ccctgccagg cccccgctgg ctcctccagg cacgtcccgg 4020

acctgtgggg ccccagagag gggacatttc cctcctggga gagaaggaga tcagggcaac 4080

tcggagaggg ctgcgagcat ttccctcccg ggagaggaga tcagggcgac ctgcacgcac 4140

tgcgtagagc ctggaaggga agtgagaaac cagccgaccg gccctgcccc tcttcccggg 4200

atcacttaat gaaccacgtg ttttgacatc atgtaaacct aagcacgtag agatgattcg 4260

gatttgacaa aataacattt gagtatccga ttcgccatca ccccctaccc cagaaatagg 4320

acaattcact tcattgacca ggatgatcac atggaaggcg gcgcagaggc agctgtgtgg 4380

gctgcagatt tcctgtgtgg ggttcagcgt agaaaacgca cctccatccc gcccttccca 4440

cagcattcct ccatcttaga tagatggtac tctccaaagg ccctaccaga gggaacacgg 4500

cctactgagc ggacagaatg atgccaaaat attgcttatg tctctacatg gtattgtaat 4560

gaatatctgc tttaatatag ctatcatttc ttttccaaaa ttacttctct ctatctggaa 4620

tttaattaat cgaaatgaat ttatctgaat ataggaagca tatgcctact tgtaatttct 4680

aactccttat gtttgaagag aaacctccgg tgtgagatat acaaatatat ttaattgtgt 4740

catattaaac ttctgattca aaaaaaa 4767

<210> 7
<211> 1015
<212> PRT
<213> Homo sapiens

<400> 7

Met Gly Pro Pro Leu Pro Leu Leu Leu Leu Leu Leu Leu Leu Leu Pro
1               5                   10                  15

Pro Arg Val Leu Pro Ala Ala Pro Ser Ser Val Pro Arg Gly Arg Gln
            20                  25                  30

Leu Pro Gly Arg Leu Gly Cys Leu Leu Glu Glu Gly Leu Cys Gly Ala
        35                  40                  45

Ser Glu Ala Cys Val Asn Asp Gly Val Phe Gly Arg Cys Gln Lys Val
    50                  55                  60

Pro Ala Met Asp Phe Tyr Arg Tyr Glu Val Ser Pro Val Ala Leu Gln
65                  70                  75                  80

Arg Leu Arg Val Ala Leu Gln Lys Leu Ser Gly Thr Gly Phe Thr Trp
                85                  90                  95

Gln Asp Asp Tyr Thr Gln Tyr Val Met Asp Gln Glu Leu Ala Asp Leu
            100                 105                 110

Pro Lys Thr Tyr Leu Arg Arg Pro Glu Ala Ser Ser Pro Ala Arg Pro
        115                 120                 125

Ser Lys His Ser Val Gly Ser Glu Arg Arg Tyr Ser Arg Glu Gly Gly
    130                 135                 140

Ala Ala Leu Ala Asn Ala Leu Arg Arg His Leu Pro Phe Leu Glu Ala
145                 150                 155                 160

Leu Ser Gln Ala Pro Ala Ser Asp Val Leu Ala Arg Thr His Thr Ala
                165                 170                 175

Gln Asp Arg Pro Pro Ala Glu Gly Asp Asp Arg Phe Ser Glu Ser Ile
            180                 185                 190

Leu Thr Tyr Val Ala His Thr Ser Ala Leu Thr Tyr Pro Pro Gly Pro
        195                 200                 205

Arg Thr Gln Leu Arg Glu Asp Leu Leu Pro Arg Thr Leu Gly Gln Leu
    210                 215                 220

Gln Pro Asp Glu Leu Ser Pro Lys Val Asp Ser Gly Val Asp Arg His
225                 230                 235                 240

His Leu Met Ala Ala Leu Ser Ala Tyr Ala Ala Gln Arg Pro Pro Ala
                245                 250                 255

Pro Pro Gly Glu Gly Ser Leu Glu Pro Gln Tyr Leu Leu Arg Ala Pro
            260                 265                 270

Ser Arg Met Pro Arg Pro Leu Leu Ala Pro Ala Ala Pro Gln Lys Trp
        275                 280                 285

Pro Ser Pro Leu Gly Asp Ser Glu Asp Pro Ser Ser Thr Gly Asp Gly
    290                 295                 300

Ala Arg Ile His Thr Leu Leu Lys Asp Leu Gln Arg Gln Pro Ala Glu
305                 310                 315                 320

Val Arg Gly Leu Ser Gly Leu Glu Leu Asp Gly Met Ala Glu Leu Met
                325                 330                 335

Ala Gly Leu Met Gln Gly Val Asp His Gly Val Ala Arg Gly Ser Pro
            340                 345                 350

Gly Arg Ala Ala Leu Gly Glu Ser Gly Glu Gln Ala Asp Gly Pro Lys
        355                 360                 365

Ala Thr Leu Arg Gly Asp Ser Phe Pro Asp Asp Gly Val Gln Asp Asp
    370                 375                 380

Asp Asp Arg Leu Tyr Gln Glu Val His Arg Leu Ser Ala Thr Leu Gly
385                 390                 395                 400

Gly Leu Leu Gln Asp His Gly Ser Arg Leu Leu Pro Gly Ala Leu Pro
                405                 410                 415

Phe Ala Arg Pro Leu Asp Met Glu Arg Lys Lys Ser Glu His Pro Glu
            420                 425                 430

Ser Ser Leu Ser Ser Glu Glu Glu Thr Ala Gly Val Glu Asn Val Lys
        435                 440                 445

Ser Gln Thr Tyr Ser Lys Asp Leu Leu Gly Gln Gln Pro His Ser Glu
    450                 455                 460

Pro Gly Ala Ala Ala Phe Gly Glu Leu Gln Asn Gln Met Pro Gly Pro
465                 470                 475                 480

Ser Lys Glu Glu Gln Ser Leu Pro Ala Gly Ala Gln Glu Ala Leu Ser
                485                 490                 495

Asp Gly Leu Gln Leu Glu Val Gln Pro Ser Glu Glu Glu Ala Arg Gly
            500                 505                 510

Tyr Ile Val Thr Asp Arg Asp Pro Leu Arg Pro Glu Glu Gly Arg Arg
        515                 520                 525

Leu Val Glu Asp Val Ala Arg Leu Leu Gln Val Pro Ser Ser Ala Phe
    530                 535                 540

Ala Asp Val Glu Val Leu Gly Pro Ala Val Thr Phe Lys Val Ser Ala
545                 550                 555                 560

Asn Val Gln Asn Val Thr Thr Glu Asp Val Glu Lys Ala Thr Val Asp
                565                 570                 575

Asn Lys Asp Lys Leu Glu Glu Thr Ser Gly Leu Lys Ile Leu Gln Thr
            580                 585                 590

Gly Val Gly Ser Lys Ser Lys Leu Lys Phe Leu Pro Pro Gln Ala Glu
        595                 600                 605

Gln Glu Asp Ser Thr Lys Phe Ile Ala Leu Thr Leu Val Ser Leu Ala
    610                 615                 620

Cys Ile Leu Gly Val Leu Leu Ala Ser Gly Leu Ile Tyr Cys Leu Arg
625                 630                 635                 640

His Ser Ser Gln His Arg Leu Lys Glu Lys Leu Ser Gly Leu Gly Gly
                645                 650                 655

Asp Pro Gly Ala Asp Ala Thr Ala Ala Tyr Gln Glu Leu Cys Arg Gln
            660                 665                 670

Arg Met Ala Thr Arg Pro Pro Asp Arg Pro Glu Gly Pro His Thr Ser
        675                 680                 685

Arg Ile Ser Ser Val Ser Ser Gln Phe Ser Asp Gly Pro Ile Pro Ser
    690                 695                 700

Pro Ser Ala Arg Ser Ser Ala Ser Ser Trp Ser Glu Glu Pro Val Gln
705                 710                 715                 720

Ser Asn Met Asp Ile Ser Thr Gly His Met Ile Leu Ser Tyr Met Glu
                725                 730                 735

Asp His Leu Lys Asn Lys Asn Arg Leu Glu Lys Glu Trp Glu Ala Leu
            740                 745                 750

Cys Ala Tyr Gln Ala Glu Pro Asn Ser Ser Phe Val Ala Gln Arg Glu
        755                 760                 765

Glu Asn Val Pro Lys Asn Arg Ser Leu Ala Val Leu Thr Tyr Asp His
    770                 775                 780

Ser Arg Val Leu Leu Lys Ala Glu Asn Ser His Ser His Ser Asp Tyr
785                 790                 795                 800

Ile Asn Ala Ser Pro Ile Met Asp His Asp Pro Arg Asn Pro Ala Tyr
                805                 810                 815

Ile Ala Thr Gln Gly Pro Leu Pro Ala Thr Val Ala Asp Phe Trp Gln
            820                 825                 830

Met Val Trp Glu Ser Gly Cys Val Val Ile Val Met Leu Thr Pro Leu
        835                 840                 845

Ala Glu Asn Gly Val Arg Gln Cys Tyr His Tyr Trp Pro Asp Glu Gly
    850                 855                 860

Ser Asn Leu Tyr His Ile Tyr Glu Val Asn Leu Val Ser Glu His Ile
865                 870                 875                 880

Trp Cys Glu Asp Phe Leu Val Arg Ser Phe Tyr Leu Lys Asn Leu Gln
                885                 890                 895

Thr Asn Glu Thr Arg Thr Val Thr Gln Phe His Phe Leu Ser Trp Tyr
            900                 905                 910

Asp Arg Gly Val Pro Ser Ser Ser Arg Ser Leu Leu Asp Phe Arg Arg
        915                 920                 925

Lys Val Asn Lys Cys Tyr Arg Gly Arg Ser Cys Pro Ile Ile Val His
    930                 935                 940

Cys Ser Asp Gly Ala Gly Arg Ser Gly Thr Tyr Val Leu Ile Asp Met
945                 950                 955                 960

Val Leu Asn Lys Met Ala Lys Gly Ala Lys Glu Ile Asp Ile Ala Ala
                965                 970                 975

Thr Leu Glu His Leu Arg Asp Gln Arg Pro Gly Met Val Gln Thr Lys
            980                 985                 990

Glu Gln Phe Glu Phe Ala Leu Thr  Ala Val Ala Glu Glu  Val Asn Ala
        995                 1000                 1005

Ile Leu  Lys Ala Leu Pro Gln
    1010                 1015

<210>  8
<211>  53
<212>  DNA
<213>  artificial sequence

<220>
<221>  source
<223>  /note="Description of artificial sequence:ICA512 shRNA oligo1"

<400>  8
tctcgcgcca tcattcgaaa caattcaaga gattgtttcg aatgatggcg cct          53

<210>  9
<211>  53
<212>  DNA
<213>  artificial sequence

<220>
<221>  source
<223>  /note="Description of artificial sequence:ICA512 shRNA oligo2"

<400>  9
tctcgcagta caagcagatg taattcaaga gattacatct gcttgtactg cct          53

<210>  10

<211>  57
<212>  DNA
<213>  artificial sequence

<220>
<221>  source
<223>  /note="Description of artificial sequence:ß2-syntrophin shRNA oligo1"

<400>  10
tctcgtagct atccacacca acatattcaa gagatatgtt ggtgtggata gctacct        57


<210>  11
<211>  57
<212>  DNA
<213>  artificial sequence

<221>  source
<223>  /note="Description of artificial sequence:ß2-syntrophin shRNA oligo"

<400>  11
ctgcaggtag ctatccacac caacatatct cttgaatatg ttggtgtgga tagctac        57

SEQUENCE LISTING

<110> Technische Universität Dresden

<120> Pharmaceutical composition for increasing insulin secretion from
      pancreatic ß-cells

<130> N1475 EP

<160> 7

<170> PatentIn version 3.3

<210> 1
<211> 979
<212> PRT
<213> Homo sapiens

<400> 1

Met Arg Arg Pro Arg Arg Pro Gly Gly Leu Gly Gly Ser Gly Gly Leu
1               5                   10                  15

Arg Leu Leu Leu Cys Leu Leu Leu Leu Ser Ser Arg Pro Gly Gly Cys
            20                  25                  30

Ser Ala Val Ser Ala His Gly Cys Leu Phe Asp Arg Arg Leu Cys Ser
        35                  40                  45

His Leu Glu Val Cys Ile Gln Asp Gly Leu Phe Gly Gln Cys Gln Val
    50                  55                  60

Gly Val Gly Gln Ala Arg Pro Leu Leu Gln Val Thr Ser Pro Val Leu
65                  70                  75                  80

Gln Arg Leu Gln Gly Val Leu Arg Gln Leu Met Ser Gln Gly Leu Ser
                85                  90                  95

Trp His Asp Asp Leu Thr Gln Tyr Val Ile Ser Gln Glu Met Glu Arg
            100                 105                 110

Ile Pro Arg Leu Arg Pro Pro Glu Pro Arg Pro Arg Asp Arg Ser Gly
        115                 120                 125

Leu Ala Pro Lys Arg Pro Gly Pro Ala Gly Glu Leu Leu Leu Gln Asp
    130                 135                 140

Ile Pro Thr Gly Ser Ala Pro Ala Ala Gln His Arg Leu Pro Gln Pro
145                 150                 155                 160

Pro Val Gly Lys Gly Gly Ala Gly Ala Ser Ser Ser Leu Ser Pro Leu
                165                 170                 175

Gln Ala Glu Leu Leu Pro Pro Leu Leu Glu His Leu Leu Leu Pro Pro
            180                 185                 190

Gln Pro Pro His Pro Ser Leu Ser Tyr Glu Pro Ala Leu Leu Gln Pro
        195                 200                 205

Tyr Leu Phe His Gln Phe Gly Ser Arg Asp Gly Ser Arg Val Ser Glu
    210                 215                 220

Gly Ser Pro Gly Met Val Ser Val Gly Pro Leu Pro Lys Ala Glu Ala
225                 230                 235                 240

Pro Ala Leu Phe Ser Arg Thr Ala Ser Lys Gly Ile Phe Gly Asp His
                245                 250                 255

Pro Gly His Ser Tyr Gly Asp Leu Pro Gly Pro Ser Pro Ala Gln Leu
            260                 265                 270

Phe Gln Asp Ser Gly Leu Leu Tyr Leu Ala Gln Glu Leu Pro Ala Pro
        275                 280                 285

Ser Arg Ala Arg Val Pro Arg Leu Pro Glu Gln Gly Ser Ser Ser Arg
    290                 295                 300

Ala Glu Asp Ser Pro Glu Gly Tyr Glu Lys Glu Gly Leu Gly Asp Arg
305                 310                 315                 320

Gly Glu Lys Pro Ala Ser Pro Ala Val Gln Pro Asp Ala Ala Leu Gln
                325                 330                 335

Arg Leu Ala Ala Val Leu Ala Gly Tyr Gly Val Glu Leu Arg Gln Leu
            340                 345                 350

Thr Pro Glu Gln Leu Ser Thr Leu Leu Thr Leu Leu Gln Leu Leu Pro
        355                 360                 365

Lys Gly Ala Gly Arg Asn Pro Gly Gly Val Val Asn Val Gly Ala Asp
    370                 375                 380

Ile Lys Lys Thr Met Glu Gly Pro Val Glu Gly Arg Asp Thr Ala Glu
385                 390                 395                 400

Leu Pro Ala Arg Thr Ser Pro Met Pro Gly His Pro Thr Ala Ser Pro
                405                 410                 415

Thr Ser Ser Glu Val Gln Gln Val Pro Ser Pro Val Ser Ser Glu Pro
            420                 425                 430

Pro Lys Ala Ala Arg Pro Pro Val Thr Pro Val Leu Leu Glu Lys Lys
        435                 440                 445

Ser Pro Leu Gly Gln Ser Gln Pro Thr Val Ala Gly Gln Pro Ser Ala
    450                 455                 460

Arg Pro Ala Ala Glu Glu Tyr Gly Tyr Ile Val Thr Asp Gln Lys Pro
465                 470                 475                 480

Leu Ser Leu Ala Ala Gly Val Lys Leu Leu Glu Ile Leu Ala Glu His
                485                 490                 495

Val His Met Ser Ser Gly Ser Phe Ile Asn Ile Ser Val Val Gly Pro
            500                 505                 510

Ala Leu Thr Phe Arg Ile Arg His Asn Glu Gln Asn Leu Ser Leu Ala
        515                 520                 525

Asp Val Thr Gln Gln Ala Gly Leu Val Lys Ser Glu Leu Glu Ala Gln
    530                 535                 540

Thr Gly Leu Gln Ile Leu Gln Thr Gly Val Gly Gln Arg Glu Glu Ala
545                 550                 555                 560

Ala Ala Val Leu Pro Gln Thr Ala His Ser Thr Ser Pro Met Arg Ser
                565                 570                 575

Val Leu Leu Thr Leu Val Ala Leu Ala Gly Val Ala Gly Leu Leu Val
            580                 585                 590

Ala Leu Ala Val Ala Leu Cys Val Arg Gln His Ala Arg Gln Gln Asp
        595                 600                 605

Lys Glu Arg Leu Ala Ala Leu Gly Pro Glu Gly Ala His Gly Asp Thr
    610                 615                 620

Thr Phe Glu Tyr Gln Asp Leu Cys Arg Gln His Met Ala Thr Lys Ser
625                 630                 635                 640

Leu Phe Asn Arg Ala Glu Gly Pro Pro Glu Pro Ser Arg Val Ser Ser
                645                 650                 655

Val Ser Ser Gln Phe Ser Asp Ala Ala Gln Ala Ser Pro Ser Ser His
            660                 665                 670

Ser Ser Thr Pro Ser Trp Cys Glu Glu Pro Ala Gln Ala Asn Met Asp
        675                 680                 685

Ile Ser Thr Gly His Met Ile Leu Ala Tyr Met Glu Asp His Leu Arg
    690                 695                 700

Asn Arg Asp Arg Leu Ala Lys Glu Trp Gln Ala Leu Cys Ala Tyr Gln
705                 710                 715                 720

Ala Glu Pro Asn Thr Cys Ala Thr Ala Gln Gly Glu Gly Asn Ile Lys
                725                 730                 735

Lys Asn Arg His Pro Asp Phe Leu Pro Tyr Asp His Ala Arg Ile Lys
            740                 745                 750

Leu Lys Val Glu Ser Ser Pro Ser Arg Ser Asp Tyr Ile Asn Ala Ser
        755                 760                 765

Pro Ile Ile Glu His Asp Pro Arg Met Pro Ala Tyr Ile Ala Thr Gln
    770                 775                 780

Gly Pro Leu Ser His Thr Ile Ala Asp Phe Trp Gln Met Val Trp Glu
785                 790                 795                 800

Ser Gly Cys Thr Val Ile Val Met Leu Thr Pro Leu Val Glu Asp Gly
                805                 810                 815

Val Lys Gln Cys Asp Arg Tyr Trp Pro Asp Glu Gly Ala Ser Leu Tyr
            820                 825                 830

His Val Tyr Glu Val Asn Leu Val Ser Glu His Ile Trp Cys Glu Asp
        835                 840                 845

Phe Leu Val Arg Ser Phe Tyr Leu Lys Asn Val Gln Thr Gln Glu Thr
    850                 855                 860

Arg Thr Leu Thr Gln Phe His Phe Leu Ser Trp Pro Ala Glu Gly Thr
865                 870                 875                 880

Pro Ala Ser Thr Arg Pro Leu Leu Asp Phe Arg Arg Lys Val Asn Lys
                885                 890                 895

Cys Tyr Arg Gly Arg Ser Cys Pro Ile Ile Val His Cys Ser Asp Gly
            900                 905                 910

Ala Gly Arg Thr Gly Thr Tyr Ile Leu Ile Asp Met Val Leu Asn Arg
        915                 920                 925

Met Ala Lys Gly Val Lys Glu Ile Asp Ile Ala Ala Thr Leu Glu His
    930                 935                 940

Val Arg Asp Gln Arg Pro Gly Leu Val Arg Ser Lys Asp Gln Phe Glu
945                 950                 955                 960

Phe Ala Leu Thr Ala Val Ala Glu Glu Val Asn Ala Ile Leu Lys Ala
                965                 970                 975

Leu Pro Gln

<210>  2
<211>  3613
<212>  DNA
<213>  Homo sapiens

<400>  2
cagcccctct ggcaggctcc cgccagcgtc gctgcggctc cggcccggga gcgagcgccc      60

ggagctcgga aagatgcggc gcccgcggcg gcctgggggt ctcgggggat ccgggggtct     120

ccggctgctc ctctgcctcc tgctgctgag cagccgcccg gggggctgca gcgccgttag     180

tgcccacggc tgtctatttg accgcaggct ctgctctcac ctggaagtct gtattcagga     240

tggcttgttt gggcagtgcc aggtgggagt ggggcaggcc cggccccttt tgcaagtcac     300

ctccccagtt ctccaacgct tacaaggtgt gctccgacaa ctcatgtccc aaggattgtc     360

ctggcacgat gacctcaccc agtatgtgat ctctcaggag atggagcgca tccccaggct     420

tcgcccccca gagccccgtc caagggacag gtctggcttg gcacccaaga gacctggtcc     480

tgctggagag ctgcttttac aggacatccc cactggctcc gcccctgctg cccagcatcg     540

gcttccacaa ccaccagtgg gcaaaggtgg agctggggcc agctcctctc tgtcccctct     600

gcaggctgag ctgctcccgc ctctcttgga gcacctgctg ctgcccccac agcctcccca     660

cccttcactg agttacgaac ctgccttgct gcagccctac ctgttccacc agtttggctc     720

ccgtgatggc tccagggtct cagagggctc cccagggatg gtcagtgtcg gccccctgcc     780

caaggctgaa gcccctgccc tcttcagcag aactgcctcc aagggcatat ttggggacca     840

ccctggccac tcctacgggg accttccagg gccttcacct gcccagcttt ttcaagactc     900

tgggctgctc tatctggccc aggagttgcc agcacccagc agggccaggg tgccaaggct     960

gccagagcaa gggagcagca gccgggcaga ggactcccca gagggctatg agaaggaagg    1020

actaggggat cgtggagaga agcctgcttc cccagctgtg cagccagatg cggctctgca    1080

gaggctggcc gctgtgctgg cgggctatgg ggtagagctg cgtcagctga cccctgagca    1140

gctctccaca ctcctgaccc tgctgcagct actgcccaag ggtgcaggaa gaaatccggg    1200

aggggttgta aatgttggag ctgatatcaa gaaaacaatg gaggggccgg tggagggcag    1260

agacacagca gagcttccag cccgcacatc ccccatgcct ggacacccca ctgccagccc    1320

tacctccagt gaagtccagc aggtgccaag ccctgtctcc tctgagcctc ccaaagctgc    1380

cagaccccct gtgacacctg tcctgctaga gaagaaaagc ccactgggcc agagccagcc 1440
cacggtggca ggacagccct cagcccgccc agcagcagag gaatatggct acatcgtcac 1500
tgatcagaag cccctgagcc tggctgcagg agtgaagctg ctggagatcc tggctgagca 1560
tgtgcacatg tcctcaggca gcttcatcaa catcagtgtg gtgggaccag ccctcacctt 1620
ccgcatccgg cacaatgagc agaacctgtc tttggctgat gtgacccaac aagcagggct 1680
ggtgaagtct gaactggaag cacagacagg gctccaaatc ttgcagacag gagtgggaca 1740
gagggaggag gcagctgcag tccttcccca aactgcgcac agcacctcac ccatgcgctc 1800
agtgctgctc actctggtgg ccctggcagg tgtggctggg ctgctggtgg ctctggctgt 1860
ggctctgtgt gtgcggcagc atgcgcggca gcaagacaag gagcgcctgg cagccctggg 1920
gcctgagggg gcccatggtg acactacctt tgagtaccag gacctgtgcc gccagcacat 1980
ggccacgaag tccttgttca accgggcaga gggtccaccg gagccttcac gggtgagcag 2040
tgtgtcctcc cagttcagcg acgcagccca ggccagcccc agctcccaca gcagcacccc 2100
gtcctggtgc gaggagccgg cccaagccaa catggacatc tccacgggac acatgattct 2160
ggcatacatg gaggatcacc tgcggaaccg ggaccgcctt gccaaggagt ggcaggccct 2220
ctgtgcctac caagcagagc caaacacctg tgccaccgcg cagggggagg gcaacatcaa 2280
aaagaaccgg catcctgact tcctgcccta tgaccatgcc cgcataaaac tgaaggtgga 2340
gagcagccct tctcggagcg attacatcaa cgccagcccc attattgagc atgaccctcg 2400
gatgccagcc tacatagcca cgcagggccc gctgtcccat accatcgcag acttctggca 2460
gatggtgtgg gagagcggct gcaccgtcat cgtcatgctg accccgctgg tggaggatgg 2520
tgtcaagcag tgtgaccgct actggccaga tgagggtgcc tccctctacc acgtatatga 2580
ggtgaacctg gtgtcggagc acatctggtg cgaggacttt ctggtgcgga gcttctacct 2640
gaagaacgtg cagacccagg agacgcgcac gctcacgcag ttccacttcc tcagctggcc 2700
ggcagagggc acaccggcct ccacgcggcc cctgctggac ttccgcagga aggtgaacaa 2760
gtgctaccgg ggccgctcct gccccatcat cgtgcactgc agtgatggtg cggggaggac 2820
cggcacctac atcctcatcg acatggtcct gaaccgcatg gcaaaaggag tgaaggagat 2880
tgacatcgct gccaccctgg agcatgtccg tgaccagcgg cctggccttg tccgctctaa 2940
ggaccagttt gaatttgccc tgacagccgt ggcggaggaa gtgaatgcca tcctcaaggc 3000
cctgccccag tgagaccctg gggccccttg gcgggcagcc cagcctctgt ccctctttgc 3060
ctgtgtgagc atctctgtgt acccactcct cactgcccca ccagccacct cttgggcatg 3120
ctcagccctt cctagaagag tcaggaaggg aaagccagaa ggggcacgcc tgcccagcct 3180
cgcatgccag agcctggggc atcccagagc ccagggcatc ccatgggggt gctgcagcca 3240
ggaggagagg aaaggacatg ggtagcaatt ctacccagag ccttctcctg cctacattcc 3300

ctggcctggc tctcctgtag ctctcctggg gttctgggag ttccctgaac atctgtgtgt 3360

gtccccctat gctccagtat ggaagaatgg ggtggagggt cgccacaccc ggctccccct 3420

gcttctcagc cccgggcctg cctctgactc acacttgggc gctctgccct ccctggcctc 3480

acgcccagcc tggtcccacc accctcccac catgcgctgc tcaacctctc tccttctggc 3540

gcaagagaac atttctagaa aaaactactt ttgtaccagt gtgaataaag ttagtgtgtt 3600

gtctgtgcag ctg 3613

<210> 3
<211> 9771
<212> DNA
<213> Homo sapiens

<400> 3
gccgaggctg cctgactgga atgagggtag ctgcggcgac tgcggcggct ggagcggggc 60

cggccatggc ggtgtggacg cgggccacca aagcggggct ggtggagctg ctcctgaggg 120

agcgctgggt ccgagtggtg gccgagctga gcggggagag cctgagcctg acgggcgacg 180

ccgccgcggc cgagctggag cccgctctgg gacccgcggc cgccgccttc aacggcctcc 240

caaacggcgg cggcgcgggc gactcgctgc ccgggagccc aagccgcggc ctggggcccc 300

cgagcccgcc ggcgccgcct cggggccccg cgggtgaggc gggcgcgtcg ccgcccgtgc 360

gccgggtgcg ggtggtgaag caagaggcgg gcggcctggg catcagcatc aagggcggcc 420

gcgagaaccg gatgccgatc ctcatctcca agatcttccc cgggctggct gccgaccaga 480

gccgggcgct gcggctgggc gacgccatcc tgtcggtgaa cggcaccgac ctgcgccagg 540

ccacccacga ccaggccgtg caggcgctga agcgcgcggg caaggaggtg ctgctggagg 600

tcaagttcat ccgagaagta acaccatata tcaagaagcc atcattagta tcagatctgc 660

cgtgggaagg tgcagccccc cagtcaccaa gctttagtgg cagtgaggac tctggttcgc 720

caaaacacca gaacagcacc aaggacagga agatcatccc tctcaaaatg tgctttgctg 780

ctagaaacct aagcatgccg gatctggaaa acagattgat agagctacat tctcctgata 840

gcaggaacac gttgatccta cgctgcaaag atacagccac agcacactcc tggttcgtag 900

ctatccacac caacataatg gctctcctcc cacaggtgtt ggctgaactc aacgccatgc 960

ttggggcaac cagtacagca ggaggcagta aagaggtgaa gcatattgcc tggctggcag 1020

aacaggcaaa actagatggt ggaagacagc aatggagacc tgtcctcatg gctgtgactg 1080

agaaggattt gctgctctat gactgtatgc cgtggacaag agatgcctgg gcgtcaccat 1140

gccacagcta cccacttgtt gccaccaggt tggttcattc tggctccgga tgtcgatccc 1200

cctcccttgg atctgacctt acatttgcta ccaggacagg ctctcgacag ggcattgaga 1260

tgcatctctt cagggtggag acacatcggg atctgtcatc ctggaccagg atacttgttc 1320

agggttgcca tgctgctgct gagctgatca aggaagtctc tctaggctgc atgttaaatg 1380

gccaagaggt gaggcttact attcactatg aaaatgggtt caccatctca agggaaaatg 1440

gaggctccag cagcatattg taccgctacc cctttgaaag gctgaagatg tctgctgatg 1500

atggcatccg aaatctatac ttggattttg gtggtcccga gggagaactg accatggacc 1560

tgcactcttg tccgaagccg attgtatttg tgttgcacac gtttttatcg gccaaagtca 1620

ctcgtatggg actgcttgta tgagcaacaa aaaatcagaa aagagccttg actgtcacaa 1680

gaaatatttc cacctcaaaa aaaaaaaagc acaaaaagaa actctttgct ctccttcagc 1740

acagtgcctt cccaaggacc tgcaaataac tgctgaacca taaccgtgtt taaagaagag 1800

cctacctttc acagtctacc ttggccagat attctagcac tctaaaaggc tccaaaatga 1860

agctgttgat ttattctcat ttcaaaatat ggtttatgag taattaggtt tatttctact 1920

gctaaaaaga atggctcatt gttttctttt tttttcattg ttttcatttt tagacataat 1980

caaataccca agcattgttt tctttttctt ctccctgtag gatttgtgtt gtgcttagcc 2040

ttgatctctt gttcagaaca attgtccctt cccaggtgct agcttgcagc agcctgttgc 2100

ttcctgagca ttccagatag gtcgtatgtc ttggaattta ctgtcctcac tagctccttc 2160

caccatagga agtccttgaa ttgtatcacc aaagggttgt tccatttgcc ctggatctcc 2220

ctctatctca gcctggccat cacttcccac cttatcccaa gctttgccac tgtctctagg 2280

acttagaact tgtaaatttg atttgccttg ccttctactt cctttctagt ggtgactgag 2340

ttgaaaagca agttggaagc cactgctttt aaagaaaagt tttgcttggt ttaggttttt 2400

ccaattttat ttttgttagg agccagggga taagatttat tttaaatgaa aatcctcttc 2460

ctaggttaag ccattttttt tattgcttac caaatgtgcc tttggttagg gttagtggag 2520

ctttattagt cactatttga ataactggaa atcactgcca tttccaggag atcacctatc 2580

ctagttttga ggaatagtca ttgaggtggg tttccttaat ggcatctctt ctagtaggca 2640

gatctgtttg aacaagataa atcctaaaga gccagcctgc ttgaggagta gacttggtgg 2700

gtgaagccag caattccgca caaacgtcat gttgaattgt tttgggctgc ccaaaaacaa 2760

caggatgcag catcctggct gtggcctggc cctttgtggc agcagtggca tggacaagca 2820

actgctaatt cgagacttac tattggcttc acagcacacc ctacagtgag cagggtgatg 2880

agaccgtgga acagctacct gcttttggtt aagattgcca gatatcatta ggacacagta 2940

gcagcaagaa tgctgatttt gtagtgttag aaaatgaact cagcttgttt ttcctaattt 3000

tgaaggtggt atatatctgc aaacatttaa aatggtataa tatcatataa aatgtcaaaa 3060

ttagcacagt ccctggatct caaagaatag gtaatattga cttggatgtt ctttatgctc 3120

tctgaagaaa ggtctaggga aagttcttgt ttgcttgatt gatcaagagt gccacggaag 3180

aatgtgagcg ctgtggaggg tggaggaggg tgtcacttca gatggggcag gcacaggggg 3240

agaaaagaag agaaaaacac acctgtgtgc aaaatgagct atttggtttc agtgcggcct 3300

gaccgatggg gaggacccat gagcagagag agttctaggt caacattgta actccattgt 3360
cctccttcac ccataagtca gtcctccttc ctcattacag tgaaaccaac aatatgcaga 3420
ggttggggag agatgcctgt tcttccccca gcttcatata attgtcaaag ttccaaaaat 3480
gaattatctg aactgcttaa aactggaaat taaaatctag acaagaatat gtctttcaag 3540
agctttttat gttttgaaag ttcataaacc taagattggc tccatctgag ataaattttc 3600
ttttcttttt ttttttgaga cggagtctcg ttctgtctcc caggctggag tgcagtggca 3660
tgatctcggc tcactgcaac ctccacctcc taggttcaag tgattctcct gagtagctgg 3720
ggttatagct acccaccacc acacccagct aatttttgta tttttagtag aggcaggttt 3780
tcaccatgtt ggctaggctg gtcttgaact cccgacctca agtgatccac ctgccttggc 3840
ctcccaaagt gctgggatta taggtgtgag cccccgtgcc tggcccacaa taaattttca 3900
tttcagagaa ggaaaaaaat ctcttgaacg tccagaaatg atttctggaa tcagaatttc 3960
aatatgtaat accctcaggt aggcaatctt gaaccctcta tgcagtgaga gcattattaa 4020
tatgagatgt tgaatgatgc ctttacagac ttcagcagca aagccatttg gttgaggttt 4080
tctgtgttaa tgtgaaaagc taagccactg agatttgcag gggagcttct gagccaccct 4140
gcttgttggt tatatgagga tgtaaaatag gtggaattaa aatgtgagca ggaaaggcaa 4200
ggcatgtgtg cattttcact cattttggtc aaggttaaac tgacatgtat agcactgagg 4260
tctgtagggt tcttaatatc ttttgagtct tgctgtgaaa tgaaagtgtg ggaggtttcc 4320
tttgtccatt agcagcccca agaaccagaa cccttttgct gcttttctta catacctaac 4380
agctctccag tcatgatgac caaggttgtt cttcaatcaa atgtgtttgt gggattttca 4440
gtccgcaaat gaagtgctct ctaatgaatg ggacaccatg ataaatatgt atttatattt 4500
agatgccaaa gtatggcaaa ttatttccaa atgataacta caaatgggaa ttttcgatat 4560
tctacctttt ttatagaacc agctcacttt tcatttcttt ttcattttga attaagaaaa 4620
ttgttgagga tgtggtgggt tccagtgtgt ggaatggaaa ggaaactgca gaatagtgtc 4680
tgctccccat tcagagggac tgcttcctgt gccccccaga cccggggctt cgacagcttc 4740
tccacattcc acacagatgc ctaggagcag cgagttggta tatgaaaagt ctcccacctt 4800
ttctcctaaa acttctctcc tttctctcca taaaaagaaa aggaaaggaa caaaagaaaa 4860
acattcagtt tttctttttc tgaaaaaggt aagtcctttc ctgaagtcat caaatgaaac 4920
attatctgga aattagtttc taatgttgta tatgaagaaa tacttaaata taagttcctg 4980
cagtatttat tagatagttg taactgtaaa ctcacctccc tagtagataa gagtttcagg 5040
ttaaatactg gaacatatat aggcagtcaa aaatactact ttaaatgtca ttcacctatt 5100
ttaaagccat gttttagcac tttttaggcc aaagaaggtc tgatagtgcc tgtttttatg 5160
ttctgtactc tcacaaactt tgttactcaa aattattgca tggcaggaga gattggatta 5220

tttatttctt atatttttat aagtaaaaaa atctttctaa acaacaaata cctaacatta 5280

ttactgattg ttttcctaat ttatcctcct aagttgaatg gtaacaaagc ttttccagct 5340

gaatgaatgc acttagctga taaaccagaa tttgttcttt tttttccttc tttttttttt 5400

gagacaggtt ctcactctgt caccgaggtt ggagtgcagt ggtatgatca tagctgactg 5460

cagcctcaac cttctgggct caaatgatcc tttcacctca gcctcctgag tagctgggac 5520

cacaggtgtg tgccaccaca cctggctaat ttttatggat ttttttttct tttttttttt 5580

tcgccatgtt gcccaggctg gtcttgaact cttggcactc aagcagtcct tctgcctcag 5640

cctcccaaag tgctgggatt acagatgtga gccactgcac ctggccccag aaattatgct 5700

taaaaaaaaa attaagctgt gtggaatctg gacaaggcta tgtttctcct agaaacaggt 5760

tttgctgtaa atggttttga tgggcgaaag tgtgagaaaa ggttataata gattcttttt 5820

tttaattttg tgagaaattc caggtcttct tgcttaactt atatgcatgt ggatatattt 5880

gttttcaaag ccatggaaga atctctgttc atatatttta atgcaaatta ccttgtatgc 5940

tgctatgcaa attctattaa aaggcctacc tgcttaagaa ttaaacattt ttgaaacttt 6000

cagggaagac ctgtagactc aagcactttc tctgcttttt tgtatcttat cttggacact 6060

tatgctgaat tatgttctta ttattttcac tggttataag ctattccttg tacataatat 6120

ttaaactatc caaatatcca gcatacattt ccatgtggct gccatcggct gttgatgcca 6180

tgaatgaaat ggctggttag aaagccaaag gtcttctttt ttcaattcct aatgaataag 6240

taaaatgcca gatctctttg gtctcaaagc aaattgctct gatcagatca aatatctatg 6300

taatgaaaag agacttactt aaatttggga ttctgcttaa actttaagtt gaatttgact 6360

gtagtcattc attcttttta tatatctctg tcaggtatga cattaattac caggtggtat 6420

tatactctac tttgagtttg gacatcactt tcagttaaat ttctgttgtt attgtggctt 6480

tttttttttt ttaaccccac tgaagatgga agtagagcag tctgccatct cctgctgttc 6540

tttctctgtg ccccctaatt gcttggtaat ctaaaattat tctgactttc cacatactgt 6600

ctttaaaaac caattgtagc aatctgcccg cctccaccct ttaagctaaa tctttcactg 6660

tgctggatac atagacactc gataaatgtg tgatgatagt tctaaaataa ttatgctata 6720

tatcagggtt ctgtattttt gagcaatatc attattccta tgctgtgatg ttaaatggaa 6780

tttattttac actagtaata gactgcttaa gtggctgatt tgaaaccttt ctctaaaaaa 6840

acaaaaacat ctggcagatg agtgacacaa attcattctc ttaaaggata gaatttcaag 6900

ttaaggataa ggcacaataa ataggcacat agaggacatc tgtttttgtt tttggttttg 6960

gttttgtttt tttttgtctc tctgactagc taaactgcca actagaagaa tggttgttat 7020

gtagtaactc agaagaatct tttaataagt ttcttctttt gtttcaaaac aaacttgagc 7080

ttcatttctt gccacctcta ttctttcttc ccacgttggg tgactgaact gatgcttatt 7140

ctagttttag aagaggatcc acatttaatg taaggcataa atatgccttt tgcaaattat 7200
tgcttgttct ccagtgaaca tttgatcata ttcaacagaa atccaccata taaaccataa 7260
tccttgaatt gcccctttta aaatagattg gttaaaacca aaaacctata agagagaaat 7320
gatatttcat tgtattgtag gtttgggaat cctggttaga tttataattt ctggccgggt 7380
gcggtggctc atgcctgtat tcccagtact ttgggagccc aaggtgggtg gatcacctga 7440
ggtaaggagt ttgagaccag cctggccaac atggtgaaac cccatctcta ctaaaaatac 7500
aaaaagttag ctgggcatgg tggtgcgcgc ctgtggtccc agctactcgg gaggctgagg 7560
tgggagaatc ccttgaaccc aggaggcaga agttgcagtg agctgagatc atgccattgc 7620
actccagcct gggcaacaga gcgagacttc atctccaaaa aaagaaaaag aagaaaaaga 7680
tttataattt ctgagtcccc taagtccctt tctcagtttg caataatctt acgtccatgc 7740
tattgacact aattttgaaa ctggaaagct aattaatgac atattcatgt gttctttcat 7800
ctaataaaca tttattaatg tatagttcta caccaggcat tctgaagggt ccaaaagaaa 7860
gttcctgccc tctagtagca cataatccgt tatttctcag cacaccatgc catctgttta 7920
tcattcagca gttaagtaat tcatcacttc atatttttac caaagtatat ttacaagtct 7980
gtttatctgg atctggcaaa ctggaaaatg tttgaaggga tactcaggct ttgatccatg 8040
ccaacatgaa aggactccaa gcacatttaa gtaggctggt cttttcatca tgcatgattt 8100
tttttttagt gcaaatttag actttagttt gggaaagaga acagtataca gtatcctttg 8160
taagataaat cacaacaaca aaaattaagt tttattgcac aaagtagata aatgagaaag 8220
tctttcttaa aaaatgcttc tttaactgta acagcaaaac taaatgcata ttttatcaac 8280
cccttatttc taattgctgt attcatttcc acccttaaac tatttaagca tttcatctgc 8340
tgaaagtcct tatatacatg gtagacggaa tatttctcca ttaatgatag atcaaaatga 8400
agatatttaa aaattaacaa aatcaggcca aggcaggagg atcactttag cccaggaggt 8460
ccagaccagc ctgggcaaca cagcgggacc ccgactccat ttaaaaaatt aaaaaaaaaa 8520
aaaaaacttt taacatttaa aaaataaaaa ttaacaaaat ttcacttatt ccaggacacg 8580
ctggcatttg gactcaatga aaagggcacc taaagaaaat aaggctgact gaatgttttc 8640
cataattttc acacaataac agtccctttc tatccagctt gccttccatt tatctctagg 8700
gttagctttt caggcaacat ccttggtcat tgcccagaaa gtacctgagc tatcagtgat 8760
tggaatggca caggaaaccg aatcacatgg gtgccctccc cttggttttc aagtatcttg 8820
gagttgtgca caaaaattag gtcatgcctt cagtgtcttg ttctttaaac ctaccctttg 8880
acaatcaggt gctaatgatt gtatactatt aaaaccagca cataagtatt gtaaatgtgt 8940
gttcctccta ggttggaaga aatgtctttc cttctatctg ggtcctgtta aagcgggtgt 9000
cagttgtgtc ttttcacctc gatttgtgaa ttaatagaat tggggggaga ggaaatgatg 9060

atgtcaatta agtttcaggt ttggcatgat catcattctc gatgatattc tcactttgtc 9120

gcaaatctgc ccttatcgta agaacaagtt tcagaatttt ccctccacta tacgactcca 9180

gtattatgtt tacaatccat tggatgagtg cagcattata agaccttggt gcccagaaaa 9240

atctgtcctt tttggtacca aacctgaggt cttttggaag ataatgtaga aaaccactac 9300

ctattgaagg cctgttttgg ctaatctgtg caaactctga tgatacctgc ttatgtggat 9360

tcttttccac actgctttca tttttaagta taaagactta gaaaactaga ataatgcttt 9420

tacaaataat taaaagtatg tgatgttctg ggttttttcc ttctttttag aaccctgtat 9480

ttaaacaagc cttcttttta agtcttgttt gaaatttaag tctcagatct tctggatacc 9540

aaatcaaaaa cccaacgcgt aaaacagggc agtatttgtg ttcctaattt taaaaagctt 9600

tatgtatact ctataaatat agatgcataa acaacacttc cccttgagta gcacatcaac 9660

atacagcatt gtacattaca atgaaaatgt gtaacttaag ggtattatat atataaatac 9720

atatatacct ttgtaacctt tatactgtaa ataaaaaagt tgctttagtc a 9771

<210> 4
<211> 540
<212> PRT
<213> Homo sapiens

<400> 4

Met Arg Val Ala Ala Ala Thr Ala Ala Ala Gly Ala Gly Pro Ala Met
1               5                   10                  15

Ala Val Trp Thr Arg Ala Thr Lys Ala Gly Leu Val Glu Leu Leu Leu
            20                  25                  30

Arg Glu Arg Trp Val Arg Val Val Ala Glu Leu Ser Gly Glu Ser Leu
        35                  40                  45

Ser Leu Thr Gly Asp Ala Ala Ala Ala Glu Leu Glu Pro Ala Leu Gly
    50                  55                  60

Pro Ala Ala Ala Ala Phe Asn Gly Leu Pro Asn Gly Gly Gly Ala Gly
65                  70                  75                  80

Asp Ser Leu Pro Gly Ser Pro Ser Arg Gly Leu Gly Pro Pro Ser Pro
                85                  90                  95

Pro Ala Pro Pro Arg Gly Pro Ala Gly Glu Ala Gly Ala Ser Pro Pro
            100                 105                 110

Val Arg Arg Val Arg Val Val Lys Gln Glu Ala Gly Gly Leu Gly Ile
        115                 120                 125

Ser Ile Lys Gly Gly Arg Glu Asn Arg Met Pro Ile Leu Ile Ser Lys
    130                 135                 140

Ile Phe Pro Gly Leu Ala Ala Asp Gln Ser Arg Ala Leu Arg Leu Gly
145                 150                 155                 160

Asp Ala Ile Leu Ser Val Asn Gly Thr Asp Leu Arg Gln Ala Thr His
                165                 170                 175

Asp Gln Ala Val Gln Ala Leu Lys Arg Ala Gly Lys Glu Val Leu Leu
            180                 185                 190

Glu Val Lys Phe Ile Arg Glu Val Thr Pro Tyr Ile Lys Lys Pro Ser
        195                 200                 205

Leu Val Ser Asp Leu Pro Trp Glu Gly Ala Ala Pro Gln Ser Pro Ser
    210                 215                 220

Phe Ser Gly Ser Glu Asp Ser Gly Ser Pro Lys His Gln Asn Ser Thr
225                 230                 235                 240

Lys Asp Arg Lys Ile Ile Pro Leu Lys Met Cys Phe Ala Ala Arg Asn
                245                 250                 255

Leu Ser Met Pro Asp Leu Glu Asn Arg Leu Ile Glu Leu His Ser Pro
            260                 265                 270

Asp Ser Arg Asn Thr Leu Ile Leu Arg Cys Lys Asp Thr Ala Thr Ala
        275                 280                 285

His Ser Trp Phe Val Ala Ile His Thr Asn Ile Met Ala Leu Leu Pro
    290                 295                 300

Gln Val Leu Ala Glu Leu Asn Ala Met Leu Gly Ala Thr Ser Thr Ala
305                 310                 315                 320

Gly Gly Ser Lys Glu Val Lys His Ile Ala Trp Leu Ala Glu Gln Ala
                325                 330                 335

Lys Leu Asp Gly Gly Arg Gln Gln Trp Arg Pro Val Leu Met Ala Val
            340                 345                 350

Thr Glu Lys Asp Leu Leu Leu Tyr Asp Cys Met Pro Trp Thr Arg Asp
        355                 360                 365

Ala Trp Ala Ser Pro Cys His Ser Tyr Pro Leu Val Ala Thr Arg Leu
    370                 375                 380

Val His Ser Gly Ser Gly Cys Arg Ser Pro Ser Leu Gly Ser Asp Leu
385                 390                 395                 400

Thr Phe Ala Thr Arg Thr Gly Ser Arg Gln Gly Ile Glu Met His Leu
                405                 410                 415

Phe Arg Val Glu Thr His Arg Asp Leu Ser Ser Trp Thr Arg Ile Leu
            420                 425                 430

Val Gln Gly Cys His Ala Ala Ala Glu Leu Ile Lys Glu Val Ser Leu
        435                 440                 445

Gly Cys Met Leu Asn Gly Gln Glu Val Arg Leu Thr Ile His Tyr Glu
    450                 455                 460

Asn Gly Phe Thr Ile Ser Arg Glu Asn Gly Gly Ser Ser Ser Ile Leu
465                 470                 475                 480

Tyr Arg Tyr Pro Phe Glu Arg Leu Lys Met Ser Ala Asp Asp Gly Ile
                485                 490                 495

Arg Asn Leu Tyr Leu Asp Phe Gly Gly Pro Glu Gly Glu Leu Thr Met
            500                 505                 510

Asp Leu His Ser Cys Pro Lys Pro Ile Val Phe Val Leu His Thr Phe
        515                 520                 525

Leu Ser Ala Lys Val Thr Arg Met Gly Leu Leu Val
    530                 535                 540

<210>  5
<211>  520
<212>  PRT
<213>  Mus musculus

<400>  5

Met Ala Val Trp Thr Arg Ala Thr Lys Ala Gly Leu Val Glu Leu Leu
1               5                   10                  15

Leu Arg Glu Arg Trp Val Arg Val Val Ala Glu Leu Ser Gly Glu Ser
            20                  25                  30

Leu Ser Leu Thr Gly Asp Ala Ala Ala Val Glu Pro Glu Pro Pro Ala
        35                  40                  45

Ala Ala Phe Asn Gly Leu Pro Asn Gly Gly Gly Gly Glu Ser Leu Pro
    50                  55                  60

Gly Ser Pro Asn Arg Gly Leu Gly Pro Pro Ser Pro Pro Ala Pro Pro
65                  70                  75                  80

Arg Gly Pro Ala Gly Glu Ala Ser Ala Ser Pro Pro Val Arg Arg Val
                85                  90                  95

Arg Val Val Lys Gln Glu Ala Gly Gly Leu Gly Ile Ser Ile Lys Gly
            100                 105                 110

Gly Arg Glu Asn Arg Met Pro Ile Leu Ile Ser Lys Ile Phe Pro Gly
        115                 120                 125

Leu Ala Ala Asp Gln Ser Arg Ala Leu Arg Leu Gly Asp Ala Ile Leu
    130                 135                 140

Ser Val Asn Gly Thr Asp Leu Arg Gln Ala Thr His Asp Gln Ala Val
145                 150                 155                 160

Gln Ala Leu Lys Arg Ala Gly Lys Glu Val Leu Leu Glu Val Lys Phe
                165                 170                 175

Ile Arg Glu Val Thr Pro Tyr Ile Lys Lys Pro Ser Leu Val Ser Asp
            180                 185                 190

Leu Pro Trp Glu Gly Ala Ser Pro Gln Ser Pro Ser Phe Ser Gly Ser
        195                 200                 205

Glu Asp Ser Gly Ser Pro Lys His Gln Asn Thr Thr Lys Asp Arg Lys
    210                 215                 220

Val Ile Pro Leu Lys Met Cys Phe Ala Ala Arg Asn Leu Ser Met Pro
225                 230                 235                 240

Asp Leu Glu Asn Arg Leu Ile Glu Leu His Ser Pro Asp Ser Arg Asn
                245                 250                 255

Thr Leu Ile Leu Arg Cys Lys Asp Thr Ala Thr Ala His Ser Trp Phe
            260                 265                 270

Val Ala Ile His Thr Asn Ile Met Ala Leu Leu Pro Gln Val Leu Ala
        275                 280                 285

Glu Leu Asn Ala Met Leu Gly Ala Thr Ser Thr Ala Gly Gly Ser Lys
    290                 295                 300

Glu Val Lys His Ile Ala Trp Leu Ala Glu Gln Ala Lys Leu Asp Gly
305                 310                 315                 320

Gly Arg Gln Gln Trp Arg Pro Val Leu Met Ala Val Thr Glu Lys Asp
                325                 330                 335

Leu Leu Leu Tyr Asp Cys Met Pro Trp Thr Arg Asp Ala Trp Ala Ser
            340                 345                 350

Pro Cys His Ser Tyr Pro Leu Val Ala Thr Arg Leu Val His Ser Gly
        355                 360                 365

Ser Gly Cys Arg Ser Pro Ser Leu Gly Ser Asp Leu Thr Phe Ala Thr
    370                 375                 380

Arg Thr Gly Ser Arg Gln Gly Ile Glu Met His Leu Phe Arg Val Glu
385                 390                 395                 400

Thr His Arg Asp Leu Ser Thr Trp Thr Arg Ile Leu Val Gln Gly Cys
                405                 410                 415

His Ala Ala Ala Glu Leu Ile Lys Glu Val Ser Leu Gly Cys Thr Leu
            420                 425                 430

Ser Gly Gln Glu Val Arg Phe Thr Val His Tyr Glu His Gly Phe Thr
        435                 440                 445

Ile Thr Arg Asp Asn Gly Gly Ala Ser Ser Ile Leu Tyr Arg Tyr Pro
    450                 455                 460

Phe Glu Arg Leu Lys Met Ser Ala Asp Asp Gly Ile Arg Asn Leu Tyr
465                 470                 475                 480

Leu Asp Phe Gly Gly Pro Glu Gly Glu Leu Thr Met Asp Leu His Ser
                485                 490                 495

Cys Pro Lys Pro Ile Val Phe Val Leu His Thr Phe Leu Ser Ala Lys
            500                 505                 510

Val Thr Arg Met Gly Leu Leu Val
        515                 520

<210> 6
<211> 4767
<212> DNA
<213> Homo sapiens

<400> 6
gcctcccgcc gcctcccgcg cggccatgga ctgagcgccg ccggccaggc cgcggggatg 60

gggccgccgc tcccgctgct gctgctgcta ctgctgctgc tgccgccacg cgtcctgcct 120

gccgcccctt cgtccgtccc ccgcggccgg cagctcccgg ggcgtctggg ctgcctgctc 180

gaggagggcc tctgcggagc gtccgaggcc tgtgtgaacg atggagtgtt tggaaggtgc 240
cagaaggttc cggcaatgga cttttaccgc tacgaggtgt cgcccgtggc cctgcagcgc 300
ctgcgcgtgg cgttgcagaa gctttccggc acaggtttca cgtggcagga tgactatact 360
cagtatgtga tggaccagga acttgcagac ctcccgaaaa cctacctgag gcgtcctgaa 420
gcatccagcc cagccaggcc ctcaaaacac agcgttggca gcgagaggag gtacagtcgg 480
gagggcggtg ctgccctggc caacgccctc cgacgccacc tgcccttcct ggaggccctg 540
tcccaggccc cagcctcaga cgtgctcgcc aggacccata cggcgcagga cagacccccc 600
gctgagggtg atgaccgctt ctccgagagc atcctgacct atgtggccca cacgtctgcg 660
ctgacctacc ctcccgggcc ccggacccag ctccgcgagg acctcctgcc gcggaccctc 720
ggccagctcc agccagatga gctcagccct aaggtggaca gtggtgtgga cagacaccat 780
ctgatggcgg ccctcagtgc ctatgctgcc cagaggcccc cagctccccc cggggagggc 840
agcctggagc cacagtacct tctgcgtgca ccctcaagaa tgcccaggcc tttgctggca 900
ccagccgccc cccagaagtg gccttcacct ctgggagatt ccgaagaccc ctccagcaca 960
ggcgatggag cacggattca taccctcctg aaggacctgc agaggcagcc ggctgaggtg 1020
aggggcctga gtggcctgga gctggacggc atggctgagc tgatggctgg cctgatgcaa 1080
ggcgtggacc atggagtagc tcgaggcagc cctgggagag cggccctggg agagtctgga 1140
gaacaggcgg atggccccaa ggccaccctc cgtggagaca gctttccaga tgacggagtg 1200
caggacgacg atgatagact ttaccaagag gtccatcgtc tgagtgccac actcgggggc 1260
ctcctgcagg accacgggtc tcgactctta cctggagccc tcccctttgc aaggcccctc 1320
gacatggaga ggaagaagtc cgagcaccct gagtcttccc tgtcttcaga agaggagact 1380
gccggagtgg agaacgtcaa gagccagacg tattccaaag atctgctggg gcagcagccg 1440
cattcggagc ccggggccgc tgcgtttggg gagctccaaa accagatgcc tgggccctcg 1500
aaggaggagc agagccttcc agcgggtgct caggaggccc tcagcgacgg cctgcaattg 1560
gaggtccagc cttccgagga agaggcgcgg ggctacatcg tgacagacag agaccccctg 1620
cgccccgagg aaggaaggcg gctggtggag gacgtcgccc gcctcctgca ggtgcccagc 1680
agtgcgttcg ctgacgtgga ggttctcgga ccagcagtga ccttcaaagt gagcgccaat 1740
gtccaaaacg tgaccactga ggatgtggag aaggccacag ttgacaacaa agacaaactg 1800
gaggaaacct ctggactgaa aattcttcaa accggagtcg ggtcgaaaag caaactcaag 1860
ttcctgcctc ctcaggcgga gcaagaagac tccaccaagt tcatcgcgct caccctggtc 1920
tccctcgcct gcatcctggg cgtcctcctg gcctctggcc tcatctactg cctccgccat 1980
agctctcagc acaggctgaa ggagaagctc tcgggactag ggggcgaccc aggtgcagat 2040
gccactgccg cctaccagga gctgtgccgc cagcgtatgg ccacgcggcc accagaccga 2100

cctgagggcc cgcacacgtc acgcatcagc agcgtctcat cccagttcag cgacgggccg 2160

atccccagcc cctccgcacg cagcagcgcc tcatcctggt ccgaggagcc tgtgcagtcc 2220

aacatggaca tctccaccgg ccacatgatc ctgtcctaca tggaggacca cctgaagaac 2280

aagaaccggc tggagaagga gtgggaagcg ctgtgcgcct accaggcgga gcccaacagc 2340

tcgttcgtgg cccagaggga ggagaacgtg cccaagaacc gctccctggc tgtgctgacc 2400

tatgaccact cccgggtcct gctgaaggcg gagaacagcc acagccactc agactacatc 2460

aacgctagcc ccatcatgga tcacgacccg aggaaccccg cgtacatcgc cacccaggga 2520

ccgctgcccg ccaccgtggc tgacttttgg cagatggtgt gggagagcgg ctgcgtggtg 2580

atcgtcatgc tgacacccct cgcggagaac ggcgtccggc agtgctacca ctactggccg 2640

gatgaaggct ccaatctcta ccacatctat gaggtgaacc tggtctccga gcacatctgg 2700

tgtgaggact tcctggtgag gagcttctat ctgaagaacc tgcagaccaa cgagacgcgc 2760

accgtgacgc agttccactt cctgagttgg tatgaccgag gagtcccttc ctcctcaagg 2820

tccctcctgg acttccgcag aaaagtaaac aagtgctaca ggggccgttc ttgtccaata 2880

attgttcatt gcagtgacgg tgcaggccgg agcggcacct acgtcctgat cgacatggtt 2940

ctcaacaaga tggccaaagg tgctaaagag attgatatcg cagcgaccct ggagcacttg 3000

agggaccaga gacccggcat ggtccagacg aaggagcagt ttgagttcgc gctgacagcc 3060

gtggctgagg aggtgaacgc catcctcaag gcccttcccc agtgagcggc agcctcaggg 3120

gcctcagggg agcccccacc ccacggatgt tgtcaggaat catgatctga ctttaattgt 3180

gtgtcttcta ttataactgc atagtaatag ggcccttagc tctcccgtag tcagcgcagt 3240

ttagcagtta aaagtgtatt tttgtttaat caaacaataa taaagagaga tttgtggaaa 3300

aatccagtta cgggtggagg ggaatcggtt catcaatttt cacttgctta aaaaaaatac 3360

tttttcttaa agcacccgtt caccttcttg gttgaagttg tgttaacaat gcagtagcca 3420

gcacgttcga ggcggtttcc aggaagagtg tgcttgtcat ctgccacttt cgggagggtg 3480

gatccactgt gcaggagtgg ccggggaagc tggcagcact cagtgaggcc gcccggcaca 3540

caaggcacgt ttggcatttc tctttgagag agtttatcat tgggagaagc cgcggggaca 3600

gaactgaacg tcctgcagct tcggggcaag tgagacaatc acagctcctc gctgcgtctc 3660

catcaacact gcgccgggta ccatggacgg ccccgtcagc cacacctgtc agcccaagca 3720

gagtgattca ggggctcccc gggggcagac acctgtgcac cccatgagta gtgcccactt 3780

gaggctggca ctcccctgac ctcacctttg caaagttaca gatgcacccc aacattgaga 3840

tgtgttttta atgttaaaat attgatttct acgttatgaa aacagatgcc cccgtgaatg 3900

cttacctgtg agataaccac aaccaggaag aacaaatctg ggcattgagc aagctatgag 3960

ggtccccggg agcacacgaa ccctgccagg cccccgctgg ctcctccagg cacgtcccgg 4020

acctgtgggg ccccagagag gggacatttc cctcctggga gagaaggaga tcagggcaac 4080

tcggagaggg ctgcgagcat ttccctcccg ggagaggaga tcagggcgac ctgcacgcac 4140

tgcgtagagc ctggaaggga agtgagaaac cagccgaccg gccctgcccc tcttcccggg 4200

atcacttaat gaaccacgtg ttttgacatc atgtaaacct aagcacgtag agatgattcg 4260

gatttgacaa aataacattt gagtatccga ttcgccatca ccccctaccc cagaaatagg 4320

acaattcact tcattgacca ggatgatcac atggaaggcg gcgcagaggc agctgtgtgg 4380

gctgcagatt tcctgtgtgg ggttcagcgt agaaaacgca cctccatccc gcccttccca 4440

cagcattcct ccatcttaga tagatggtac tctccaaagg ccctaccaga gggaacacgg 4500

cctactgagc ggacagaatg atgccaaaat attgcttatg tctctacatg gtattgtaat 4560

gaatatctgc tttaatatag ctatcatttc ttttccaaaa ttacttctct ctatctggaa 4620

tttaattaat cgaaatgaat ttatctgaat ataggaagca tatgcctact tgtaatttct 4680

aactccttat gtttgaagag aaacctccgg tgtgagatat acaaatatat ttaattgtgt 4740

catattaaac ttctgattca aaaaaaa 4767

<210> 7
<211> 1015
<212> PRT
<213> Homo sapiens

<400> 7

Met Gly Pro Pro Leu Pro Leu Leu Leu Leu Leu Leu Leu Leu Leu Pro
1               5                   10                  15

Pro Arg Val Leu Pro Ala Ala Pro Ser Ser Val Pro Arg Gly Arg Gln
            20                  25                  30

Leu Pro Gly Arg Leu Gly Cys Leu Leu Glu Glu Gly Leu Cys Gly Ala
        35                  40                  45

Ser Glu Ala Cys Val Asn Asp Gly Val Phe Gly Arg Cys Gln Lys Val
    50                  55                  60

Pro Ala Met Asp Phe Tyr Arg Tyr Glu Val Ser Pro Val Ala Leu Gln
65                  70                  75                  80

Arg Leu Arg Val Ala Leu Gln Lys Leu Ser Gly Thr Gly Phe Thr Trp
                85                  90                  95

Gln Asp Asp Tyr Thr Gln Tyr Val Met Asp Gln Glu Leu Ala Asp Leu
            100                 105                 110

Pro Lys Thr Tyr Leu Arg Arg Pro Glu Ala Ser Ser Pro Ala Arg Pro
        115                 120                 125

Ser Lys His Ser Val Gly Ser Glu Arg Arg Tyr Ser Arg Glu Gly Gly
    130                 135                 140

Ala Ala Leu Ala Asn Ala Leu Arg Arg His Leu Pro Phe Leu Glu Ala
145                 150                 155                 160

Leu Ser Gln Ala Pro Ala Ser Asp Val Leu Ala Arg Thr His Thr Ala
                165                 170                 175

Gln Asp Arg Pro Pro Ala Glu Gly Asp Asp Arg Phe Ser Glu Ser Ile
            180                 185                 190

Leu Thr Tyr Val Ala His Thr Ser Ala Leu Thr Tyr Pro Pro Gly Pro
        195                 200                 205

Arg Thr Gln Leu Arg Glu Asp Leu Leu Pro Arg Thr Leu Gly Gln Leu
    210                 215                 220

Gln Pro Asp Glu Leu Ser Pro Lys Val Asp Ser Gly Val Asp Arg His
225                 230                 235                 240

His Leu Met Ala Ala Leu Ser Ala Tyr Ala Ala Gln Arg Pro Pro Ala
                245                 250                 255

Pro Pro Gly Glu Gly Ser Leu Glu Pro Gln Tyr Leu Leu Arg Ala Pro
            260                 265                 270

Ser Arg Met Pro Arg Pro Leu Leu Ala Pro Ala Ala Pro Gln Lys Trp
        275                 280                 285

Pro Ser Pro Leu Gly Asp Ser Glu Asp Pro Ser Ser Thr Gly Asp Gly
    290                 295                 300

Ala Arg Ile His Thr Leu Leu Lys Asp Leu Gln Arg Gln Pro Ala Glu
305                 310                 315                 320

Val Arg Gly Leu Ser Gly Leu Glu Leu Asp Gly Met Ala Glu Leu Met
                325                 330                 335

Ala Gly Leu Met Gln Gly Val Asp His Gly Val Ala Arg Gly Ser Pro
            340                 345                 350

Gly Arg Ala Ala Leu Gly Glu Ser Gly Glu Gln Ala Asp Gly Pro Lys
        355                 360                 365

Ala Thr Leu Arg Gly Asp Ser Phe Pro Asp Asp Gly Val Gln Asp Asp
    370                 375                 380

Asp Asp Arg Leu Tyr Gln Glu Val His Arg Leu Ser Ala Thr Leu Gly
385                 390                 395                 400

Gly Leu Leu Gln Asp His Gly Ser Arg Leu Leu Pro Gly Ala Leu Pro
                405                 410                 415

Phe Ala Arg Pro Leu Asp Met Glu Arg Lys Lys Ser Glu His Pro Glu
            420                 425                 430

Ser Ser Leu Ser Ser Glu Glu Glu Thr Ala Gly Val Glu Asn Val Lys
        435                 440                 445

Ser Gln Thr Tyr Ser Lys Asp Leu Leu Gly Gln Gln Pro His Ser Glu
    450                 455                 460

Pro Gly Ala Ala Ala Phe Gly Glu Leu Gln Asn Gln Met Pro Gly Pro
465                 470                 475                 480

Ser Lys Glu Glu Gln Ser Leu Pro Ala Gly Ala Gln Glu Ala Leu Ser
                485                 490                 495

Asp Gly Leu Gln Leu Glu Val Gln Pro Ser Glu Glu Glu Ala Arg Gly
            500                 505                 510

Tyr Ile Val Thr Asp Arg Asp Pro Leu Arg Pro Glu Glu Gly Arg Arg
        515                 520                 525

Leu Val Glu Asp Val Ala Arg Leu Leu Gln Val Pro Ser Ser Ala Phe
    530                 535                 540

Ala Asp Val Glu Val Leu Gly Pro Ala Val Thr Phe Lys Val Ser Ala
545                 550                 555                 560

Asn Val Gln Asn Val Thr Thr Glu Asp Val Glu Lys Ala Thr Val Asp
                565                 570                 575

Asn Lys Asp Lys Leu Glu Glu Thr Ser Gly Leu Lys Ile Leu Gln Thr
            580                 585                 590

Gly Val Gly Ser Lys Ser Lys Leu Lys Phe Leu Pro Pro Gln Ala Glu
        595                 600                 605

Gln Glu Asp Ser Thr Lys Phe Ile Ala Leu Thr Leu Val Ser Leu Ala
    610                 615                 620

Cys Ile Leu Gly Val Leu Leu Ala Ser Gly Leu Ile Tyr Cys Leu Arg
625                 630                 635                 640

His Ser Ser Gln His Arg Leu Lys Glu Lys Leu Ser Gly Leu Gly Gly
                645                 650                 655

Asp Pro Gly Ala Asp Ala Thr Ala Ala Tyr Gln Glu Leu Cys Arg Gln
            660                 665                 670

Arg Met Ala Thr Arg Pro Pro Asp Arg Pro Glu Gly Pro His Thr Ser
        675                 680                 685

Arg Ile Ser Ser Val Ser Ser Gln Phe Ser Asp Gly Pro Ile Pro Ser
    690                 695                 700

Pro Ser Ala Arg Ser Ser Ala Ser Ser Trp Ser Glu Glu Pro Val Gln
705                 710                 715                 720

Ser Asn Met Asp Ile Ser Thr Gly His Met Ile Leu Ser Tyr Met Glu
                725                 730                 735

Asp His Leu Lys Asn Lys Asn Arg Leu Glu Lys Glu Trp Glu Ala Leu
            740                 745                 750

Cys Ala Tyr Gln Ala Glu Pro Asn Ser Ser Phe Val Ala Gln Arg Glu
        755                 760                 765

Glu Asn Val Pro Lys Asn Arg Ser Leu Ala Val Leu Thr Tyr Asp His
    770                 775                 780

Ser Arg Val Leu Leu Lys Ala Glu Asn Ser His Ser His Ser Asp Tyr
785                 790                 795                 800

Ile Asn Ala Ser Pro Ile Met Asp His Asp Pro Arg Asn Pro Ala Tyr
                805                 810                 815

Ile Ala Thr Gln Gly Pro Leu Pro Ala Thr Val Ala Asp Phe Trp Gln
            820                 825                 830

Met Val Trp Glu Ser Gly Cys Val Val Ile Val Met Leu Thr Pro Leu
        835                 840                 845

Ala Glu Asn Gly Val Arg Gln Cys Tyr His Tyr Trp Pro Asp Glu Gly
    850                 855                 860

Ser Asn Leu Tyr His Ile Tyr Glu Val Asn Leu Val Ser Glu His Ile
865                 870                 875                 880

Trp Cys Glu Asp Phe Leu Val Arg Ser Phe Tyr Leu Lys Asn Leu Gln
                885                 890                 895

Thr Asn Glu Thr Arg Thr Val Thr Gln Phe His Phe Leu Ser Trp Tyr
            900                 905                 910

Asp Arg Gly Val Pro Ser Ser Ser Arg Ser Leu Leu Asp Phe Arg Arg
        915                 920                 925

Lys Val Asn Lys Cys Tyr Arg Gly Arg Ser Cys Pro Ile Ile Val His
    930                 935                 940

Cys Ser Asp Gly Ala Gly Arg Ser Gly Thr Tyr Val Leu Ile Asp Met
945                 950                 955                 960

Val Leu Asn Lys Met Ala Lys Gly Ala Lys Glu Ile Asp Ile Ala Ala
                965                 970                 975

Thr Leu Glu His Leu Arg Asp Gln Arg Pro Gly Met Val Gln Thr Lys
            980                 985                 990

Glu Gln Phe Glu Phe Ala Leu Thr Ala Val Ala Glu Glu Val Asn Ala
        995                 1000                1005

Ile Leu Lys Ala Leu Pro Gln
    1010                1015